# EUROPÄISCHE PATENTSCHRIFT

(11) **EP 2 185 552 B1**
(45) Veröffentlichungstag und Bekanntmachung des Hinweises auf die Patenterteilung: **12.03.2014**
(21) Anmeldenummer: 08784986.5
(22) Anmeldetag: 23.07.2008
(51) Int. Cl.: C07D 417/12, A61K 31/4439, A61P 9/00

(54) **DIPEPTOID-PRODRUGS UND IHRE VERWENDUNG**
DIPEPTOID PRODRUGS AND THE USE THEREOF
PRODROGUES DIPEPTOÏDES ET LEUR UTILISATION

(30) Priorität: 01.08.2007 DE 102007036076
(43) Veröffentlichungstag der Anmeldung: 19.05.2010
(73) Patentinhaber: Bayer Intellectual Property GmbH, 40789 Monheim (DE)
(72) Erfinder: LERCHEN, Hans-Georg, 51375 Leverkusen (DE); KRENZ, Ursula, 42799 Leichlingen (DE); KELDENICH, Jörg, 42113 Wuppertal (DE); DIEDRICHS, Nicole, 42553 Velbert (DE); KRAHN, Thomas, 58135 Hagen (DE); HIRTH-DIETRICH, Claudia, 42115 Wuppertal (DE); ALBRECHT-KÜPPER, Barbara, 42289 Wülfrath (DE)
(74) Vertreter: BIP Patents
(86) Internationale Anmeldenummer: PCT/EP2008/006027
(87) Internationale Veröffentlichungsnummer: WO 2009/015811

(56) Entgegenhaltungen:
- WO-A-03/053441
- ANAND B S ET AL: "NOVEL DIPEPTIDE PRODRUGS OF ACYCLOVIR FOR OCULAR HERPES INFECTIONS: BIOREVERSION, ANTIVIRAL ACTIVITY AND TRANSPORT ACROSS RABBIT CORNEA" CURRENT EYE RESEARCH, IRL PRESS, OXFORD, GB, Bd. 26, Nr. 3-04, 1. März 2003 (2003-03-01), Seiten 151-163, XP009036464 ISSN: 0271-3683

## Beschreibung

Die vorliegende Anmeldung betrifft Prodrug-Derivate von 2-Amino-6-({[2-(4-chlorphenyl)-1,3-thiazol-4-yl]methyl}thio)-4-[4-(2-hydroxyethoxy)phenyl]pyridin-3,5-dicarbonitril, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von kardiovaskulären Erkrankungen.

Prodrugs sind Derivate eines Wirkstoffs, die *in vivo* eine ein- oder mehrstufige Biotransformation enzymatischer und/oder chemischer Art durchlaufen, bevor der eigentliche Wirkstoff freigesetzt wird. Ein Prodrug-Rest wird in der Regel genutzt, um das Eigenschaftsprofil des zu Grunde liegenden Wirkstoffs zu verbessern [P. Ettmayer et al., J. Med. Chem. 47, 2393-2404 (2004)]. Um ein optimales Wirkprofil zu erreichen, muss dabei das Design des Prodrug-Restes ebenso wie der angestrebte Freisetzungsmechanismus sehr genau auf den individuellen Wirkstoff, die Indikation, den Wirkort und die Applikationsroute abgestimmt werden. Eine große Zahl von Arzneimitteln wird als Prodrugs verabreicht, die gegenüber dem zu Grunde liegenden Wirkstoff eine verbesserte Bioverfügbarkeit aufweisen, beispielsweise erzielt durch eine Verbesserung des physikochemischen Profils, speziell der Löslichkeit, der aktiven oder passiven Absorptionseigenschaften oder der gewebsspezifischen Verteilung. Aus der umfangreichen Literatur über Prodrugs sei beispielhaft genannt: H. Bundgaard (Ed.), Design of Prodrugs: Bioreversible derivatives for various functional groups and chemical entities, Elsevier Science Publishers B.V., 1985.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Wirkungen als hormonähnliche Substanz oder Neurotransmitter aus. Über Adenosin A1-Rezeptoren werden essentielle Funktionen vor allem in erregbaren und/oder arbeitenden Zellen verschiedener Gewebe beeinflusst [vgl. K. A. Jacobson und Z.-G. Gao, Nat. Rev. Drug Discover. 5, 247-264 (2006)].

Die Verbindung 2-Amino-6-({[2-(4-chlorphenyl)-1,3-thiazol-4-yl]methyl}thio)-4-[4-(2-hydroxyethoxy)phenyl]pyridin-3,5-dicarbonitril [Verbindung (A)] ist ein oral wirksamer Adenosin A1-Rezeptor-Agonist und befindet sich gegenwärtig in vertiefter klinischer Prüfung als möglicher neuer Arzneimittelwirkstoff für die Prävention und Therapie insbesondere kardiovaskulärer Erkrankungen [WHO Drug Information Vol. 20, No. 2 (2006); zur Herstellung und Verwendung siehe WO 03/053441, Beispiel 6].

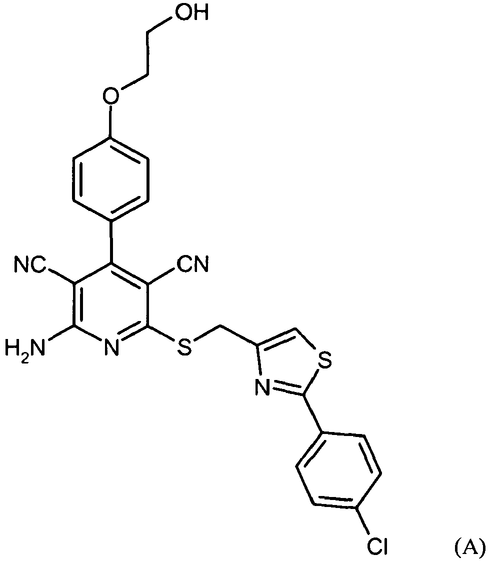

Verbindung (A) weist jedoch nur eine begrenzte Löslichkeit in Wasser, physiologischen Medien und organischen Lösungsmitteln sowie eine nur geringe Bioverfügbarkeit nach oraler Applikation einer Suspension kristallinen Materials auf. Dies erlaubt einerseits eine intravenöse Applikation des Wirkstoffs nur in sehr geringen Dosierungen; Infusionslösungen auf Basis physiologischer Salzlösungen sind mit gebräuchlichen Lösungsvermittlern nur schwer herstellbar. Andererseits ist eine Formulierung in Tablettenform erschwert. Aufgabe der vorliegenden Erfindung war daher die Identifizierung von Derivaten oder Prodrugs von Verbindung (A), die eine verbesserte Löslichkeit in den genannten Medien und/oder eine verbesserte Bioverfügbarkeit nach oraler Applikation besitzen und gleichzeitig nach Applikation eine kontrollierte Freisetzung des Wirkstoffs (A) im Körper des Patienten erlauben. Durch eine verbesserte intravenöse Applizierbarkeit könnten zudem weitere therapeutische Einsatzgebiete für diesen Wirkstoff erschlossen werden.

Eine Übersicht zu Prodrug-Derivaten auf Basis von Carbonsäureestern und möglichen Eigenschaften solcher Verbindungen ist beispielsweise in K. Beaumont et al., Curr. Drug Metab. 4, 461-485 (2003) gegeben.

Gegenstand der vorliegenden Erfindung sind Verbindungen der allgemeinen Formel (I) 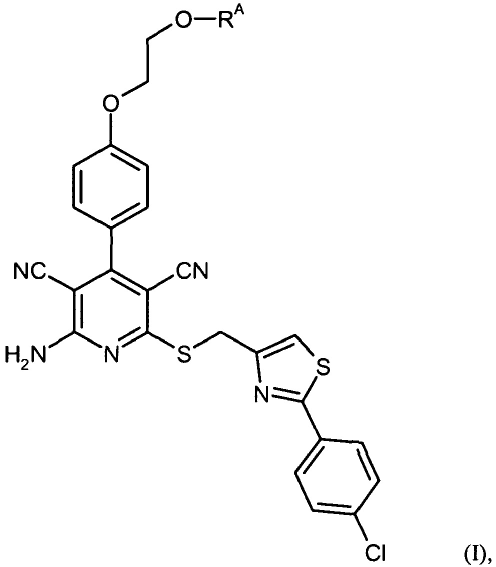 in welcher
- R^{A}: für eine Gruppe der Formel 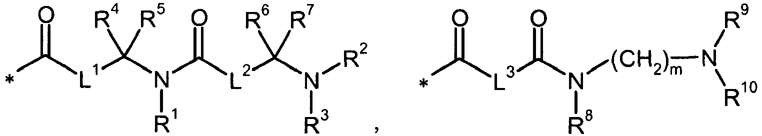 oder 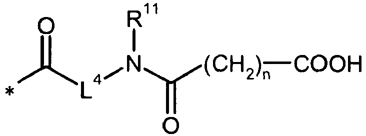 steht, worin
* die Verknüpfungsstelle mit dem O-Atom bedeutet,
L¹ und L² unabhängig voneinander eine Bindung oder -CH₂- bedeuten,
R¹, R² und R³ unabhängig voneinander Wasserstoff oder Methyl bedeuten,
R⁴ und R⁶ gleich oder verschieden sind und unabhängig voneinander Wasserstoff oder Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), *tert*.-Butyl (2-*tert*.-Butylglycin), Phenyl (2-Phenylglycin), Benzyl (Phenylalanin), p-Hydroxybenzyl (Tyrosin), Indol-3-yl-methyl (Tryptophanl, Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S*-Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin) bedeuten,
R⁵ und R⁷ unabhängig voneinander Wasserstoff oder Methyl bedeuten,
L³ geradkettiges oder verzweigtes (C₂-C₄)-Alkandiyl, welches darüber hinaus mit Amino substituiert ist, bedeutet,
R⁸, R⁹ und R¹⁰ unabhängig voneinander Wasserstoff oder Methyl bedeuten,
m die Zahl 2, 3, 4, 5 oder 6 bedeutet,
L⁴ geradkettiges oder verzweigtes (C₂-C₄)-Alkandiyl, welches darüber hinaus mit Carboxyl substituiert ist, bedeutet,
R¹¹ Wasserstoff oder Methyl bedeutet
und
n die Zahl 1, 2, 3 oder 4 bedeutet,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können. Neben Mono-Salzen sind von der vorliegenden Erfindung auch gegebenenfalls mögliche Mehrfach-Salze wie Di- oder Tri-Salze eingeschlossen.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Cholin, Dicyclohexylamin, Dimethylaminoethanol, Prokain, Dibenzylamin, Morpholin, *N*-Methylmorpholin, Arginin, Lysin, Ethylendiamin, Piperidin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₂-C₄)-Alkandiyl steht im Rahmen der Erfindung für einen geradkettigen oder verzweigten divalenten Alkylrest mit 2 bis 4 Kohlenstoffatomen. Bevorzugt ist ein geradkettiger Alkandiylrest mit 2 bis 4 Kohlenstoffatomen. Beispielhaft und vorzugsweise seien genannt: Ethan-1,2-diyl (1,2-Ethylen), Ethan-1,1-diyl, Propan-1,3-diyl (1,3-Propylen), Propan-1,1-diyl, Propan-1,2-diyl, Propan-2,2-diyl, Butan-1,4-diyl (1,4-Butylen), Butan-1,2-diyl, Butan-1,3-diyl, Butan-2,3-diyl. Der Alkandiylrest ist darüber hinaus im Fall der Gruppe L³ mit einer Amino-Gruppe bzw. im Fall der Gruppe L⁴ mit einer Carboxyl-Gruppe substituiert.

Die Seitengruppen einer α-Aminosäure in der Bedeutung von R⁴ und R⁶ umfasst sowohl die Seitengruppen der natürlich vorkommenden α-Aminosäuren als auch die Seitengruppen von Homologen und Isomeren dieser α-Aminosäuren. Die α-Aminosäure kann hierbei sowohl in der L- als auch in der D-Konfiguration oder auch als Gemisch der L- und D-Form vorliegen. Als Seitengruppen seien genannt: Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), *tert*.-Butyl (2-*tert.-*Butylglycin), Phenyl (2-Phenylglycin), Benzyl (Phenylalanin), p-Hydroxybenzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (*S-*Methylcystein), 2-Mercaptoethyl (Homocystein), 2-Methylthioethyl (Methionin), Carbamoylmethyl (Asparagin), 2-Carbamoylethyl (Glutamin), Carboxymethyl (Asparaginsäure), 2-Carboxyethyl (Glutaminsäure), 4-Aminobutan-1-yl (Lysin), 4-Amino-3-hydroxybutan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Guanidinopropan-1-yl (Arginin), 3-Ureidopropan-1-yl (Citrullin). Bevorzugte α-Aminosäure-Seitengruppen in der Bedeutung von R⁴ sind Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), Benzyl (Phenylalanin), p-Hydroxybenzyl (Tyrosin), Imidazol-4-ylmethyl (Histidin), Hydroxymethyl (Serin), 1-Hydroxyethyl (Threonin), Carbamoylmethyl (Asparagin), 2-Carbamoylethyl (Glutamin). Bevorzugte α-Aminosäure-Seitengruppen in der Bedeutung von R⁶ sind Imidazol-4-ylmethyl (Histidin), 4-Aminobutan-1-yl (Lysin), 3-Aminopropan-1-yl (Ornithin), 2-Aminoethyl (2,4-Diaminobuttersäure), Aminomethyl (2,3-Diaminopropionsäure), 3-Guanidino-propan-1-yl (Arginin). Bevorzugt ist jeweils die L-Konfiguration.

Bevorzugt sind Verbindungen der Formel (I), in welcher
- R^{A}: für eine Gruppe der Formel 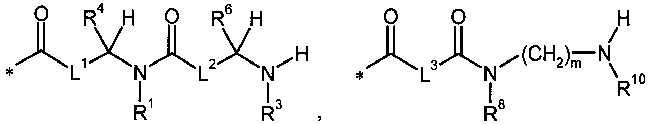 oder 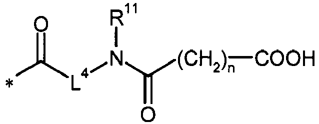 steht, worin
* die Verknüpfungsstelle mit dem O-Atom bedeutet,
L¹ eine Bindung bedeutet,
L² eine Bindung oder -CH₂- bedeutet,
R¹ und R³ unabhängig voneinander Wasserstoff oder Methyl bedeuten,
R⁴ Wasserstoff, Methyl, Propan-2-yl, Propan-1-yl, 2-Methylpropan-1-yl, 1-Methylpropan-1-yl, Butan-1-yl, Benzyl, p-Hydroxybenzyl, Imidazol-4-ylmethyl, Hydroxymethyl, 1-Hydroxyethyl, Carbamoylmethyl oder 2-Carbamoylethyl bedeutet,
R⁶ Wasserstoff, Imidazol-4-ylmethyl, 4-Aminobutan-1-yl, 3-Aminopropan-1-yl, 2-Aminoethyl, Aminomethyl oder 3-Guanidinopropan-1-yl bedeutet,
L³ geradkettiges (C₂-C₄)-Alkandiyl, welches darüber hinaus mit Amino substituiert ist, bedeutet,
R⁸ und R¹⁰ unabhängig voneinander Wasserstoff oder Methyl bedeuten,
m die Zahl 2, 3 oder 4 bedeutet,
L⁴ geradkettiges (C₂-C₄)-Alkandiyl, welches darüber hinaus mit Carboxyl substituiert ist, bedeutet,
R¹¹ Wasserstoff oder Methyl bedeutet
und
n die Zahl 2, 3 oder 4 bedeutet,
sowie ihre Salze, Solvate und Solvate der Salze.

Besonders bevorzugt sind Verbindungen der Formel (I), in welcher
- R^{A}: für eine Gruppe der Formel 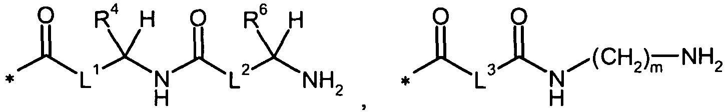 oder 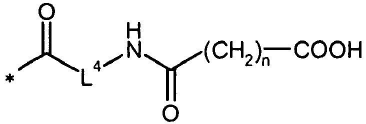 steht, worin
* die Verknüpfungsstelle mit dem O-Atom bedeutet,
L¹ und L² jeweils eine Bindung bedeuten,
R⁴ Wasserstoff, Methyl, Propan-2-yl, Propan-1-yl, 2-Methylpropan-1-yl, 1-Methylpropan-1-yl, Butan-1-yl, Benzyl, p-Hydroxybenzyl, Imidazol-4-ylmethyl, Hydroxymethyl, 1-Hydroxyethyl, Carbamoylmethyl oder 2-Carbamoylethyl bedeutet,
R⁶ Imidazol-4-ylmethyl, 4-Aminobutan-1-yl, 3-Aminopropan-1-yl, 2-Aminoethyl, Aminomethyl oder 3-Guanidinopropan-1-yl bedeutet,
L³ eine Gruppe der Formel -CH(NH₂)-CH₂-, -CH₂-CH(NH₂)-, -CH₂-CH(NH₂)-CH₂-, -CH(NH₂)-CH₂-CH₂- oder -CH₂-CH₂-CH(NH₂)- bedeutet,
m die Zahl 2, 3 oder 4 bedeutet,
L⁴ eine Gruppe der Formel **-CH₂-CH(COOH)- oder **-CH₂-CH₂-CH(COOH)-bedeutet, worin
** die Verknüpfungsstelle mit der angrenzenden Carbonylgruppe darstellt, und
n die Zahl 2 oder 3 bedeutet,
sowie ihre Salze, Solvate und Solvate der Salze.

Ganz besonders bevorzugt sind Verbindungen der Formel (I), in welcher
- R^{A}: für eine Gruppe der Formel 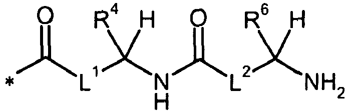 steht, worin
* die Verknüpfungsstelle mit dem O-Atom bedeutet,
L¹ und L² jeweils eine Bindung bedeuten,
R⁴ Wasserstoff, Methyl, Propan-2-yl, 2-Methylpropan-1-yl, Benzyl, Hydroxymethyl oder 1-Hydroxyethyl bedeutet
und
R⁶ Imidazol-4-ylmethyl, 4-Aminobutan-1-yl, 3-Aminopropan-1-yl, 2-Aminoethyl, Aminomethyl oder 3-Guanidinopropan-1-yl bedeutet,
sowie ihre Salze, Solvate und Solvate der Salze.

Ganz besonders bevorzugt sind auch Verbindungen der Formel (I), in welcher
- R^{A}: für eine Gruppe der Formel 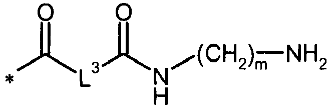 steht, worin
- *: die Verknüpfungsstelle mit dem O-Atom bedeutet,
- L³: eine Gruppe der Formel -CH(NH₂)-CH₂-, -CH₂-CH(NH₂)-, -CH(NH₂)-CH₂-CH₂- oder -CH₂-CH₂-CH(NH₂)- bedeutet
und
- m: die Zahl 2 oder 3 bedeutet,
sowie ihre Salze, Solvate und Solvate der Salze.

Weiterer Gegenstand der Erfindung ist ein Verfahren zur Herstellung der erfindungsgemäßen Verbindungen der Formel (I), dadurch gekennzeichnet, dass man die Verbindung (A) 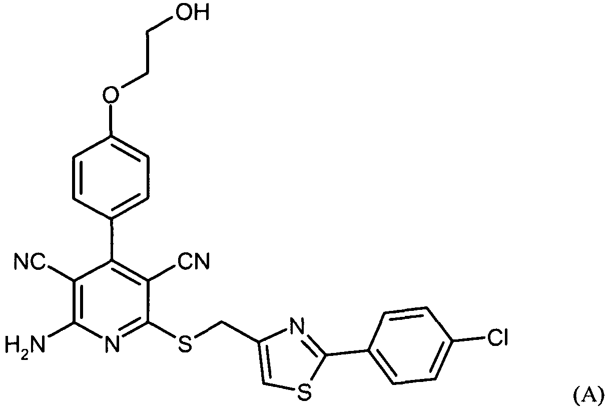 entweder
[A] in einem inerten Lösungsmittel in Gegenwart eines Kondensationsmittels zunächst mit einer Carbonsäure der Formel (II), (III) oder (IV) 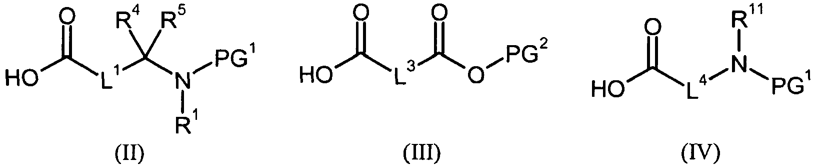
   in welchen L¹, L³, L⁴, R¹, R⁴, R⁵ und R¹¹ jeweils die zuvor angegebenen Bedeutungen haben und

   - PG¹: für eine temporäre Amino-Schutzgruppe wie beispielsweise tert.-Butoxycarbonyl
   und
   - PG²: für eine temporäre Carboxyl-Schutzgruppe wie beispielsweise *tert*.-Butyl stehen,
   zu Verbindungen der Formel (V), (VI) bzw. (VII) 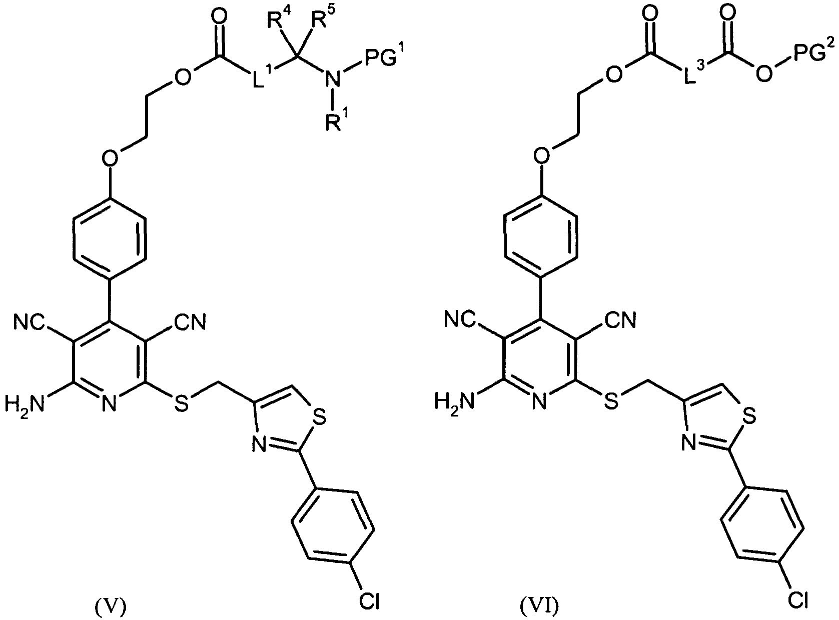 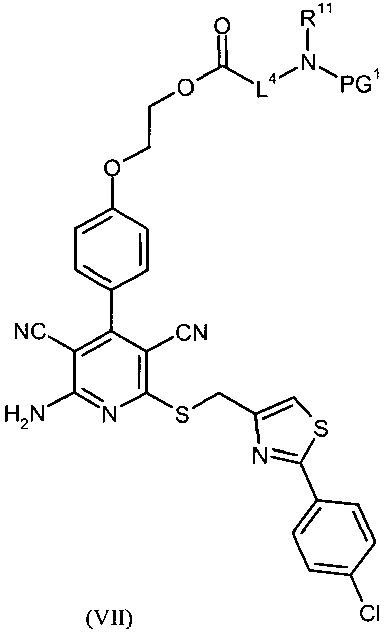 in welchen L¹, L³, L⁴, R¹, R⁴, R⁵, R¹¹, PG¹ und PG² jeweils die oben angegebenen Bedeutungen haben,
   verestert, nach Abspaltung der Schutzgruppe PG¹ bzw. PG² dann in einem inerten Lösungsmittel in Gegenwart eines Kondensationsmittels im Fall der Verbindung (V) mit einer Verbindung der Formel (VIII), im Fall der Verbindung (VI) mit einer Verbindung der Formel (IX) und im Fall der Verbindung (VII) mit einer Verbindung der Formel (X) 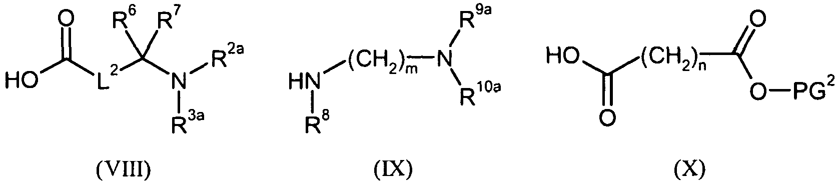
   in welchen L², R⁶, R⁷, R⁸, PG², m und n jeweils die zuvor angegebenen Bedeutungen haben und
   R^{2a} und R^{3a} sowie R^{9a} und R^{10a} jeweils gleich oder verschieden sind und die oben angegebenen Bedeutungen von R², R³, R⁹ bzw. R¹⁰ haben oder für eine temporäre Amino-Schutzgruppe wie beispielsweise *tert*.-Butoxycarbonyl stehen,
   kuppelt und anschließend gegebenenfalls vorhandene Schutzgruppen wieder entfernt
   oder
[B] in einem inerten Lösungsmittel in Gegenwart eines Kondensationsmittels mit einer Verbindung der Formel (XI), (XII) oder (XIII) 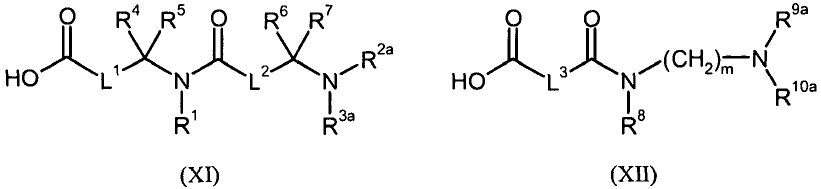 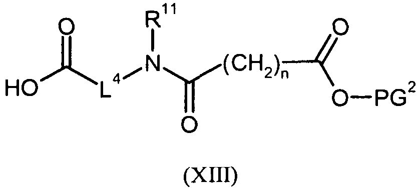
   in welchen L¹, L², L³, L⁴, R¹, R⁴, R⁵, R⁶, R⁷, R⁸, R¹¹, m und n jeweils die zuvor angegebenen Bedeutungen haben,
      - R^{2a} und R^{3a} sowie R^{9a} und R^{10a}: jeweils gleich oder verschieden sind und die oben angegebenen Bedeutungen von R², R³, R⁹ bzw. R¹⁰ haben oder für eine temporäre Amino-Schutzgruppe wie beispielsweise tert.-Butoxycarbonyl stehen
      und
      - PG²: für eine temporäre Carboxyl-Schutzgruppe wie beispielsweise *tert*.-Butyl steht,
   kuppelt und anschließend gegebenenfalls vorhandene Schutzgruppen wieder entfernt
und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Die Transformation (A) → (I) erfolgt somit entweder durch sequentielle Kupplung der einzelnen, gegebenenfalls geeignet geschützten Carbonsäure- bzw. Amin-Komponenten (Verfahrensvariante [A]) oder durch direkte Acylierung mit einem geeignet geschützten Dipeptoid-Derivat (Verfahrensvariante [B]). Die Kupplungsreaktionen (Ester- bzw. Amid-Bildung) werden hierbei nach bekannten Methoden der Peptidchemie durchgeführt [vgl. z.B. M. Bodanszky, Principles of Peptide Synthesis, Springer-Verlag, Berlin, 1993; H.-D. Jakubke und H. Jeschkeit, Aminosäuren, Peptide, Proteine, Verlag Chemie, Weinheim, 1982].

Inerte Lösungsmittel für die Kupplungsreaktionen sind beispielsweise Ether wie Diethylether, *tert*.-Butyl-methylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Aceton, Ethylacetat, Pyridin, Dimethylsulfoxid, Dimethylformamid, *N,N'*-Dimethylpropylenharnstoff (DMPU), *N*-Methylpyrrolidon (NMP) oder Acetonitril. Ebenso ist es möglich, Gemische der genannten Lösungsmittel zu verwenden. Bevorzugt sind Dichlormethan, Dimethylformamid oder Gemische dieser beiden Lösungsmittel.

Als Kondensationsmittel bei diesen Kupplungsreaktionen eignen sich beispielsweise Carbodiimide wie *N,N'*-Diethyl-, *N,N'*-Dipropyl-, *N,N*'-Diisopropyl-, *N,N'*-Dicyclohexylcarbodiimid (DCC) oder *N*-(3-Dimethylaminoisopropyl)-*N'*-ethylcarbodiimid-Hydrochlorid (EDC), Phosgen-Derivate wie *N,N'*-Carbonyldiimidazol (CDI), 1,2-Oxazoliumverbindungen wie 2-Ethyl-5-phenyl-1,2-oxazolium-3-sulfat oder 2-*tert*.-Butyl-5-methylisoxazolium-perchlorat, Acylaminoverbindungen wie 2-Ethoxy-1-ethoxycarbonyl-1,2-dihydrochinolin, oder Isobutylchlorformiat, Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris-(pyrrolidino)phosphonium-hexafluorophosphat (PyBOP), *O*-(Benzotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyluronium-hexafluorophosphat (HATU) oder *O*-(1*H*-6-Chlorbenzotriazol-1-yl)-1,1,3,3-tetramethyluronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder organische Basen wie Triethylamin, *N*-Methylmorpholin, *N-*Methylpiperidin, *N,N*-Diisopropylethylamin oder 4-*N,N-*Dimethylaminopyridin. Zur Ester-Bildung wird bevorzugt *N*-(3-Dimethylaminoisopropyl)-*N*'-ethylcarbodiimid-Hydrochlorid (EDC) in Kombination mit 4-*N,N*-Dimethylaminopyridin eingesetzt. Für eine Amid-Bildung wird vorzugsweise *N*-(3-Dimethylaminoisopropyl)-*N*'-ethylcarbodiimid-Hydrochlorid (EDC) in Kombination mit 1-Hydroxybenzotriazol (HOBt) oder *N*-Hydroxysuccinimid (HOSu) und gegebenenfalls einer Base wie *N,N-*Diisopropylethylamin verwendet.

Die Kupplungen werden im Allgemeinen in einem Temperaturbereich von 0°C bis +60°C, bevorzugt bei +20°C bis +40°C durchgeführt. Die Reaktionen können bei normalem, bei erhöhtem oder bei erniedrigtem Druck erfolgen (z.B. von 0.5 bis 5 bar). Im Allgemeinen arbeitet man bei Normaldruck.

Die Verbindungen der Formel (I) können bei der Herstellung nach den oben beschriebenen Verfahren auch direkt in Form von Salzen entstehen. Diese Salze können gegebenenfalls durch Behandlung mit einer Base bzw. Säure in einem inerten Lösungsmittel, über chromatographische Methoden oder mittels Ionenaustauscherharzen in die jeweiligen freien Basen bzw. Säuren überführt werden. Weitere Salze der erfindungsgemäßen Verbindungen können gegebenenfalls auch durch Austausch von Gegenionen mit Hilfe der Ionenaustausch-Chromatographie, beispielsweise mit Amberlite^{®}-Harzen, hergestellt werden.

In den Resten R⁴, R⁶, L³ und/oder L⁴ gegebenenfalls vorhandene funktionelle Gruppen - wie insbesondere Amino-, Guanidino-, Hydroxy-, Mercapto- und Carboxylgruppen - können bei den zuvor beschriebenen Reaktionssequenzen, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984].

Als Amino- und Guanidino-Schutzgruppe wird bevorzugt *tert*.-Butoxycarbonyl (Boc) oder Benzyloxycarbonyl (Z) verwendet. Als Schutzgruppe für eine Hydroxy- oder Carboxyl-Funktion wird vorzugsweise *tert*.-Butyl oder Benzyl eingesetzt. Die Abspaltung dieser Schutzgruppen wird nach üblichen Methoden, vorzugsweise durch Reaktion mit einer starken Säure wie Chlorwasserstoff, Bromwasserstoff oder Trifluoressigsäure in einem inerten Lösungsmittel wie Dioxan, Dichlormethan oder Essigsäure durchgeführt; gegebenenfalls kann die Abspaltung auch ohne ein zusätzliches inertes Lösungsmittel erfolgen. Im Falle von Benzyl und Benzyloxycarbonyl als Schutzgruppe können diese auch Hydrogenolyse in Gegenwart eines Palladium-Katalysators entfernt werden. Die Abspaltung der genannten Schutzgruppen kann gegebenenfalls simultan in einer Eintopf-Reaktion oder in separaten Reaktionsschritten vorgenommen werden.

Die Verbindungen der Formeln (II), (III), (IV), (VIII), (IX), (X), (XI), (XII) und (XIII) sind kommerziell erhältlich, literaturbekannt oder können nach literaturüblichen Methoden hergestellt werden. So können beispielsweise Verbindungen der Formeln (II) und (VIII), in welchen L¹ bzw. L² für -CH₂- steht, nach bekannten Methoden zur Kettenverlängerung von Carbonsäuren, wie beispielsweise der Arndt-Eistert-Reaktion [Eistert et al., Ber. Dtsch. Chem. Ges. 60, 1364-1370 (1927); Ye et al., Chem. Rev. 94, 1091-1160 (1994); Cesar et al., Tetrahedron Lett. 42, 7099-7102 (2001)] oder der Reaktion mit *N*-Hydroxy-2-thiopyridon [vgl. Barton et al., Tetrahedron Lett. 32, 3309-3312 (1991)], ausgehend von den entsprechenden Verbindungen, in welchen L¹ bzw. L² für eine Bindung steht, erhalten werden.

Die Herstellung von Verbindung (A), 2-Amino-6-({[2-(4-chlorphenyl)-1,3-thiazol-4-yl]methyl}-thio)-4-[4-(2-hydroxyethoxy)phenyl]pyridin-3,5-dicarbonitril, ist in WO 03/053441 als Beispiel 6 beschrieben.

Die Herstellung der erfindungsgemäßen Verbindungen kann durch die folgenden Syntheseschemata veranschaulicht werden:

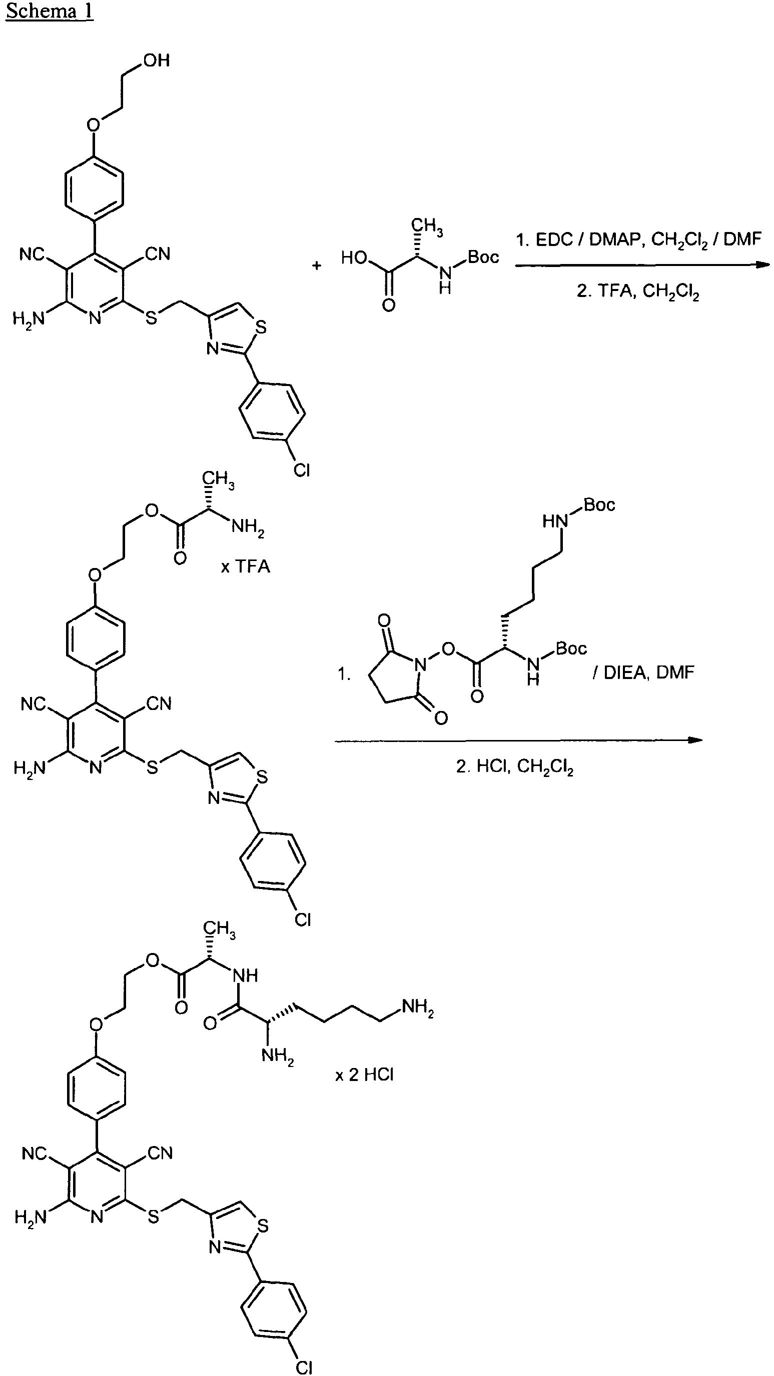

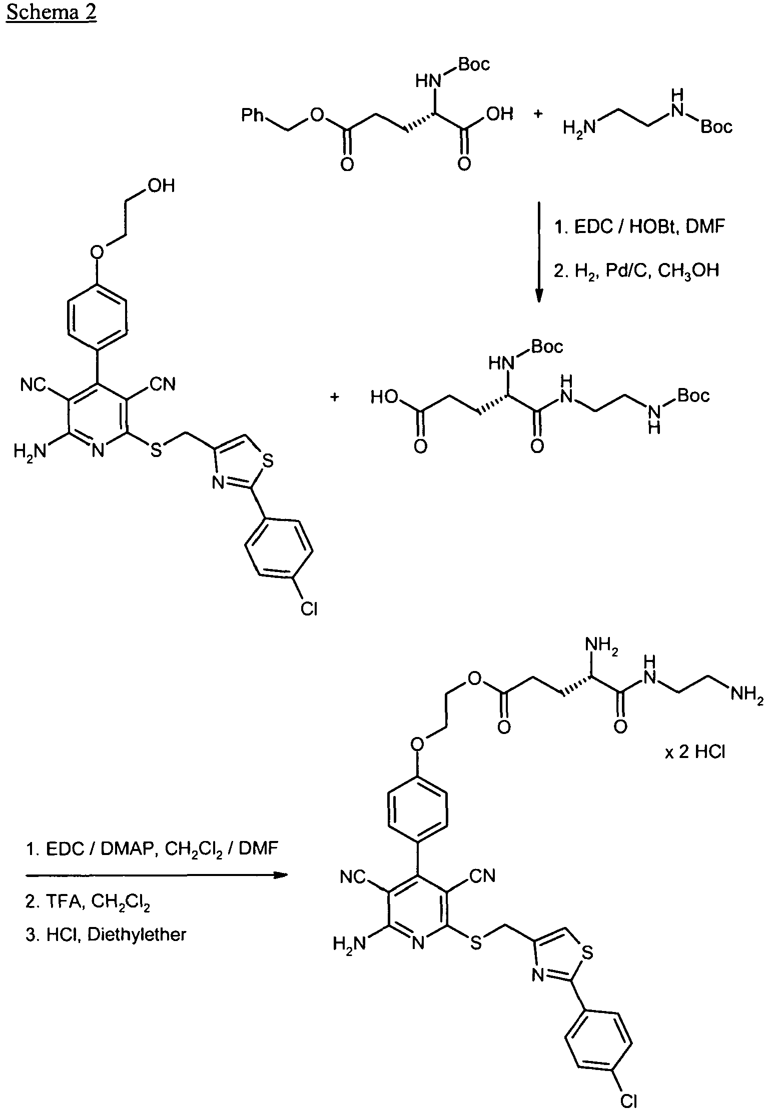

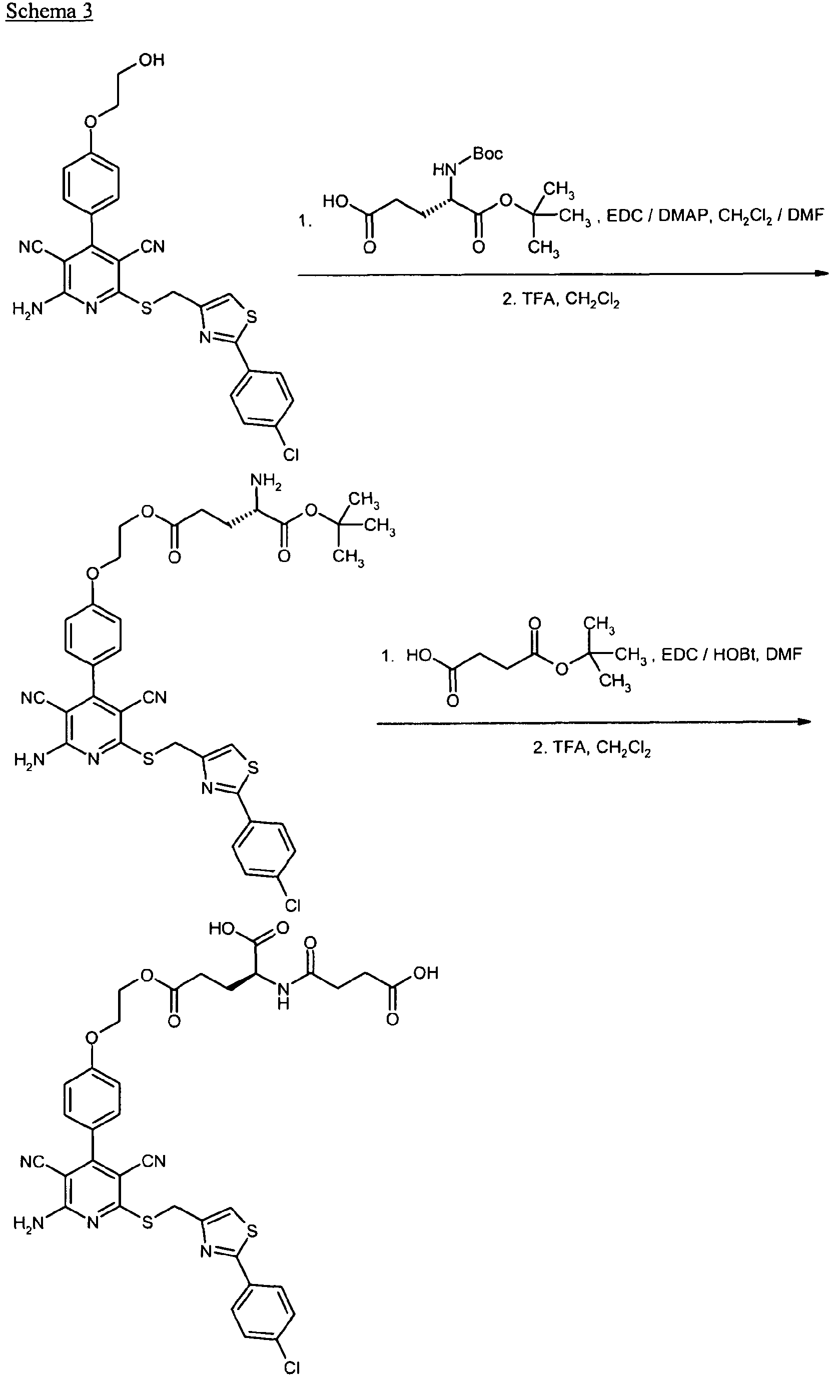

Die erfindungsgemäßen Verbindungen und ihre Salze stellen nützliche Prodrugs der Wirkstoff-Verbindung (A) dar. Sie weisen einerseits eine gute Stabilität bei verschiedenen pH-Werten auf und zeigen andererseits eine effiziente Konversion zur Wirkstoff-Verbindung (A) bei einem physiologischen pH-Wert und insbesondere *in vivo.* Darüber hinaus besitzen die erfindungsgemäßen Verbindungen verbesserte Löslichkeiten in wässrigen oder anderen physiologisch verträglichen Medien, was sie für die therapeutische Anwendung insbesondere bei intravenöser Applikation geeignet macht. Außerdem ist die Bioverfügbarkeit aus Suspension nach oraler Applikation gegenüber der Muttersubstanz (A) verbessert.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere von Erkrankungen des Herzkreislauf-Systems (kardiovaskulären Erkrankungen), zur Kardioprotektion nach Schädigungen des Herzens sowie von Stoffwechsel-Erkrankungen.

Im Sinne der vorliegenden Erfindung sind unter Erkrankungen des Herzkreislauf-Systems bzw. kardiovaskulären Erkrankungen beispielsweise die folgenden Erkrankungen zu verstehen: Hypertonie (Bluthochdruck), periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal-Angina, persistierende ischämische Dysfunktion ("hibernating myocardium"), vorübergehende postischämische Dysfunktion ("stunned myocardium"), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmern mit eingeschränkter linksventrikulärer Funktion, Wolff-Parkinson-White-Syndrom, periphere Durchblutungsstörungen, erhöhte Spiegel von Fibrinogen und von LDL geringer Dichte sowie erhöhte Konzentrationen von Plasminogenaktivator-Inhibitor 1 (PAI-1), insbesondere Hypertonie, koronare Herzerkrankung, akutes Koronarsyndrom, Angina pectoris, Herzinsuffizienz, Myokardinfarkt und Vorhofflimmern.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Weiterhin eignen sich die erfindungsgemäßen Verbindungen insbesondere auch zur Reduktion des von einem Infarkt betroffenen Myokardbereichs sowie zur Prophylaxe von Sekundärinfarkten.

Des weiteren sind die erfindungsgemäßen Verbindungen insbesondere zur Prophylaxe und/oder Behandlung von thromboembolischen Erkrankungen, Reperfusionsschäden nach Ischämie, mikro- und makrovaskulären Schädigungen (Vaskulitis), arteriellen und venösen Thrombosen, Ödemen, Ischämien wie Myokardinfarkt, Hirnschlag und transitorischen ischämischen Attacken, zur Kardioprotektion bei Koronararterien-Bypass-Operationen (CABG), primären perkutan-transluminalen Koronarangioplastien (PTCAs), PTCAs nach Thrombolyse, Rescue-PTCA, Herztransplantationen und Operationen am offenen Herzen, sowie zur Organprotektion bei Transplantationen, Bypass-Operationen, Katheteruntersuchungen und anderen operativen Eingriffen geeignet.

Weitere Indikationsgebiete, für die die erfindungsgemäßen Verbindungen verwendet werden können, sind beispielsweise die Prophylaxe und/oder Behandlung von Erkrankungen des Urogenitalbereiches, wie z.B. akutes Nierenversagen, Reizblase, urogenitale Inkontinenz, erektile Dysfunktion und weibliche sexuelle Dysfunktion, daneben aber auch die Prophylaxe und/oder Behandlung von inflammatorischen Erkrankungen, wie z.B. entzündliche Dermatosen und Arthritis, insbesondere rheumatische Arthritis, von Erkrankungen des Zentralen Nervensystems und neurodegenerativen Störungen (Zustände nach Schlaganfall, Alzheimer'sche Krankheit, Parkinson'sche Krankheit, Demenz, Chorea Huntington, Epilepsie, Depressionen, Multiple Sklerose), von Schmerzzuständen und Migräne, Leberfibrose und Leberzirrhose, von Krebserkrankungen und von Übelkeit und Erbrechen in Verbindung mit Krebstherapien, sowie zur Wundheilung.

Ein weiteres Indikationsgebiet ist beispielsweise die Prophylaxe und/oder Behandlung von Erkrankungen der Atemwege wie beispielsweise Asthma, chronisch-obstruktive Atemwegserkrankungen (COPD, chronische Bronchitis), Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prophylaxe und/oder Behandlung von Stoffwechsel-Erkrankungen in Betracht, wie beispielsweise Diabetes, insbesondere Diabetes mellitus, Gestationsdiabetes, Insulin-abhängiger Diabetes und Nicht-Insulin-abhängiger Diabetes, diabetische Folgeerkrankungen wie z.B. Retinopathie, Nephropathie und Neuropathie, metabolische Erkrankungen wie z.B. Metabolisches Syndrom, Hyperglykämie, Hyperinsulinämie, Insulinresistenz, Glukose-Intoleranz und Fettsucht (Adipositas), sowie Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antiarrhythmika, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten) sowie Analgetika wie beispielsweise Aspirin.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff, einem Antiarrhythmikum und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Adrenozeptor-Antagonisten, alpha-Adrenozeptor-Antagonisten, Diuretika, Aldosteron-Antagonisten, Mineralocorticoid-Rezeptor-Antagonisten, ECE-Inhibitoren sowie der Vasopeptidase-Inhibitoren;
- antithrombotisch wirkenden Mitteln, beispielhaft und vorzugsweise aus der Gruppe der Thrombozytenaggregationshemmer oder der Antikoagulantien;
- Antiarrhythmika, insbesondere solchen zur Behandlung von supraventrikulären Arrhythmien und Tachykardien;
- Substanzen zur Prophylaxe und Behandlung von Ischämie- und Reperfusionsschäden;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost und Cicaprost;
- Calcium-Sensitizern, wie beispielhaft und vorzugsweise Levosimendan;
- Kalium-Supplements;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib;
- den Energiestoffwechsel des Herzens beeinflussenden Verbindungen, wie beispielweise Etomoxir, Dichloracetat, Ranolazine und Trimetazidine;
- Schmerzmitteln; und/oder
- Substanzen zur Prophylaxe und Behandlung von Übelkeit und Erbrechen
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren, DPP-IV-Inhibitoren und PPAR-γ-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin oder Vildagliptin, verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Renin-Inhibitoren, beta-Adrenozeptor-Antagonisten, alpha-Adrenozeptor-Antagonisten und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem beta-Adrenozeptor-Antagonisten, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Adrenozeptor-Antagonisten, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Glycerinnitrat, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin) sowie beta-adrenergen und dopaminergen Agonisten, wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, oder in Kombination mit Kaliumkanal-Agonisten wie Minoxidil, Diazoxid, Dihydralazin oder Hydralazin verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter Antiarrhythmika werden vorzugsweise Substanzen aus der Gruppe der Klasse Ia-Antiarrhythmika (z.B. Chinidin), der Klasse Ic-Antiarrhythmika (z.B. Flecainid, Propafenon), der Klasse II-Antiarrhythmika (z.B. Metoprolol, Atenolol, Sotalol, Oxprenolol und andere beta-Rezeptoren-Blocker), der Klasse III-Antiarrhythmika (z.B. Sotalol, Amiodaron) und der Klasse IV-Antiarrhythmika (z.B. Digoxin sowie Verapamil, Diltiazem und andere Calcium-Antagonisten) verstanden.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Adrenozeptor-Antagonisten, alpha-Adrenozeptor-Antagonisten, organische Nitrate und NO-Donatoren, Calcium-Antagonisten, ACE-Inhibitoren, Angiotensin AII-Antagonisten, Aldosteron- und Mineralocorticoid-Rezeptor-Antagonisten, Vasopressin-Rezeptor-Antagonisten, Thrombozytenaggregationshemmer, Antikoagulantien und Antiarrhythmika, sowie deren Verwendung zur Behandlung und/oder Prophylaxe der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent. Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- Boc: *tert*.-Butoxycarbonyl
- DIEA: *N,N-*Diisopropylethylamin
- DMAP: 4-*N,N*-Dimethylaminopyridin
- DMF: *N,N*-Dimethylformamid
- DMSO: Dimethylsulfoxid
- d. Th.: der Theorie (bei Ausbeute)
- EDC: 1-(3-Dimethylaminopropyl)-3-ethylcarbodiimid-Hydrochlorid
- ESI: Elektrospray-Ionisation (bei MS)
- h: Stunde(n)
- HOBt: 1-Hydroxybenzotriazol
- HPLC: Hochdruck-, Hochleistungsflüssigchromatographie
- LC-MS: Flüssigchromatographie-gekoppelte Massenspektrometrie
- min: Minute(n)
- MS: Massenspektrometrie
- NMR: Kernresonanzspektrometrie
- p: para
- Pd/C: Palladium auf Aktivkohle
- Ph: Phenyl
- quant.: quantitativ (bei Ausbeute)
- RT: Raumtemperatur
- Rₜ: Retentionszeit (bei HPLC)
- tert.: tertiär
- TFA: Trifluoressigsäure
- THF: Tetrahydrofuran
- UV: Ultraviolett-Spektrometrie
- v/v: Volumen-zu-Volumen-Verhältnis (einer Lösung)
- Z: Benzyloxycarbonyl

### LC-MS- und HPLC-Methoden:

### Methode 1 (LC-MS):

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min - 2.5 min/3.0 min/4.5 min 2 ml/min; Ofen: 50°C; UV-Detektion: 210 nm.

### Methode 2 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 3 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (präparative HPLC):

Gerätetyp HPLC: Abimed/Gilson Pump 305/306; Manometric Module 806; UV Knauer Variable Wavelength Monitor; Säule: Gromsil C18, 10 nm, 250 mm x 30 mm; Eluent A: 1 l Wasser + 0.5 ml 99%-ige Trifluoressigsäure, Eluent B: 1 l Acetonitril; Gradient: 0.0 min 2% B → 10 min 2% B → 50 min 90% B; Fluss: 20 ml/min; Volumen: 628 ml A und 372 ml B.

### Methode 6a (präparative HPLC):

Säule: VP 250/21 Nukleodur 100-5 C18 ec, Macherey & Nagel Nr. 762002; Eluent A: Wasser / 0.01% Trifluoressigsäure, Eluent B: Acetonitril / 0.01% Trifluoressigsäure; Gradient: 0 min 0% B → 20 min 20% B → 40 min 20% B → 60 min 30% B → 80 min 30% B → 90 min 100% B → 132 min 100% B; Fluss: 5 ml/min; Temperatur: RT; UV-Detektion: 210 nm.

### Methode 6b (präparative HPLC):

Säule: VP 250/21 Nukleodur 100-5 C18 ec, Macherey & Nagel Nr. 762002; Eluent A: 1 Liter Wasser / 1 ml 99%-ige Trifluoressigsäure, Eluent B: 1 Liter Acetonitril / 1 ml 99%-ige Trifluoressigsäure; Gradient: 0 min 30% B → 20 min 50% B → 40 min 80% B → 60 min 100% B; Fluss: 5 ml/min; Temperatur: RT; UV-Detektion: 210 nm.

### Methode 7 (analytische HPLC):

Säule: XTerra 3.9 x 150 WAT 186000478; Eluent A: 10 ml 70%-ige Perchlorsäure in 2.5 Liter Wasser, Eluent B: Acetonitril; Gradient: 0.0 min 20% B → 1 min 20% B → 4 min 90% B → 9 min 90% B; Temperatur: RT; Fluss: 1 ml/min.

### Methode 8 (LC-MS):

Instrument: Micromass Quattro LCZ mit HPLC Agilent Serie 1100; Säule: Phenomenex Onyx Monolithic C18, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 9 (LC-MS):

Instrument: Micromass Platform LCZ mit HPLC Agilent Serie 1100; Säule: Thermo Hypersil GOLD 3µ, 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 10 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 11 (LC-MS):

Gerätetyp MS: Micromass ZQ; Gerätetyp HPLC: Waters Alliance 2795; Säule: Phenomenex Synergi 2.5 µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 12 (LC-MS):

Instrument: Micromass Quattro LCZ mit HPLC Agilent Serie 1100; Säule: Phenomenex Synergi 2.5 µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208-400 nm.

### Methode 13 (LC-MS):

Instrument: Micromass QuattroPremier mit Waters UPLC Acquity; Säule: Thermo Hypersil GOLD 1.9µ, 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.1 min 100% A → 1.5 min 10% A → 2.2 min 10% A; Ofen: 50°C; Fluss: 0.33 ml/min; UV-Detektion: 210 nm.

### Ausgangsverbindungen und Intermediate:

### Beispiel 1A

### 2-{4-[2-Amino-6-({[2-(4-chlorphenyl)-1,3-thiazol-4-yl]methyl}thio)-3,5-dicyanopyridin-4-yl]-phenoxy}ethyl-L-valinat-Trifluoracetat

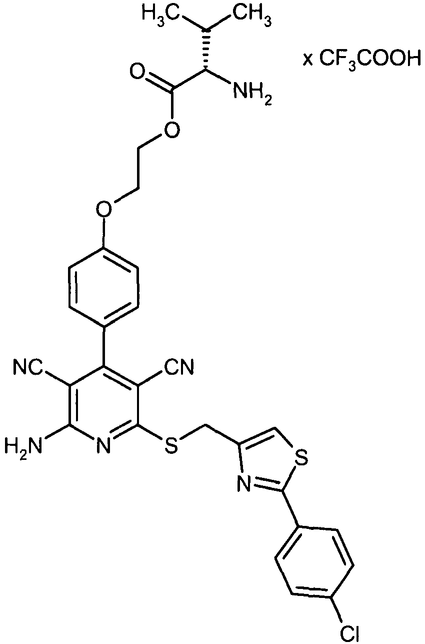

1 g (1.92 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril [WO 03/053441, Beispiel 6], 0.460 g (2.11 mmol) N-Boc-L-Valin, 0.442 g (2.31 mmol) 1-(3-Dimethylaminopropyl)-3-ethylcarbodiimid-Hydrochlorid sowie 0.023 g (0.19 mmol) 4-Dimethylaminopyridin werden in 40 ml Dichlormethan und 10 ml DMF zusammengegeben und über Nacht bei Raumtemperatur gerührt. Es entsteht eine klare Lösung. Der Ansatz wird dann auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird durch Flash-Chromatographie an Kieselgel mit Dichlormethan/Ethylacetat als Eluent gereinigt (Gradient 10:1 → 7:1 → 5: 1). Die entsprechenden Fraktionen werden vereinigt und das Lösungsmittel im Vakuum entfernt. Nach Trocknen des Rückstands im Hochvakuum verbleiben 0.85 g (62% d. Th.) des Boc-geschützten Intermediats.

Der Rückstand wird in 5 ml Dichlormethan und 5 ml wasserfreier Trifluoressigsäure aufgenommen und die Lösung 2 h bei Raumtemperatur gerührt. Der Ansatz wird danach bis zur Trockene eingeengt und der Rückstand mit Ethylacetat verrührt. Der ausfallende Niederschlag wird abfiltriert, mit Diethylether gewaschen und anschließend im Hochvakuum getrocknet. Es resultieren 935 mg (quant.) der Titelverbindung als farblose Kristalle.
HPLC (Methode 7): Rₜ = 5.5 min;
LC-MS (Methode 10): Rₜ = 2.06 min; MS (ESIpos): m/z = 619 (M+H⁺.

### Beispiel 2A

### 2-{4-[2-Amino-6-({[2-(4-chlorphenyl)-1,3-thiazol-4-yl]methyl}thio)-3,5-dicyanopyridin-4-yl]-phenoxy} ethyl-L-alaninat-Trifluoracetat

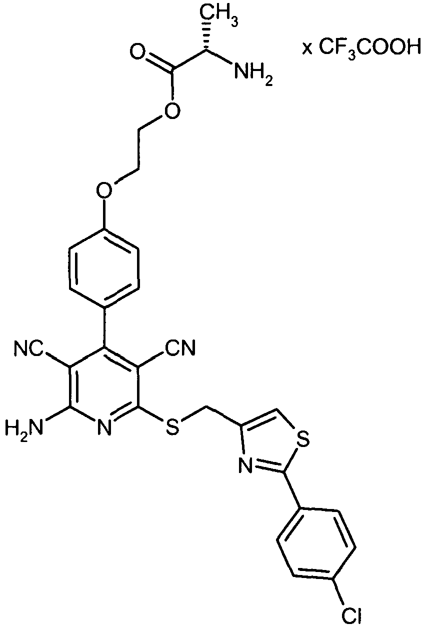

1.5 g (2.88 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 1.64 g (8.66 mmol) N-Boc-L-Alanin, 0.719 g (3.75 mmol) 1-(3-Dimethylaminopropyl)-3-ethylcarbodiimid-Hydrochlorid sowie 0.176 g (1.44 mmol) 4-Dimethylaminopyridin werden in 25 ml Dichlormethan und 25 ml DMF zusammengegeben und 2 h bei Raumtemperatur gerührt. Der Ansatz wird danach im Vakuum eingeengt und der Rückstand in Ethylacetat aufgenommen. Man schüttelt zweimal mit 5%-iger Zitronensäure und zweimal mit Natriumhydrogencarbonat-Lösung aus. Die organische Phase wird eingeengt und der Rückstand mit 50 ml Diethylether sowie 50 ml Pentan verrührt. Der Niederschlag wird abgesaugt und mit Pentan gewaschen. Nach Trocknen des Rückstands im Hochvakuum verbleiben 1.23 g (62% d. Th.) des Boc-geschützten Intermediats.

Der Rückstand wird in 18 ml Dichlormethan und 2 ml wasserfreier Trifluoressigsäure aufgenommen und die Lösung 1 h bei Raumtemperatur im Ultraschallbad behandelt. Der Ansatz wird danach bis zur Trockene eingeengt und der verbleibende Rückstand mit Diethylether verrührt. Der ausfallende Niederschlag wird abfiltriert, mit Diethylether gewaschen und anschließend im Hochvakuum getrocknet. Es resultieren 1200 mg (96% d. Th.) der Titelverbindung als farblose Kristalle.
HPLC (Methode 7): Rₜ = 5.3 min;
LC-MS (Methode 12): Rₜ = 1.73 min; MS (ESIpos): m/z = 591 (M+H)⁺.

### Beispiel 3A

### 2-{4-[2-Amino-6-({[2-(4-chlorphenyl)-1,3-thiazol-4-yl]methyl}thio)-3,5-dicyanopyridin-4-yl]-phenoxy}ethyl-O-tert.-butyl-L-serinat

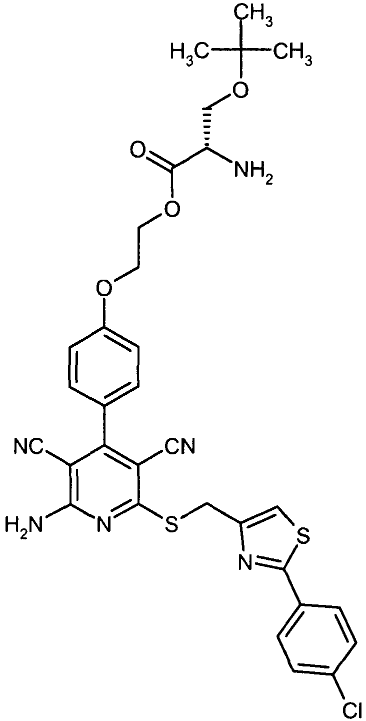

1 g (1.92 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 0.612 g (2.12 mmol) *N*-(*tert*.-Butoxycarbonyl)-*O-tert*.-butyl-L-serin, 0.442 g (2.31 mmol) 1-(3-Dimethylaminopropyl)-3-ethylcarbodiimid-Hydrochlorid sowie 0.024 g (0.192 mmol) 4-Dimethylaminopyridin werden in 40 ml Dichlormethan und 10 ml DMF zusammengegeben und über Nacht bei Raumtemperatur gerührt. Der Ansatz wird dann auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird in Dichlormethan aufgenommen und mit Diethylether versetzt. Der ausfallende Niederschlag wird abgesaugt und mit Diethylether gewaschen. Nach Trocknen im Hochvakuum verbleiben 1.25 g (85% d. Th.) des geschützten Intermediats.

Der Rückstand wird in 100 ml Dichlormethan und 10 ml wasserfreier Trifluoressigsäure aufgenommen und die Lösung 1 h bei Raumtemperatur gerührt. Der Ansatz wird danach auf eine Mischung aus halbgesättigter Natriumhydrogencarbonat-Lösung und Dichlormethan gegossen. Die organische Phase wird abgetrennt, über Magnesiumsulfat getrocknet, filtriert und eingeengt. Nach Trocknen im Hochvakuum resultieren 1020 mg (95% d. Th.) der Titelverbindung als farbloses Pulver.
HPLC (Methode 7): Rₜ = 5.4 min;
LC-MS (Methode 11): Rₜ = 1.65 min; MS (ESIpos): m/z = 663 (M+H)⁺.

### Beispiel 4A

### N²-(tert.-Butoxycarbonyl)-N-{2-[(tert.-butoxycarbonyl)amino]ethyl}-L-α-glutamin

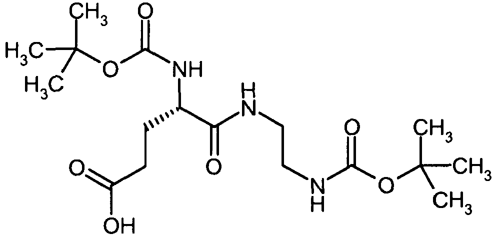

1.5 g (4.45 mmol) (2*S*)-5-(Benzyloxy)-2-[(*tert*.-butoxycarbonyl)amino]-5-oxopentansäure, 783 mg (4.89 mmol) *tert*.-Butyl-(2-aminoethyl)carbamat, 938 mg (4.89 mmol) 1-(3-Dimethylaminopropyl)-3-ethylcarbodiimid-Hydrochlorid sowie 749 mg (0.489 mmol) 1-Hydroxy-1*H*-benzotriazol-Hydrat werden in 140 ml DMF zusammengegeben und über Nacht bei Raumtemperatur gerührt. Der Ansatz wird dann auf eine Mischung aus halbgesättigter Ammoniumchlorid-Lösung und Ethylacetat gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird mit Diethylether verrührt. Der ausfallende Niederschlag wird abgesaugt, mit Diethylether gewaschen und im Hochvakuum getrocknet. Es verbleiben 1.9 g (89% d. Th.) des geschützten Intermediats.
LC-MS (Methode 12): Rₜ = 2.19 min; MS (ESIpos): m/z = 480 (M+H)⁺.

1.9 g (3.96 mmol) des erhaltenen Intermediats werden in 125 ml Methanol gelöst und nach Zugabe von 250 mg 10% Palladium auf Aktivkohle 2 h bei RT unter Normaldruck hydriert. Der Katalysator wird dann abfiltriert und das Lösungsmittel im Vakuum entfernt. Man erhält 1500 mg (97% d. Th.) der Titelverbindung als farblosen Schaum.
LC-MS (Methode 10): Rₜ = 1.94 min; MS (ESIpos): m/z = 390 (M+H)⁺.

### Beispiel 5A

### N²-(tert.-Butoxycarbonyl)-N-{2-[(tert.-butoxycarbonyl)amino]ethyl}-L-glutamin

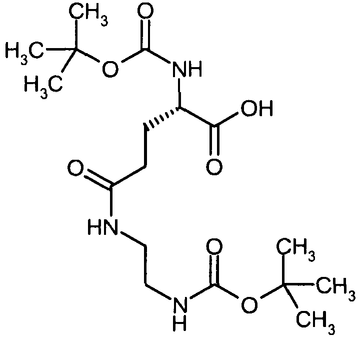

1.5 g (4.45 mmol) (4*S*)-5-(Benzyloxy)-4-[(*tert*.-butoxycarbonyl)amino]-5-oxopentansäure, 783 mg (4.89 mmol) tert.-Butyl-(2-aminoethyl)carbamat, 938 mg (4.89 mmol) 1-(3-Dimethylaminopropyl)-3-ethylcarbodiimid-Hydrochlorid sowie 749 mg (4.89 mmol) 1-Hydroxy-1H-benzotriazol-Hydrat werden in 140 ml DMF zusammengegeben und über Nacht bei Raumtemperatur gerührt. Der Ansatz wird dann auf eine Mischung aus halbgesättigter Ammoniumchlorid-Lösung und Ethylacetat gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird mit Diethylether verrührt. Der ausfallende Niederschlag wird abgesaugt, mit Diethylether gewaschen und im Hochvakuum getrocknet. Es verbleiben 2.1 g (98% d. Th.) des geschützten Intermediats.
LC-MS (Methode 10): Rₜ = 2.47 min; MS (ESIpos): m/z = 480 (M+H)⁺.

2.1 g (4.38 mmol) des erhaltenen Intermediats werden in 140 ml Methanol gelöst und nach Zugabe von 250 mg 10% Palladium auf Aktivkohle 2 h bei RT unter Normaldruck hydriert. Der Katalysator wird dann abfiltriert und das Lösungsmittel im Vakuum entfernt. Man erhält 1540 mg (90% d. Th.) der Titelverbindung als farblosen Schaum.
LC-MS (Methode 11): Rₜ = 1.35 min; MS (ESIpos): m/z = 390 (M+H)⁺.

### Beispiel 6A

### N²-(tert.-Butoxycarbonyl)-N-{2-[(tert.-butoxycarbonyl)amino]ethyl}-L-asparagin

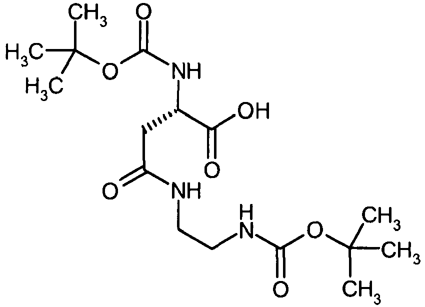

1.5 g (4.64 mmol) (3*S*)-4-(Benzyloxy)-3-[(*tert*.-butoxycarbonyl)amino]-4-oxobuttersäure, 818 mg (5.1 mmol) *tert*.-Butyl-(2-aminoethyl)carbamat, 978 mg (5.1 mmol) 1-(3-Dimethylaminopropyl)-3-ethylcarbodiimid-Hydrochlorid sowie 781 mg (5.1 mmol) 1-Hydroxy-1*H*-benzotriazol-Hydrat werden in 75 ml DMF zusammengegeben und über Nacht bei Raumtemperatur gerührt. Der Ansatz wird dann auf eine Mischung aus halbgesättigter Ammoniumchlorid-Lösung und Ethylacetat gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird mit Diethylether verrührt. Der ausfallende Niederschlag wird abgesaugt, mit Diethylether gewaschen und im Hochvakuum getrocknet. Es verbleiben 2.1 g (81% d. Th.) des geschützten Intermediats.
LC-MS (Methode 10): Rₜ = 2.47 min; MS (ESIpos): m/z = 466 (M+H)⁺.

2.1 g (4.51 mmol) des erhaltenen Intermediats werden in 140 ml Methanol gelöst und nach Zugabe von 250 mg 10% Palladium auf Aktivkohle 2 h bei RT unter Normaldruck hydriert. Der Katalysator wird dann abfiltriert und das Lösungsmittel im Vakuum entfernt. Man erhält 1690 mg (99% d. Th.) der Titelverbindung als farblosen Schaum.
LC-MS (Methode 11): Rₜ = 1.35 min; MS (ESIpos): m/z = 376 (M+H)⁺.

### Beispiel 7A

### 5-(2-{4-[2-Amino-6-({[2-(4-chlorphenyl)-1,3-thiazol-4-yl]methyl}thio)-3,5-dicyanopyridin-4-yl]-phenoxy}ethyl)-1-tert.-butyl-L-glutamat

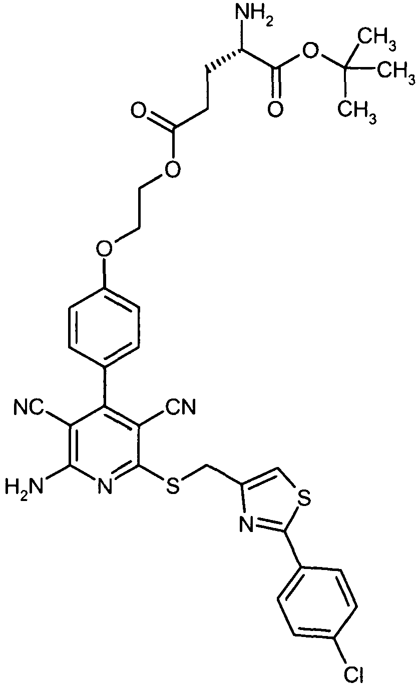

3.117 g (6 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 2 g (6.59 mmol) (4*S*)-5-*tert*.-Butoxy-4-[(*tert*.-butoxycar-bonyl)amino]-5-oxopentanoat, 1.38 g (7.19 mmol) 1-(3-Dimethylaminopropyl)-3-ethylcarbodiimid-Hydrochlorid sowie 0.073 g (0.6 mmol) 4-Dimethylaminopyridin werden in 80 ml Dichlormethan und 20 ml DMF zusammengegeben und über Nacht bei Raumtemperatur gerührt. Der Ansatz wird dann auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird aus Dichlormethan mit Petrolether gefällt. Das Produkt wird abgesaugt, mit Diethylether gewaschen und im Hochvakuum getrocknet. Es verbleiben 4.44 g (92% d. Th.) des geschützten Intermediats.
LC-MS (Methode 10): Rₜ = 3.38 min; MS (ESIpos): m/z = 805 (M+H)⁺.

57 mg (0.07 mmol) des erhaltenen Intermediats werden in 6 ml Dichlormethan und 0.6 ml wasserfreier Trifluoressigsäure aufgenommen und die Lösung 2.5 h bei Raumtemperatur gerührt. Der Ansatz wird dann auf eine Mischung aus halbgesättigter Natriumhydrogencarbonat-Lösung und Di-chlormethan gegossen. Die organische Phase wird abgetrennt, über Magnesiumsulfat getrocknet, filtriert und eingeengt. Nach Trocknen im Hochvakuum resultieren 50 mg (quant.) der Titelverbindung als farbloses Pulver.
HPLC (Methode 7): Rₜ = 5.4 min;
LC-MS (Methode 11): Rₜ = 1.72 min; MS (ESIpos): m/z = 705 (M+H)⁺.

### Beispiel 8A

### 2-{4-[2-Amino-6-({[2-(4-chlorphenyl)-1,3-thiazol-4-yl]methyl} sulfanyl)-3,5-dicyanopyridin-4-yl]-phenoxy}ethyl-L-leucinat-Trifluoracetat

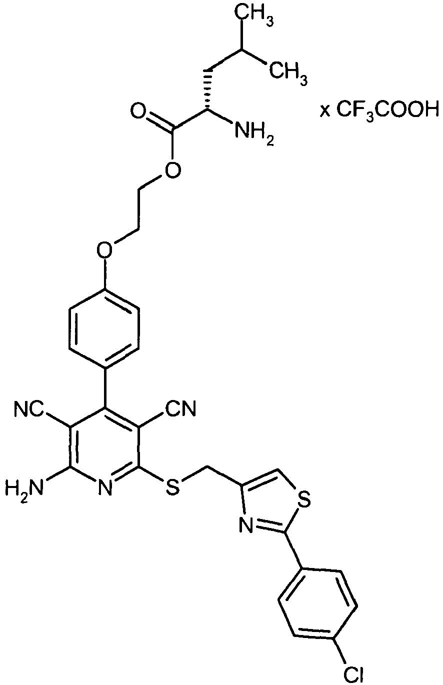

Die Herstellung der Titelverbindung erfolgt in Analogie zu Beispiel 1A ausgehend von 2-Amino-6-({[2-(4-chlorphenyl)-1,3-thiazol-4-yl]methyl}thio)-4-[4-(2-hydroxyethoxy)phenyl]pyridin-3,5-di-carbonitril und Boc-L-Leucin.
HPLC (Methode 7): Rₜ = 5.5 min;
LC-MS (Methode 12): Rₜ = 1.75 min; MS (ESIpos): m/z = 633 (M+H)⁺.

### Beispiel 9A

### 2-{4-[2-Amino-6-({[2-(4-chlorphenyl)-1,3-thiazol-4-yl]methyl}sulfanyl)-3,5-dicyanopyridin-4-yl]-phenoxy} ethyl-D-alaninat-Trifluoracetat

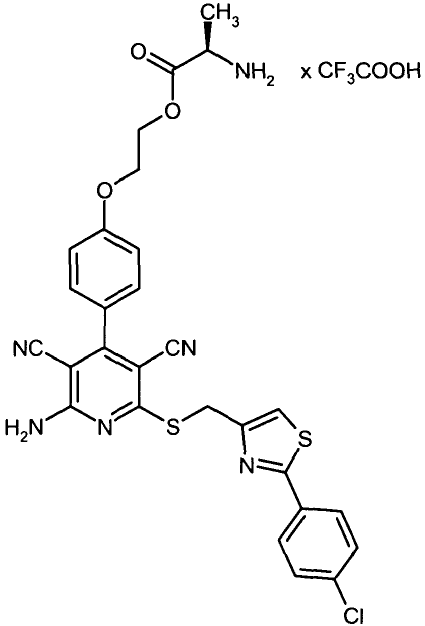

Die Herstellung der Titelverbindung erfolgt in Analogie zu Beispiel 2A ausgehend von 2-Amino-6-({[2-(4-chlorphenyl)-1,3-thiazol-4-yl]methyl}thio)-4-[4-(2-hydroxyethoxy)phenyl]pyridin-3,5-di-carbonitril und Boc-D-Alanin.
HPLC (Methode 7): Rₜ = 5.2 min;
LC-MS (Methode 13): Rₜ = 1.15 min; MS (ESIpos): m/z = 591 (M+H)⁺.

### Beispiel 10A

### N²-(tert.-Butoxycarbonyl)-N-{2-[(tert.-butoxycarbonyl)amino]ethyl}-D-α-glutamin

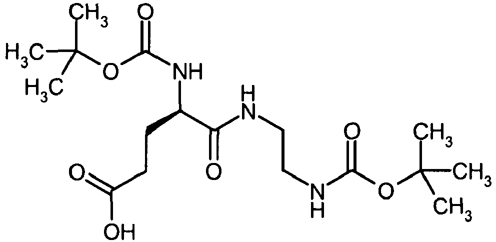

Die Herstellung der Titelverbindung erfolgt in Analogie zu Beispiel 4A ausgehend von (2R)-5-(Benzyloxy)-2-[(*tert*.-butoxycarbonyl)amino]-5-oxopentansäure.
HPLC (Methode 7): Rₜ = 4.4 min;
LC-MS (Methode 11): Rₜ = 1.37 min; MS (ESIpos): m/z = 390 (M+H)⁺.

### Beispiel 11A

### 2-{4-[2-Amino-6-({[2-(4-chlorphenyl)-1,3-thiazol-4-yl]methyl}sulfanyl)-3,5-dicyanopyridin-4-yl]-phenoxy} ethyl-glycinat-Trifluoracetat

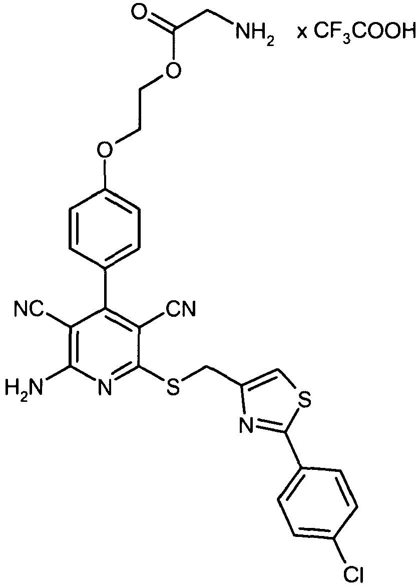

Die Herstellung der Titelverbindung erfolgt in Analogie zu Beispiel 2A ausgehend von 2-Amino-6-({[2-(4-chlorphenyl)-1,3-thiazol-4-yl]methyl}thio)-4-[4-(2-hydroxyethoxy)phenyl]pyridin-3,5-di-carbonitril und Boc-Glycin.
HPLC (Methode 7): Rₜ = 5.1 min;
LC-MS (Methode 13): Rₜ = 1.13 min; MS (ESIpos): m/z = 577 (M+H)⁺.

### Beispiel 12A

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-phenylalaninat-Trifluoracetat

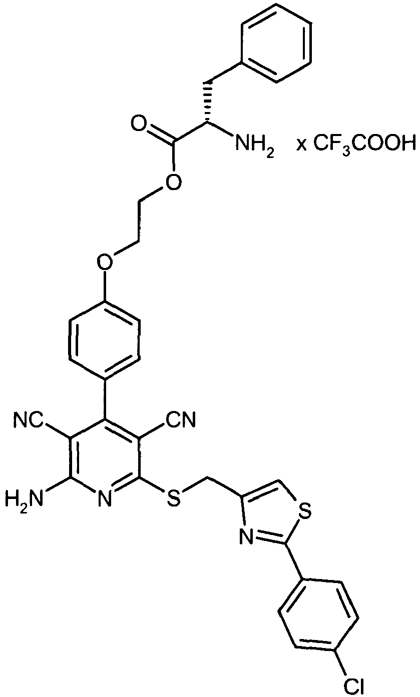

Die Herstellung der Titelverbindung erfolgt in Analogie zu Beispiel 2A ausgehend von 2-Amino-6-({[2-(4-chlorphenyl)-1,3-thiazol-4-yl]methyl}thio)-4-[4-(2-hydroxyethoxy)phenyl]pyridin-3,5-di-carbonitril und Boc-L-Phenylalanin.
HPLC (Methode 7): Rₜ = 5.1 min;
LC-MS (Methode 13): Rₜ = 1.13 min; MS (ESIpos): m/z = 577 (M+H)⁺.

### Ausführungsbeispiele:

### Beispiel 1

### 2-{4-[2-Amino-6-({[2-(4-chlorphenyl)-1,3-thiazol-4-yl]methyl}thio)-3,5-dicyanopyridin-4-yl]-phenoxy}ethyl-L-lysyl-L-valinat-Dihydrochlorid

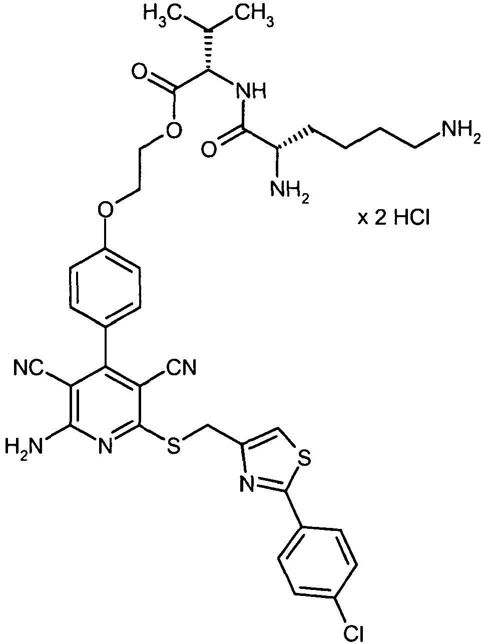

1.5 g (1.77 mmol) der Verbindung aus Beispiel 1A, 2.36 g (5.31 mmol) 2,5-Dioxopyrrolidin-1-yl-*N*²,*N*⁶-bis(*tert*.-butoxycarbonyl)-L-lysinat und 1.5 ml *N,N-*Diisopropylethylamin werden in 20 ml DMF zusammengegeben und über Nacht bei Raumtemperatur gerührt. Das Lösungsmittel wird dann im Vakuum entfernt und der Rückstand durch Flash-Chromatographie an Kieselgel mit Di-chlormethan/Ethylacetat als Eluent gereinigt (Gradient 3:1 → 2: 1). Die entsprechenden Fraktionen werden vereinigt und das Lösungsmittel im Vakuum entfernt. Nach Trocknen des Rückstands im Hochvakuum verbleiben 1.2 g (66% d. Th.) des geschützten Intermediats.
HPLC (Methode 7): Rₜ = 6.7 min.

1.2 g (1.27 mmol) des erhaltenen Intermediats werden in 3 ml Dichlormethan aufgenommen und unter Rühren mit 50 ml einer gesättigten Lösung von Chlorwasserstoff in Dichlormethan versetzt. Man rührt den Ansatz 30 min bei RT, wobei das Zielprodukt ausfällt. Das Lösungsmittel wird abgedampft und der verbleibende Rückstand mit 70 ml Diethylether verrührt. Man filtriert ab, wäscht den Filterrückstand mit Diethylether nach und trocknet anschließend im Hochvakuum. Es resultieren 893 mg (86% d. Th.) der Titelverbindung als farblose Kristalle.
HPLC (Methode 7): Rₜ = 5.1 min;
LC-MS (Methode 12): Rₜ = 1.47 min; MS (ESIpos): m/z = 747 (M+H)⁺;
¹H-NMR (400 MHz, DMSO-d₆): δ = 0.94 und 0.95 (2d, 6H), 1.4 (m, 2H), 1.55 (m, 2H), 1.75 (m, 2H), 2.14 (m, 1H), 2.7-2.8 (m, 2H), 3.95 (m, 1H), 4.3-4.5 (m, 2H), 4.65 (s, 2H), 7.12 (d, 2H), 7.51 (d, 2H), 7.58 (d, 2H), 7.95 (d, 2H), 7.97 (s, 1H), 8.0 (m, 2H), 8.3 (m, 2H), 8.8 (d, 1H).

### Beispiel 2

### 2-{4-[2-Amino-6-({[2-(4-chlorphenyl)-1,3-thiazol-4-yl]methyl} thio)-3,5-dicyanopyridin-4-yl]-phenoxy} ethyl-β-alanyl-L-valinat-Hydrochlorid

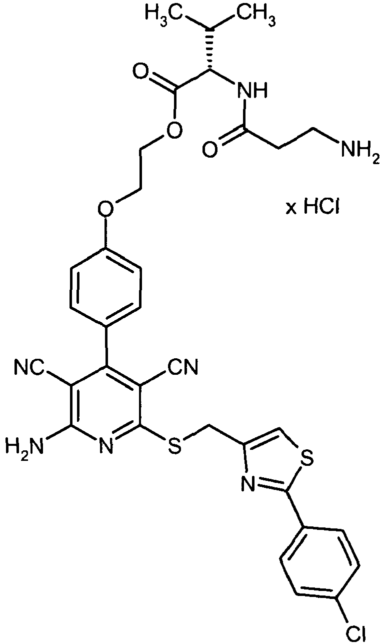

0.1 g (0.12 mmol) der Verbindung aus Beispiel 1A, 0.051 g (0.18 mmol) 2,5-Dioxopyrrolidin-1-yl-*N*-(*tert*.-butoxycarbonyl)-β-alaninat und 82 µl *N,N-*Diisopropylethylamin werden in 6 ml DMF zusammengegeben und über Nacht bei Raumtemperatur gerührt. Das Lösungsmittel wird dann im Vakuum entfernt und der Rückstand durch Flash-Chromatographie an Kieselgel mit Dichlor-methan/Ethylacetat als Eluent gereinigt (Gradient 4:1 → 3:1 → 2:1). Die entsprechenden Fraktionen werden vereinigt und das Lösungsmittel im Vakuum entfernt. Nach Trocknen des Rückstands im Hochvakuum verbleiben 0.052 g (56% d. Th.) des geschützten Intermediats.
HPLC (Methode 7): Rₜ = 6.3 min.

0.05 g (0.063 mmol) des erhaltenen Intermediats werden in 1 ml Dichlormethan aufgenommen und unter Rühren mit 15 ml einer gesättigten Lösung von Chlorwasserstoff in Dichlormethan versetzt. Man rührt den Ansatz 3 h bei RT, wobei die Zielverbindung ausfällt. Das Lösungsmittel wird abgedampft und der verbleibende Rückstand mit 10 ml Diethylether verrührt. Man filtriert ab, wäscht den Filterrückstand mit Diethylether nach und trocknet anschließend im Hochvakuum. Es resultieren 41 mg (85% d. Th.) der Titelverbindung als farblose Kristalle.
HPLC (Methode 7): Rₜ = 5.2 min;
LC-MS (Methode 12): Rₜ = 2.1 min; MS (ESIpos): m/z = 690 (M+H)⁺;
¹H-NMR (400 MHz, DMSO-d₆): δ = 0.89 und 0.9 (2d, 6H), 2.04 (m, 2H), 2.5 (m, 2H), 2.9-3.0 (m, 2H), 4.2 (m, 1H), 4.25 (m, 2H), 4.35-4.5 (m, 2H), 4.67 (s, 2H), 7.12 (d, 2H), 7.5 (d, 2H), 7.57 (d, 2H), 7.8 (m, 2H), 7.94 (s, 1H), 7.95 (d, 2H), 8.5 (d, 1H).

### Beispiel 3

### 2-{4-[2-Amino-6-({[2-(4-chlorphenyl)-1,3-thiazol-4-yl]methyl}thio)-3,5-dicyanopyridin-4-yl]-phenoxy} ethyl-L-arginyl-L-valinat-Dihydrochlorid

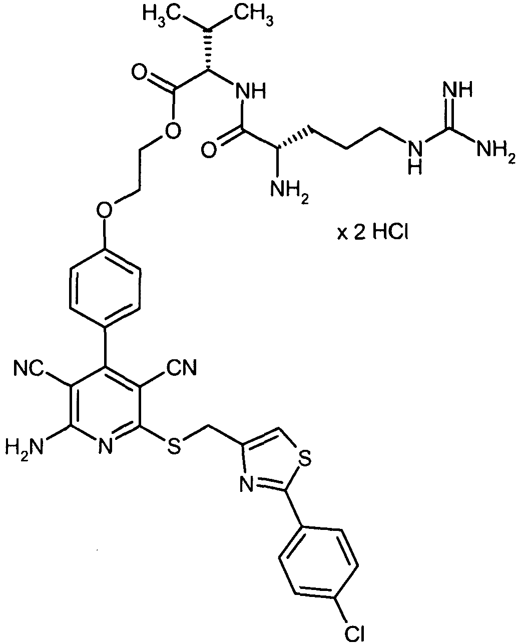

2.24 g (4.72 mmol) *N²*,*N⁵*-Bis(*tert*.-butoxycarbonyl)-*N⁵*-{[(*tert*.-butoxycarbonyl)amino](imino)-methyl}-L-ornithin, 0.96 g (7.08 mmol) 1-Hydroxy-1*H*-benzotriazol-Hydrat und 1.09 g (5.66 mmol) *N*-(3-Dimethylaminopropyl)-*N*'-ethylcarbodiimid-Hydrochlorid werden in 200 ml DMF zusammengegeben. Nach 30 min Rühren werden 2 g (2.36 mmol) der Verbindung aus Beispiel 1A sowie 1.65 ml *N,N-*Diisopropylethylamin hinzugefügt und der Ansatz über Nacht bei Raumtemperatur gerührt. Danach wird eingeengt und der verbleibende Rückstand mit Wasser verrührt. Man saugt ab und reinigt den Rückstand durch Flash-Chromatographie an Kieselgel mit Dichlormethan/Ethylacetat als Eluent (Gradient 4:1 → 3:1). Die entsprechenden Fraktionen werden vereinigt und das Lösungsmittel im Vakuum entfernt. Nach Trocknen des Rückstands im Hochvakuum verbleiben 1.12 g (44% d. Th.) des geschützten Intermediats.
HPLC (Methode 7): Rₜ = 6.1 min.

1.12 g (1.04 mmol) des Intermediats werden in 10 ml Dichlormethan aufgenommen, mit 10 ml wasserfreier Trifluoressigsäure versetzt und über Nacht bei RT gerührt. Man engt danach ein und zieht den Rückstand zweimal mit THF ab. Man filtriert ab und nimmt den Filterrückstand in einer Mischung aus 25 ml THF und 5 ml Methanol auf. Unter Rühren werden 20 ml einer 2 M Lösung von Chlorwasserstoff in Diethylether zugegeben. Nach kurzem Nachrühren wird der ausfallende Niederschlag abgesaugt und der Filterrückstand mit Diethylether gewaschen. Nach Trocknen im Hochvakuum verbleiben 920 mg (99% d. Th.) der Titelverbindung als farblose Kristalle.
HPLC (Methode 7): Rₜ = 5.1 min;
LC-MS (Methode 10): Rₜ = 1.7 min; MS (ESIpos): m/z = 775 (M+H)⁺;
¹H-NMR (400 MHz, DMSO-d₆): δ = 0.95 und 0.97 (2d, 6H), 1.6 (m, 2H), 1.75 (m, 2H), 2.14 (m, 1H), 3.25 (m, 2H), 4.05 (m, 1H), 4.25 (t, 1H), 4.3 (m, 2H), 4.4-4.5 (2m, 2H), 4.65 (s, 2H), 7.12 (d, 2H), 7.48 (d, 2H), 7.58 (d, 2H), 7.95 (m, 3H), 8.4 (m, 2H), 8.9 (d, 1H).

### Beispiel 4

### 2-{4-[2-Amino-6-({[2-(4-chlorphenyl)-1,3-thiazol-4-yl]methyl}thio)-3,5-dicyanopyridin-4-yl]-phenoxy} ethyl-L-lysyl-L-alaninat-Dihydrochlorid

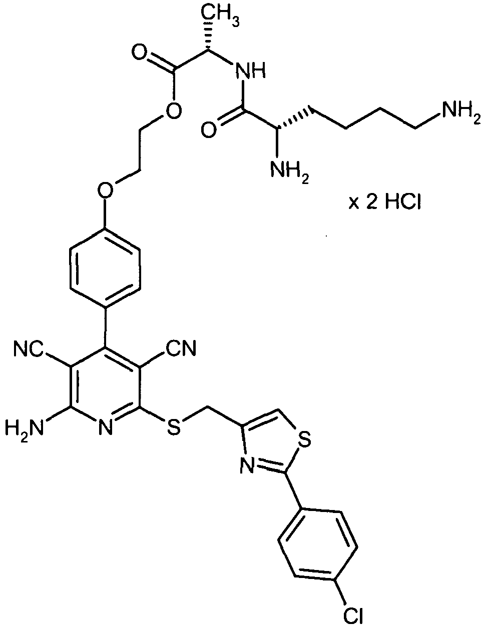

1.2 g (1.7 mmol) der Verbindung aus Beispiel 2A, 1.13 g (2.55 mmol) 2,5-Dioxopyrrolidin-1-yl-*N*²,*N⁶*-bis(*tert*.-butoxycarbonyl)-L-lysinat und 1.5 ml *N,N-*Diisopropylethylamin werden in 40 ml DMF zusammengegeben und über Nacht bei Raumtemperatur gerührt. Der Ansatz wird danach eingeengt und der Rückstand zwischen Ethylacetat und Wasser verteilt. Die organische Phase wird abgetrennt und nacheinander zweimal mit 5%-iger Zitronensäure und zweimal mit 5%-iger Natriumhydrogencarbonat-Lösung ausgeschüttelt. Die organische Phase wird dann eingeengt und der Rückstand durch Flash-Chromatographie an Kieselgel mit Dichlormethan/Ethylacetat als Eluent gereinigt (Gradient 3:1 → 2: 1). Die entsprechenden Fraktionen werden vereinigt und das Lösungsmittel im Vakuum entfernt. Der Rückstand wird mit 50 ml Diethylether sowie 50 ml Pentan verrührt und abgesaugt. Nach Trocknen des Rückstands im Hochvakuum verbleiben 1.14 g (73% d. Th.) des geschützten Intermediats.
HPLC (Methode 7): Rₜ = 6.4 min;
LC-MS (Methode 11): Rₜ = 2.64 min; MS (ESIpos): m/z = 919 (M+H)⁺.

1.14 g (1.24 mmol) des Intermediats werden in 10 ml Dichlormethan aufgenommen und unter Rühren mit 60 ml einer gesättigten Lösung von Chlorwasserstoff in Dichlormethan versetzt. Man rührt den Ansatz über Nacht bei RT, wobei die Zielverbindung ausfällt. Das Lösungsmittel wird auf ca. ein Drittel des Volumens eingeengt und die resultierende Suspension mit 200 ml Diethylether versetzt. Man filtriert ab, wäscht den Filterrückstand mit Diethylether nach und trocknet anschließend im Hochvakuum. Es resultieren 1.0 g (quant.) der Titelverbindung als farblose Kristalle.
HPLC (Methode 7): Rₜ = 5.0 min;
LC-MS (Methode 10): Rₜ = 1.67 min; MS (ESIpos): m/z = 719 (M+H)⁺;
¹H-NMR (400 MHz, DMSO-d₆): δ = 1.35 (d, 3H), 1.4 (m, 2H), 1.6 (m, 2H), 1.75 (m, 2H), 2.75 (m, 2H), 3.8 (m, 1H), 4.25 (m, 2H), 4.3-4.5 (m, 3H), 4.63 (s, 2H), 7.12 (d, 2H), 7.48 (d, 2H), 7.58 (d, 2H), 7.9-8.0 (m, 5H), 8.3 (m, 2H), 9.05 (d, 1H).

### Beispiel 5

### 2-{4-[2-Amino-6-({[2-(4-chlorphenyl)-1,3-thiazol-4-yl]methyl}thio)-3,5-dicyanopyridin-4-yl]-phenoxy} ethyl-L-lysyl-L-serinat-Dihydrochlorid

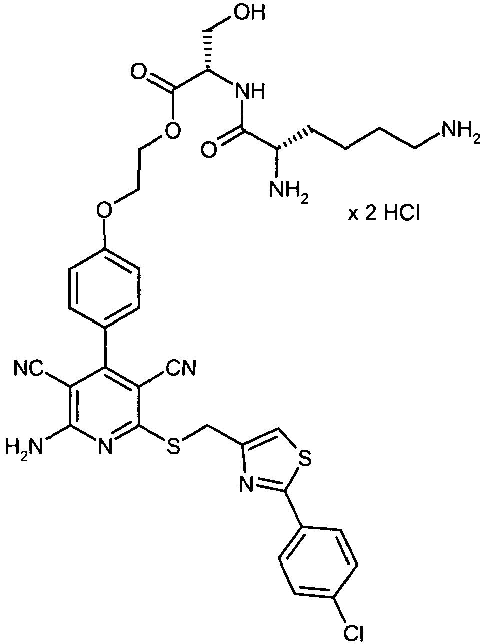

0.58 g (1.675 mmol) *N²*,*N⁶*-Bis(*tert*.-butoxycarbonyl)-L-lysin, 0.28 g (1.83 mmol) 1-Hydroxy-1*H* benzotriazol-Hydrat und 0.35 g (1.83 mmol) *N*-(3-Dimethylaminopropyl)-*N*'-ethylcarbodiimid-Hydrochlorid werden in 40 ml DMF zusammengegeben und dann mit 1.01 g (1.52 mmol) der Verbindung aus Beispiel 3A versetzt. Der Ansatz wird über Nacht bei Raumtemperatur gerührt und danach auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird durch Flash-Chromatographie an Kieselgel zunächst mit Dichlormethan/Ethylacetat als Eluent gereinigt (Gradient 3:1 → 2:1); anschließend wird mit Dichlormethan/Ethylacetat/Methanol (150:50:5) eluiert. Die entsprechenden Fraktionen werden vereinigt und eingeengt. Nach Trocknen des Rückstands im Hochvakuum verbleiben 1.23 g (81% d. Th.) des geschützten Intermediats.
HPLC (Methode 7): Rₜ = 6.5 min;
LC-MS (Methode 12): Rₜ = 2.99 min; MS (ESIpos): m/z = 991 (M+H)⁺.

1.216 g (1.48 mmol) des Intermediats werden in 6 ml Dichlormethan aufgenommen, mit 6 ml wasserfreier Trifluoressigsäure versetzt und über Nacht bei RT gerührt. Man engt danach ein und zieht den Rückstand nochmals mit Dichlormethan ab. Man filtriert ab und nimmt den Filterrückstand in einer Mischung aus 25 ml Dichlormethan und 25 ml Ethylacetat auf. Unter Rühren werden 20 ml einer 2 M Lösung von Chlorwasserstoff in Diethylether zugegeben. Nach kurzem Nachrühren wird der ausfallende Niederschlag abgesaugt, mit Diethylether gewaschen und im Hochvakuum getrocknet. Anschließend wird der Rückstand aus 20 ml Methanol und 20 ml Ethylacetat umkristallisiert. Der Niederschlag wird erneut abgesaugt, mit Ethylacetat gewaschen und im Hochvakuum getrocknet. Man erhält 845 mg (70% d. Th.) der Titelverbindung als farblose Kristalle.
HPLC (Methode 7): Rₜ = 4.9 min;
LC-MS (Methode 10): Rₜ = 1.62 min; MS (ESIpos): m/z = 735 (M+H)⁺.

### Beispiel 6

### 2-{4-[2-Amino-6-({[2-(4-chlorphenyl)-1,3-thiazol-4-yl]methyl}thio)-3,5-dicyanopyridin-4-yl]-phenoxy}ethyl-N-(2-aminoethyl)-L-α-glutaminat-Dihydrochlorid

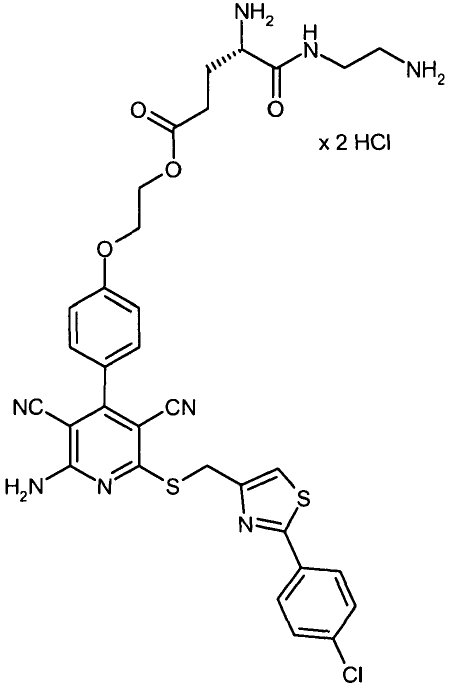

1 g (1.92 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 0.824 g (2.11 mmol) der Verbindung aus Beispiel 4A, 0.442 g (2.31 mmol) 1-(3-Dimethylaminopropyl)-3-ethylcarbodiimid-Hydrochlorid sowie 0.024 g (0.19 mmol) 4-Dimethylaminopyridin werden in 40 ml Dichlormethan und 10 ml DMF zusammengegeben und über Nacht bei Raumtemperatur gerührt. Der Ansatz wird dann auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird durch Flash-Chromatographie an Kieselgel zunächst mit Dichlormethan/ Ethylacetat (3: 1) als Eluent gereinigt; anschließend wird mit Dichlormethan/ Ethylacetat/Methanol eluiert (Gradient 300:100:5 → 300:100:10). Die entsprechenden Fraktionen werden vereinigt und das Lösungsmittel im Vakuum entfernt. Nach Trocknen des Rückstands im Hochvakuum verbleiben 1.52 g (89% d. Th.) des geschützten Intermediats.
HPLC (Methode 7): Rₜ = 6.0 min;
LC-MS (Methode 11): Rₜ = 2.54 min; MS (ESIpos): m/z = 891 (M+H)⁺.

1.518 g (1.7 mmol) des Intermediats werden in 5 ml Dichlormethan aufgenommen, mit 5 ml wasserfreier Trifluoressigsäure versetzt und 1 h bei RT gerührt. Man engt danach ein und zieht den Rückstand nochmals mit Dichlormethan ab. Der Rückstand wird dann in 20 ml Ethylacetat gelöst. Unter Rühren werden 20 ml einer 2 M Lösung von Chlorwasserstoff in Diethylether zugegeben. Nach kurzem Nachrühren wird abgesaugt und der Filterrückstand mit Diethylether gewaschen und getrocknet. Man erhält 1300 mg (99% d. Th.) der Titelverbindung. Anschließend wird aus 25 ml Methanol und 25 ml Ethylacetat umkristallisiert. Der Niederschlag wird erneut abgesaugt, mit Ethylacetat gewaschen und dann im Hochvakuum getrocknet. Es verbleiben 1080 mg (83% d. Th.) der Titelverbindung.
HPLC (Methode 7): Rₜ = 4.9 min;
LC-MS (Methode 10): Rₜ = 1.59 min; MS (ESIpos): m/z = 691 (M+H)⁺;
¹H-NMR (400 MHz, DMSO-d₆): δ = 2.05 (m, 2H), 2.45 (m, 2H), 2.85 und 2.95 (2m, 2H), 3.25 und 3.5 (2m, 2H), 3.7 (m, 1H), 4.3 (m, 2H), 4.4 (m, 2H), 4.65 (s, 2H), 7.12 (d, 2H), 7.48 (d, 2H), 7.58 (d, 2H), 7.93 (s, 1H), 7.94 (d, 2H), 8.1 (m, 3H), 8.45 (m, 3H), 8.95 (t, 1H).

### Beispiel 7

### 2-{4-[2-Amino-6-({[2-(4-chlorphenyl)-1,3-thiazol-4-yl]methyl}thio)-3,5-dicyanopyridin-4-yl]-phenoxy}ethyl-N-(2-aminoethyl)-L-glutaminat-Dihydrochlorid

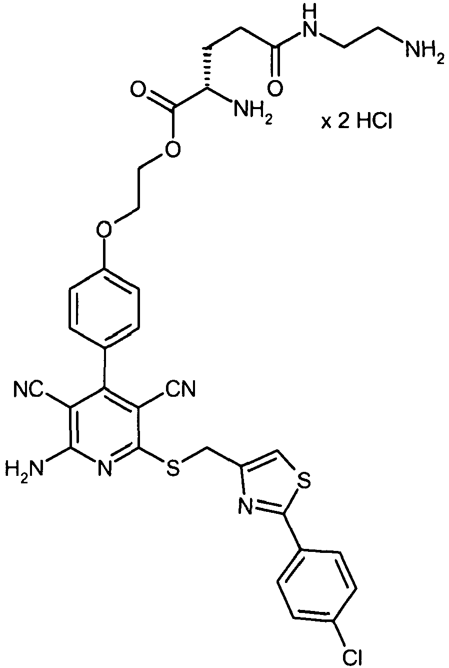

1 g (1.92 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 0.824 g (2.11 mmol) der Verbindung aus Beispiel 5A, 0.442 g (2.31 mmol) 1-(3-Dimethylaminopropyl)-3-ethylcarbodiimid-Hydrochlorid sowie 0.024 g (0.192 mmol) 4-Dimethylaminopyridin werden in 40 ml Dichlormethan und 10 ml DMF zusammengegeben und über Nacht bei Raumtemperatur gerührt. Der Ansatz wird danach auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird aus Dichlormethan mit Diethylether gefällt. Der Niederschlag wird abfiltriert, mit Diethylether gewaschen und im Hochvakuum getrocknet. Es verbleiben 1.5 g (87% d. Th.) des geschützten Intermediats.
HPLC (Methode 7): Rₜ = 6.0 min;
LC-MS (Methode 10): Rₜ = 3.12 min; MS (ESIpos): m/z = 891 (M+H)⁺.

1.5 g (1.7 mmol) des Intermediats werden in 20 ml Dichlormethan aufgenommen, mit 5 ml wasserfreier Trifluoressigsäure versetzt und 1 h bei RT gerührt. Man engt danach ein und zieht den Rückstand noch mehrmals mit Toluol ab. Der Rückstand wird dann in 15 ml Dichlormethan, 5 ml Ethylacetat und 1 ml Methanol aufgenommen. Unter Rühren werden 20 ml einer 2 M Lösung von Chlorwasserstoff in Diethylether zugegeben. Nach kurzem Nachrühren wird abgesaugt, der Filterrückstand zweimal mit Diethylether gewaschen und getrocknet. Der Rückstand wird dann in 50 ml verdünnter Salzsäure (pH 3) gelöst und lyophilisiert. Es verbleiben 1265 mg (98% d. Th.) der Titelverbindung.
HPLC (Methode 7): Rₜ = 4.9 min;
LC-MS (Methode 11): Rₜ = 1.19 min; MS (ESIpos): m/z = 691 (M+H)⁺.

### Beispiel 8

### 2-{4-[2-Amino-6-({[2-(4-chlorphenyl)-1,3-thiazol-4-yl]methyl}thio)-3,5-dicyanopyridin-4-yl]-phenoxy}ethyl-N-(2-aminoethyl)-L-asparaginat-Dihydrochlorid

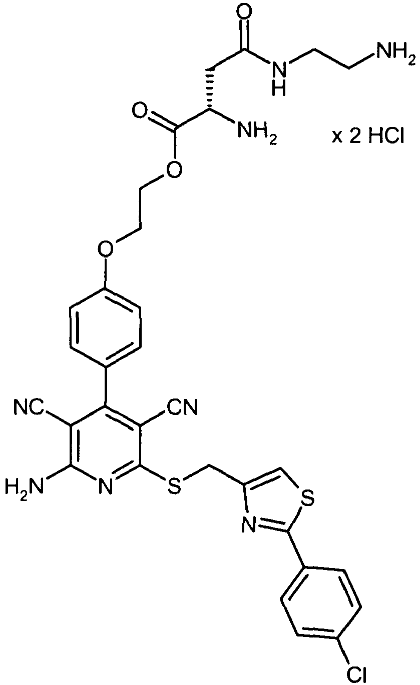

1 g (1.92 mmol) 2-Amino-6-({[2-(4-chlorphenyl)-1,3-thiazol-4-yl]methyl}thio)-4-[4-(2-hydroxy-ethoxy)phenyl]pyridin-3,5-dicarbonitril, 0.794 g (2.12 mmol) der Verbindung aus Beispiel 6A, 0.442 g (2.31 mmol) 1-(3-Dimethylaminopropyl)-3-ethylcarbodiimid-Hydrochlorid sowie 0.024 g (0.192 mmol) 4-Dimethylaminopyridin werden in 40 ml Dichlormethan und 10 ml DMF zusammengegeben und über Nacht bei Raumtemperatur gerührt. Der Ansatz wird danach auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird aus Dichlormethan mit Diethylether gefällt. Der Niederschlag wird abfiltriert, mit Diethylether gewaschen und im Hochvakuum getrocknet. Es verbleiben 1.28 g (74% d. Th.) des geschützten Intermediats.
HPLC (Methode 7): Rₜ = 6.0 min;
LC-MS (Methode 10): Rₜ = 3.13 min; MS (ESIpos): m/z = 877 (M+H)⁺.

1.28 g (1.46 mmol) des Intermediats werden in 20 ml Dichlormethan aufgenommen, mit 5 ml wasserfreier Trifluoressigsäure versetzt und 1 h bei RT gerührt. Man engt danach ein und zieht den Rückstand noch mehrmals mit Toluol ab. Der Rückstand wird dann in 15 ml Dichlormethan, 5 ml Ethylacetat und 1 ml Methanol aufgenommen. Unter Rühren werden 5 ml einer 2 M Lösung von Chlorwasserstoff in Diethylether zugegeben. Nach kurzem Nachrühren wird abgesaugt, der Filterrückstand zweimal mit Diethylether gewaschen und getrocknet. Es verbleiben 1096 mg (quant.) der Titelverbindung.
HPLC (Methode 7): Rₜ = 4.9 min;
LC-MS (Methode 10): Rₜ = 1.58 min; MS (ESIpos): m/z = 677 (M+H)⁺.

### Beispiel 9

### (2S)-5-(2-{4-[2-Amino-6-({[2-(4-chlorphenyl)-1,3-thiazol-4-yl]methyl}thio)-3,5-dicyanopyridin-4-yl]phenoxy} ethoxy)-2-[(3-carboxypropanoyl)amino]-5-oxopentansäure

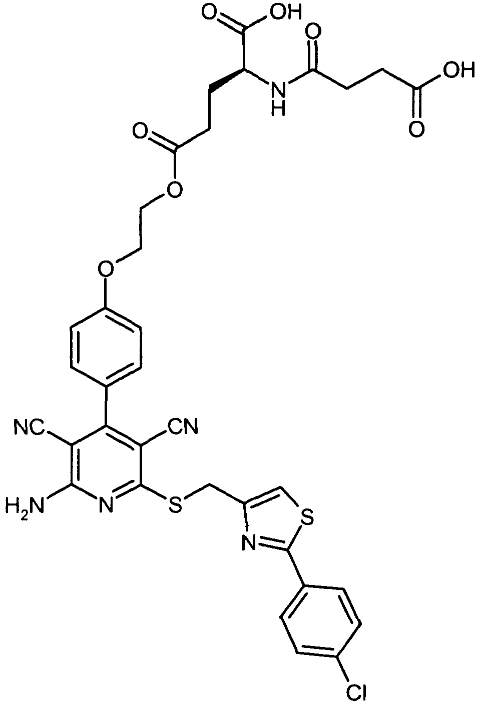

0.432 g (2.48 mmol) 4-*tert*.-Butoxy-4-oxobuttersäure, 0.475 g (2.48 mmol) 1-(3-Dimethylaminopropyl)-3-ethylcarbodiimid-Hydrochlorid, 0.380 g (2.48 mmol) 1-Hydroxy-1*H-*benzotriazol-Hydrat sowie 1.59 g (2.254 mmol) der Verbindung aus Beispiel 7A werden in 70 ml DMF zusammengegeben und über Nacht bei Raumtemperatur gerührt. Der Ansatz wird danach auf eine Mischung aus halbgesättigter Ammoniumchlorid-Lösung und Ethylacetat gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, fil-triert und eingeengt. Der Rückstand wird durch Flash-Chromatographie an Kieselgel mit Dichlormethan/Ethylacetat als Eluent gereinigt (Gradient 10:1 → 5:1 → 3:1). Die entsprechenden Fraktionen werden vereinigt und das Lösungsmittel im Vakuum entfernt. Anschließend wird der Rückstand nochmals portionsweise mittels präparativer HPLC (Methode 6b) gereinigt. Die entsprechenden Fraktionen werden vereinigt und das Lösungsmittel im Vakuum entfernt. Nach Trocknen des Rückstands im Hochvakuum verbleiben 1.63 g (84% d. Th.) des geschützten Intermediats.
HPLC (Methode 7): Rₜ = 6.3 min;
LC-MS (Methode 11): Rₜ = 2.71 min; MS (ESIpos): m/z = 861 (M+H)⁺.

1.285 g (1.49 mmol) des Intermediats werden in 10 ml Dichlormethan aufgenommen, mit 10 ml wasserfreier Trifluoressigsäure versetzt und 1 h bei RT gerührt. Man engt danach ein und zieht den Rückstand noch mehrmals mit Toluol ab. Der verbleibende Rückstand wird dann mit Diethylether verrührt, der ausfallende Feststoff abgesaugt und mit Diethylether gewaschen. Nach Trocknen im Hochvakuum verbleiben 1.06 g (95% d. Th.) der Titelverbindung.
HPLC (Methode 7): Rₜ = 5.4 min;
LC-MS (Methode 10): Rₜ = 2.23 min; MS (ESIpos): m/z = 749 (M+H)⁺.

### Beispiel 10

### (2S)-5-(2-{4-[2-Amino-6-({[2-(4-chlorphenyl)-1,3-thiazol-4-yl]methyl)thio)-3,5-dicyanopyridin-4-yl]phenoxy}ethoxy)-2-[(3-carboxypropanoyl)amino]-5-oxopentansäure-Dinatrium-Salz

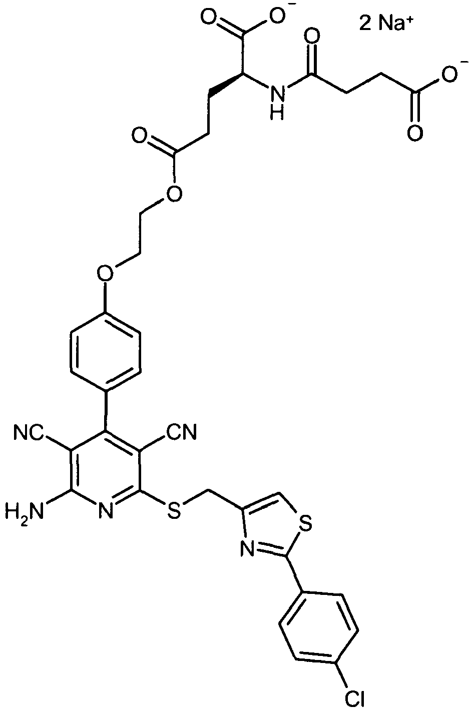

0.5 g (0.667 mmol) der Verbindung aus Beispiel 9 werden in 12.5 ml Acetonitril und 62.5 ml Wasser gelöst und mit 13 ml 0.1 N Natronlauge versetzt. Nach kurzem Rühren wird der Ansatz lyophilisiert. Nach Trocknen im Hochvakuum verbleiben 0.53 g (quant.) der Titelverbindung.
HPLC (Methode 7): Rₜ = 5.4 min;
LC-MS (Methode 11): Rₜ = 2.06 min; MS (ESIpos): m/z = 749 (M+H)⁺.

### Beispiel 11

### 2-{4-[2-Amino-6-({[2-(4-chlorphenyl)-1,3-thiazol-4-yl]methyl}sulfanyl)-3,5-dicyanopyridin-4-yl]-phenoxy} ethyl-L-lysyl-L-leucinat-Dihydrochlorid

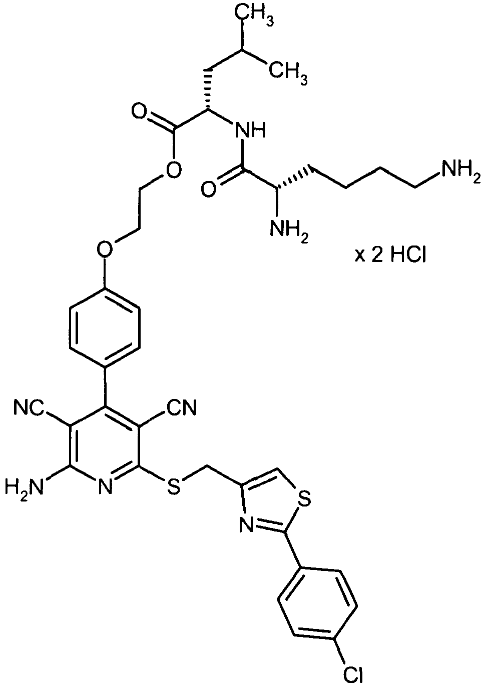

0.928 g (2.68 mmol) *N*²,*N*⁶-Bis(*tert*.-butoxycarbonyl)-L-lysin, 0.362 g (2.68 mmol) 1-Hydroxy-1*H-*benzotriazol-Hydrat und 0.411 g (2.144 mmol) *N*-(3-Dimethylaminopropyl)-*N*'-ethylcarbodiimid-Hydrochlorid werden in 200 ml DMF zusammengegeben. Dann werden 1.335 g (1.787 mmol) der Verbindung aus Beispiel 8A sowie 935 µl *N,N*-Diisopropylethylamin hinzugefügt und der Ansatz über Nacht bei Raumtemperatur gerührt. Anschließend wird im Vakuum eingeengt, der Rückstand in Ethylacetat aufgenommen und nacheinander mit Wasser, mit 5%-iger Zitronensäure und zweimal mit 5%-iger Natriumhydrogencarbonat-Lösung ausgeschüttelt. Die organische Phase wird eingeengt und der Rückstand durch Flash-Chromatographie an Kieselgel mit Dichlormethan/ Ethylacetat als Eluent gereinigt (Gradient 3:1 → 2:1). Die entsprechenden Fraktionen werden vereinigt und das Lösungsmittel im Vakuum entfernt. Nach Trocknen des Rückstands im Hochvakuum verbleiben 1.39 g (81% d. Th.) des geschützten Intermediats.
HPLC (Methode 7): Rₜ = 6.8 min.

1.385 g (1.44 mmol) des erhaltenen Intermediats werden in 20 ml Dichlormethan aufgenommen und unter Rühren mit 50 ml einer gesättigten Lösung von Chlorwasserstoff in Dichlormethan versetzt. Man rührt den Ansatz 1 h bei RT, wobei das Zielprodukt ausfällt. Das Lösungsmittel wird abgedampft und der verbleibende Rückstand mit 70 ml Pentan versetzt, kurz gerührt und dann abgesaugt. Nach Trocknen im Hochvakuum resultieren 1.17 g (97% d. Th.) der Titelverbindung.
HPLC (Methode 7): Rₜ = 5.17 min;
LC-MS (Methode 10): Rₜ = 1.76 min; MS (ESIpos): m/z = 761 (M+H)⁺.

### Beispiel 12

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-lysyl-D-alaninat-Dihydrochlorid

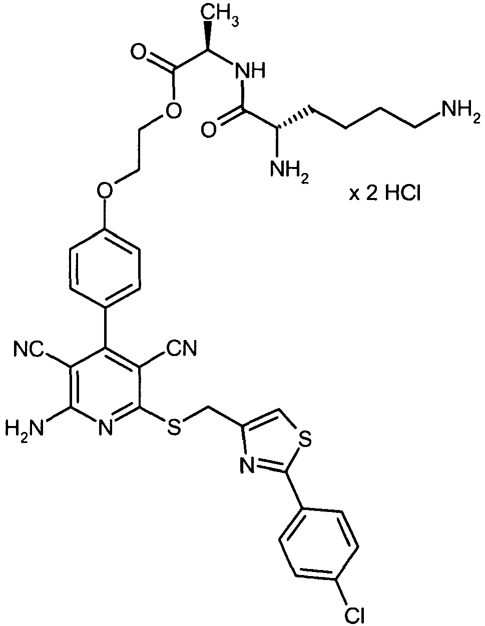

0.59 g (1.702 mmol) *N*²,*N*⁶-Bis(*tert*.-butoxycarbonyl)-L-lysin, 0.345 g (2.553 mmol) 1-Hydroxy-1*H*-benzotriazol-Hydrat und 0.391 g (2.042 mmol) *N*-(3-Dimethylaminopropyl)-*N*'-ethylcarbodiimid-Hydrochlorid werden in 24 ml DMF zusammengegeben. Nach 5 min Rühren bei RT werden 1.2 g (1.702 mmol) der Verbindung aus Beispiel 9A sowie 1.5 ml *N,N*-Diisopropylethylamin hinzugefügt und der Ansatz über Nacht bei Raumtemperatur gerührt. Anschließend wird eingeengt und der Rückstand zwischen 500 ml Ethylacetat und 500 ml Wasser verteilt. Die organische Phase wird abgetrennt und nacheinander dreimal mit 5%-iger Zitronensäure und dreimal mit 10%-iger Natriumhydrogencarbonat-Lösung ausgeschüttelt. Die organische Phase wird dann eingeengt und der Rückstand durch Flash-Chromatographie an Kieselgel mit Dichlormethan/Ethylacetat als Eluent gereinigt (Gradient 2:1 → 1:1). Die entsprechenden Fraktionen werden vereinigt und das Lösungsmittel im Vakuum entfernt. Der Rückstand wird mit 30 ml Ethylacetat verrührt und anschließend mit 100 ml Diethylether versetzt. Man saugt ab und wäscht den verbleibenden Rückstand mit Diethylether. Nach Trocknen im Hochvakuum verbleiben 0.773 g (49% d. Th.) des geschützten Intermediats.
HPLC (Methode 7): Rₜ = 6.1 min.

0.74 g (0.805 mmol) des erhaltenen Intermediats werden in 200 ml Dichlormethan aufgenommen. In diese Lösung wird unter Rühren Chlorwasserstoff-Gas eingeleitet. Dabei fällt die entschützte Titelverbindung aus. Es wird weiter bei RT gerührt, und nach 1 h ist die Reaktion abgeschlossen. Man engt den Ansatz im Vakuum auf die Hälfte des Volumens ein und filtriert den Niederschlag ab. Der Filterrückstand wird mit Diethylether gewaschen und anschließend im Hochvakuum bei 100°C getrocknet. Man erhält so 539 mg (85% d. Th.) der Titelverbindung als farblose Kristalle.
HPLC (Methode 7): Rₜ = 5.0 min;
LC-MS (Methode 13): Rₜ = 1.04 min; MS (ESIpos): m/z = 719 (M+H)⁺;
¹H-NMR (400 MHz, DMSO-d₆): δ = 1.3 (d, 3H), 1.35 (m, 2H), 1.52 (m, 2H), 1.75 (m, 2H), 2.75 (m, 2H), 3.8 (m, 1H), 4.3 (m, 2H), 4.3-4.5 (m, 3H), 4.63 (s, 2H), 7.12 (d, 2H), 7.48 (d, 2H), 7.58 (d, 2H), 7.8-8.0 (m, 5H), 8.25 (m, 2H), 9.0 (d, 1H).

### Beispiel 13

### 2-{4-[2-Amino-6-({[2-(4-chlorphenyl)-1,3-thiazol-4-yl]methyl}sulfanyl)-3,5-dicyanopyridin-4-yl]-phenoxy} ethyl-N-(2-aminoethyl)-D-α-glutaminat-Dihydrochlorid

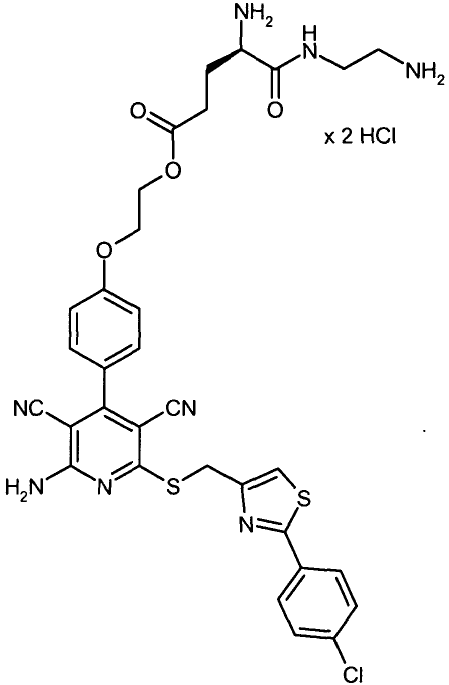

0.522 g (1.0 mmol) 2-Amino-6-({[2-(4-chlorphenyl)-1,3-thiazol-4-yl]methyl}thio)-4-[4-(2-hydroxyethoxy)phenyl]pyridin-3,5-dicarbonitril, 0.430 g (1.104 mmol) der Verbindung aus Beispiel 10A, 0.231 g (1.204 mmol) 1-(3-Dimethylaminopropyl)-3-ethylcarbodiimid-Hydrochlorid sowie 0.012 g (0.1 mmol) 4-Dimethylaminopyridin werden in 20 ml Dichlormethan und 5 ml DMF zusammengegeben und über Nacht bei Raumtemperatur gerührt. Der Ansatz wird danach auf eine Mischung aus halbgesättigter Ammoniumchlorid-Lösung und Dichlormethan gegossen. Die organische Phase wird abgetrennt, nacheinander mit Wasser, gesättigter Natriumhydrogencarbonat-Lösung und gesättigter Natriumchlorid-Lösung gewaschen, über Magnesiumsulfat getrocknet, filtriert und eingeengt. Der Rückstand wird durch Flash-Chromatographie an Kieselgel zunächst mit Dichlormethan/Ethylacetat (3: 1) als Eluent gereinigt; anschließend wird mit Dichlormethan/Ethyl-acetat/Methanol eluiert (Gradient 300:100:5 → 300:100:10). Die entsprechenden Fraktionen werden vereinigt und das Lösungsmittel im Vakuum entfernt. Nach Trocknen des Rückstands im Hochvakuum verbleiben 0.711 g (79% d. Th.) des geschützten Intermediats.
HPLC (Methode 7): Rₜ = 6.0 min;
LC-MS (Methode 13): Rₜ = 1.54 min; MS (ESIpos): m/z = 891 (M+H)⁺.

0.711 g (0.798 mmol) des erhaltenen Intermediats werden in 4 ml Dichlormethan aufgenommen, mit 4 ml wasserfreier Trifluoressigsäure versetzt und 1 h bei RT gerührt. Man engt danach ein und zieht den Rückstand nochmals mit Dichlormethan ab. Der Rückstand wird dann in 50 ml Ethylacetat gelöst. Unter Rühren werden 20 ml einer 2 M Lösung von Chlorwasserstoff in Diethylether zugegeben. Nach kurzem Nachrühren wird abgesaugt und der Filterrückstand mit Diethylether gewaschen und getrocknet. Man erhält 590 mg (97% d. Th.) der Titelverbindung.
HPLC (Methode 7): Rₜ = 4.9 min;
LC-MS (Methode 11): Rₜ = 1.22 min; MS (ESIpos): m/z = 691 (M+H)⁺;
¹H-NMR (400 MHz, DMSO-d₆): δ = 2.05 (m, 2H), 2.45 (m, 2H), 2.85 und 2.95 (2m, 2H), 3.25 und 3.5 (2m, 2H), 3.7 (m, 1H), 4.3 (m, 2H), 4.4 (m, 2H), 4.65 (s, 2H), 7.12 (d, 2H), 7.48 (d, 2H), 7.58 (d, 2H), 7.93 (s, 1H), 7.94 (d, 2H), 8.1 (m, 3H), 8.45 (m, 3H), 8.93 (t, 1H).

### Beispiel 14

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-arginyl-L-alaninat-Dihydrochlorid

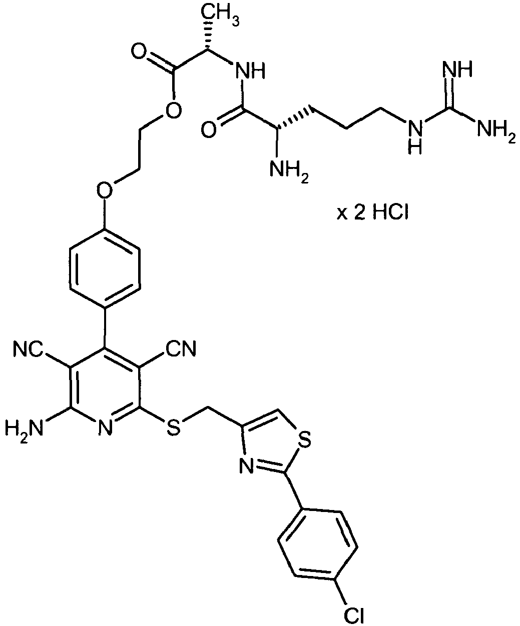

0.269 g (0.567 mmol) *N*⁵ -[*N,N*'-Bis(*tert*.-butoxycarbonyl)carbamidoyl]-*N*²-(*tert*.-butoxycarbonyl)-L-omithin, 0.115 g (0.851 mmol) 1-Hydroxy-1*H*-benzotriazol-Hydrat und 0.131 g (0.681 mmol) *N-*(3-Dimethylaminopropyl)-*N*'-ethylcarbodiimid-Hydrochlorid werden in 20 ml DMF zusammengegeben. Nach 30 min Rühren werden 0.2 g (0.284 mmol) der Verbindung aus Beispiel 2A sowie 200 µl *N,N*-Diisopropylethylamin hinzugefügt und der Ansatz über Nacht bei Raumtemperatur gerührt. Anschließend wird im Vakuum eingeengt, der Rückstand in Dichlormethan aufgenommen und nacheinander mit 5%-iger Zitronensäure, 5%-iger Natriumhydrogencarbonat-Lösung und Wasser ausgeschüttelt. Die organische Phase wird eingeengt und der Rückstand durch Flash-Chromatographie an Kieselgel mit Dichlormethan/Ethylacetat (3:1) als Eluent gereinigt. Die entsprechenden Fraktionen werden vereinigt und das Lösungsmittel im Vakuum entfernt. Nach Trocknen des Rückstands im Hochvakuum verbleiben 0.179 g (60% d. Th.) des geschützten Intermediats.
HPLC (Methode 7): Rₜ = 5.8 min.

0.178 g (0.17 mmol) des erhaltenen Intermediats werden in 30 ml einer gesättigten Lösung von Chlorwasserstoff in Dichlormethan aufgenommen und über Nacht bei RT gerührt. Man engt den Ansatz im Vakuum auf die Hälfte des Volumens ein und filtriert den entstandenen Niederschlag ab. Der Filterrückstand wird mit Diethylether gewaschen und anschließend im Hochvakuum getrocknet. Man erhält so 119 mg (82% d. Th.) der Titelverbindung als farblose Kristalle.
HPLC (Methode 7): Rₜ = 4.9 min;
LC-MS (Methode 10): Rₜ = 1.58 min; MS (ESIpos): m/z = 747 (M+H)⁺;
¹H-NMR (400 MHz, DMSO-d₆): δ = 1.35 (d, 3H), 1.55 (m, 2H), 1.75 (m, 2H), 3.25 (m, 2H), 3.85 (m, 1H), 4.25 (m, 2H), 4.3-4.5 (m, 3H), 4.65 (s, 2H), 7.12 (d, 2H), 7.48 (d, 2H), 7.58 (d, 2H), 7.75 (t, 1H), 7.93 (s, 1H), 7.94 (d, 2H), 8.3 (m, 3H), 9.1 (d, 1H).

### Beispiel 15

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-histidyl-L-alaninat-Dihydrochlorid

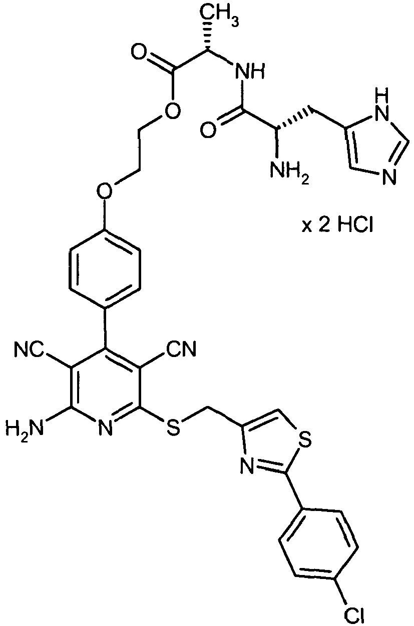

0.145 g (0.567 mmol) N-(*tert*.-Butoxycarbonyl)-L-histidin, 0.115 g (0.851 mmol) 1-Hydroxy-1*H-*benzotriazol-Hydrat und 0.131 g (0.681 mmol) *N*-(3-Dimethylaminopropyl)-*N*'-ethylcarbodiimid-Hydrochlorid werden in 20 ml DMF zusammengegeben. Nach 30 min Rühren werden 0.2 g (0.284 mmol) der Verbindung aus Beispiel 2A sowie 200 µl *N,N-*Diisopropylethylamin hinzugefügt und der Ansatz über Nacht bei Raumtemperatur gerührt. Anschließend wird im Vakuum eingeengt, der Rückstand in Dichlormethan aufgenommen und nacheinander mit 5%-iger Zitronensäure, 5%-iger Natriumhydrogencarbonat-Lösung und Wasser ausgeschüttelt. Die organische Phase wird eingeengt und der Rückstand durch Flash-Chromatographie an Kieselgel mit Toluol/Ethanol (3: 1) als Eluent gereinigt. Die entsprechenden Fraktionen werden vereinigt und das Lösungsmittel im Vakuum entfernt. Nach Trocknen des Rückstands im Hochvakuum verbleiben 0.206 g (88% d. Th.) des geschützten Intermediats.
HPLC (Methode 7): Rₜ = 5.3 min.

0.206 g (0.249 mmol) des erhaltenen Intermediats werden in 30 ml einer gesättigten Lösung von Chlorwasserstoff in Dichlormethan aufgenommen und 2 h bei RT gerührt. Man engt den Ansatz im Vakuum auf die Hälfte des Volumens ein und filtriert den entstandenen Niederschlag ab. Der Filterrückstand wird mit Diethylether gewaschen und anschließend im Hochvakuum getrocknet. Man erhält so 171 mg (90% d. Th.) der Titelverbindung als farblose Kristalle.
HPLC (Methode 7): Rₜ = 4.8 min;
LC-MS (Methode 10): Rₜ = 1.59 min; MS (ESIpos): m/z = 728 (M+H)⁺;
¹H-NMR (400 MHz, DMSO-d₆): δ = 1.35 (d, 3H), 3.15 und 3.25 (2dd, 2H), 4.25 (m, 3H), 4.3-4.5 (m, 3H), 4.65 (s, 2H), 7.12 (d, 2H), 7.4 (s, 1H), 7.48 (d, 2H), 7.58 (d, 2H), 7.93 (s, 1H), 7.94 (d, 2H), 8.5 (m, 3H), 9.05 (s, 1H), 9.2 (d, 1H).

### Beispiel 16

### 2-{4-[2-Amino-6-({[2-(4-chlorphenyl)-1,3-thiazol-4-yl]methyl}sulfanyl)-3,5-dicyanopyridin-4-yl]-phenoxy} ethyl-N-[(2S)-2,4-diaminobutanoyl]-L-alaninat-Dihydrochlorid

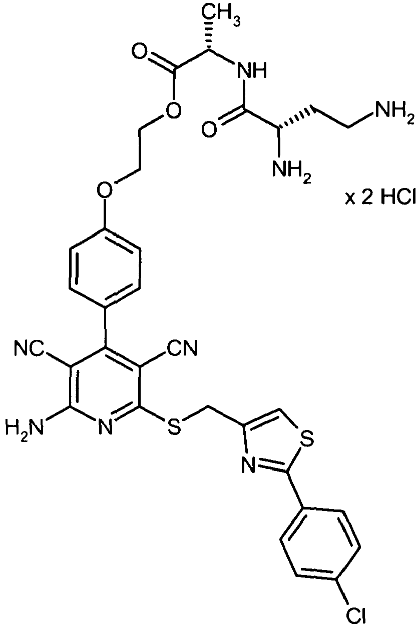

0.181 g (0.567 mmol) (2*S*)-2,4-Bis[(*tert*.-butoxycarbonyl)amino]buttersäure, 0.115 g (0.851 mmol) 1-Hydroxy-1*H*-benzotriazol-Hydrat und 0.131 g (0.681 mmol) *N*-(3-Dimethylaminopropyl)-*N*'-ethylcarbodiimid-Hydrochlorid werden in 20 ml DMF zusammengegeben. Nach 30 min Rühren bei RT werden 0.2 g (0.284 mmol) der Verbindung aus Beispiel 2A sowie 200 µl *N,N-*Diisopropyl-ethylamin hinzugefügt und der Ansatz über Nacht bei Raumtemperatur gerührt. Anschließend wird im Vakuum eingeengt, der Rückstand in Dichlormethan aufgenommen und nacheinander mit 5%-iger Zitronensäure, 5%-iger Natriumhydrogencarbonat-Lösung und Wasser ausgeschüttelt. Die organische Phase wird eingeengt und der Rückstand durch Flash-Chromatographie an Kieselgel mit Toluol/Ethanol (10:1) als Eluent gereinigt. Die entsprechenden Fraktionen werden vereinigt und das Lösungsmittel im Vakuum entfernt. Nach Trocknen des Rückstands im Hochvakuum verbleiben 0.217 g (86% d. Th.) des geschützten Intermediats.
HPLC (Methode 7): Rₜ = 6.3 min.

0.212 g (0.238 mmol) des erhaltenen Intermediats werden in 25 ml einer gesättigten Lösung von Chlorwasserstoff in Dichlormethan aufgenommen und 1.5 h bei RT gerührt. Man engt den Ansatz im Vakuum auf die Hälfte des Volumens ein und filtriert den entstandenen Niederschlag ab. Der Filterrückstand wird mit Diethylether gewaschen und anschließend im Hochvakuum getrocknet. Man erhält so 171 mg (94% d. Th.) der Titelverbindung als farblose Kristalle.
HPLC (Methode 7): Rₜ = 4.8 min;
LC-MS (Methode 10): Rₜ = 1.56 min; MS (ESIpos): m/z = 691 (M+H)⁺.

### Beispiel 17

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-lysyl-glycinat-Dihydrochlorid

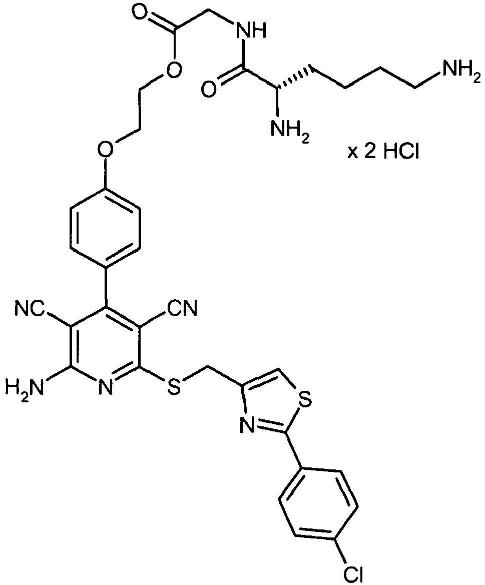

0.185 g (0.535 mmol) *N*²,*N*⁶-Bis(*tert*.-butoxycarbonyl)-L-Iysin, 0.108 g (0.803 mmol) 1-Hydroxy-1*H*-benzotriazol-Hydrat und 0.123 g (0.642 mmol) *N*-(3-Dimethylaminopropyl)-*N*'-ethylcarbodiimid-Hydrochlorid werden in 15 ml DMF zusammengegeben und 5 min bei RT gerührt. Dann werden 0.37 g (0.535 mmol) der Verbindung aus Beispiel 11A sowie 466 µl *NN-*Diisopropylethyl-amin hinzugefügt und der Ansatz über Nacht bei Raumtemperatur gerührt. Anschließend wird im Vakuum eingeengt und der Rückstand zwischen 500 ml Ethylacetat und 200 ml Wasser verteilt. Die organische Phase wird abgetrennt, nacheinander dreimal mit 5%-iger Zitronensäure und dreimal mit 5%-iger Natriumhydrogencarbonat-Lösung ausgeschüttelt und über Magnesiumsulfat getrocknet. Die organische Phase wird eingeengt und der Rückstand 10 min mit 50 ml Ethylacetat verrührt. Dann setzt man langsam 100 ml Diethylether zu und saugt den Niederschlag ab. Nach Trocknen im Hochvakuum verbleiben 0.303 g (63% d. Th.) des geschützten Intermediats.
HPLC (Methode 7): Rₜ = 6.1 min.

0.279 g (0.308 mmol) des erhaltenen Intermediats werden in 120 ml Dichlormethan aufgenommen. In diese Lösung wird unter Rühren Chlorwasserstoff-Gas eingeleitet, wobei die entschützte Titelverbindung ausfällt. Man rührt den Ansatz noch 1 h bei RT nach. Dann engt man im Vakuum auf die Hälfte des Volumens ein und setzt langsam 30 ml absolutes THF hinzu. Man rührt weitere 15 min und filtriert den Niederschlag dann ab. Der Filterrückstand wird mit Diethylether gewaschen und anschließend im Hochvakuum getrocknet. Man erhält so 212 mg (88% d. Th.) der Titelverbindung als farblose Kristalle.
HPLC (Methode 7): Rₜ = 4.8 min;
LC-MS (Methode 10): Rₜ = 1.52 min; MS (ESIpos): m/z = 705 (M+H)⁺.

### Beispiel 18

### 2-{4-[2-Amino-6-({[2-(4-chlorphenyl)-1,3-thiazol-4-yl]methyl}sulfanyl)-3,5-dicyanopyridin-4-yl]-phenoxy}ethyl-L-lysyl-L-phenylalaninat-Dihydrochlorid

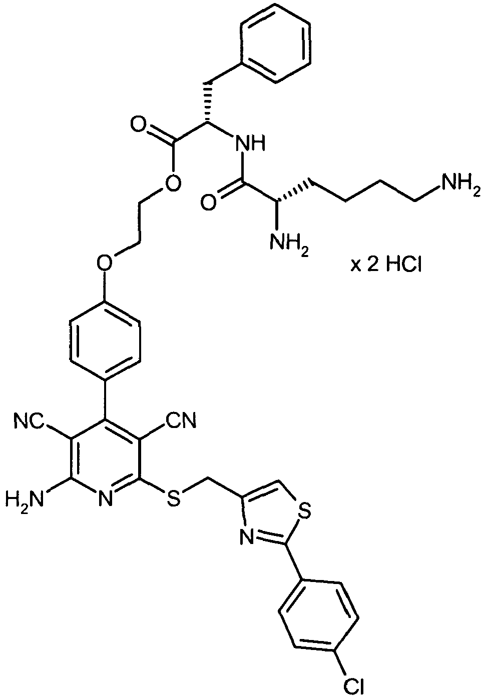

0.166 g (0.48 mmol) *N²*,*N⁶*-Bis(*tert*.-butoxycarbonyl)-L-lysin, 0.097 g (0.72 mmol) 1-Hydroxy-1*H-*benzotriazol-Hydrat und 0.110 g (0.576 mmol) *N*-(3-Dimethylaminopropyl)-*N*'-ethylcarbodiimid-Hydrochlorid werden in 7 ml DMF zusammengegeben und 5 min bei RT gerührt. Dann werden 0.375 g (0.48 mmol) der Verbindung aus Beispiel 12A sowie 418 µl *N,N-*Diisopropylethylamin hinzugefügt und der Ansatz über Nacht bei Raumtemperatur gerührt. Anschließend wird im Vakuum eingeengt, der Rückstand in 200 ml Ethylacetat aufgenommen, nacheinander zweimal mit 10%-iger Zitronensäure und zweimal mit 1 0%-iger Natriumhydrogencarbonat-Lösung ausgeschüttelt und über Magnesiumsulfat getrocknet. Die organische Phase wird eingeengt, der Rückstand in 10 ml Ethylacetat aufgenommen und das Produkt durch Zugabe von Diethylether gefällt. Man saugt den Niederschlag ab und trocknet im Hochvakuum. Es verbleiben 0.338 g (71% d. Th.) des geschützten Intermediats.
HPLC (Methode 7): Rₜ = 6.7 min.

0.32 g (0.321 mmol) des erhaltenen Intermediats werden in 100 ml Dichlormethan aufgenommen. In diese Lösung wird unter Rühren Chlorwasserstoff-Gas eingeleitet, wobei die entschützte Titelverbindung ausfällt. Man rührt den Ansatz noch 1 h bei RT nach, engt dann im Vakuum auf die Hälfte des Volumens ein und setzt langsam 10 ml absolutes THF hinzu. Man rührt weitere 15 min und filtriert den Niederschlag dann ab. Der Filterrückstand wird mit Diethylether gewaschen und anschließend im Hochvakuum getrocknet. Man erhält so 188 mg (67% d. Th.) der Titelverbindung als farblose Kristalle.
HPLC (Methode 7): Rₜ = 5.0 min;
LC-MS (Methode 11): Rₜ = 1.33 min; MS (ESIpos): m/z = 795 (M+H)⁺.

### B. Bestimmung von Löslichkeit, Stabilität und Freisetzungsverhalten

### a) Bestimmung der Löslichkeit:

Die Prüfsubstanz wird in 5%-iger wässriger Dextrose-Lösung suspendiert. Diese Suspension wird 24 h bei Raumtemperatur geschüttelt. Nach Ultra-Zentrifugation bei 224000g für 30 min wird der Überstand mit DMSO verdünnt und per HPLC analysiert. Quantifiziert wird über eine Zwei-Punkt-Kalibrationskurve der Testverbindung in DMSO.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 5 µm, 50 mm x 2 mm; Temperatur: 40°C; Eluent A: Wasser/Phosphorsäure pH 2, Eluent B: Acetonitril; Flussrate: 0.7 ml/min; Gradient: 0-0.5 min 85% A, 15% B; Rampe 0.5-3 min 10% A, 90% B; 3-3.5 min 10% A, 90% B; Rampe 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 3.5 µm, 60 mm x 2.1 mm; Temperatur: 30°C; Eluent A: Wasser + 5 ml Perchlorsäure/Liter, Eluent B: Acetonitril; Flussrate: 0.75 ml/min; Gradient: 0-0.5 min 98% A, 2% B; Rampe 0.5-4.5 min 10% A, 90% B; 4.5-6 min 10% A, 90% B; Rampe 6.5-6.7 min 98% A, 2% B; 6.7-7.5 min 98% A, 2% B.

In Tabelle 1 sind die Löslichkeitswerte repräsentativer Ausführungsbeispiele in 5%-iger wässriger Dextroselösung dargestellt:

**Tabelle 1**

| **Beispiel Nr.** | **Löslichkeit [mg/Liter]** |
|---|---|
| 1 | >500 |
| 3 | 450 |
| 4 | >500 |
| 5 | >500 |
| 6 | >500 |
| 7 | 100 |
| 10 | 390 |
| 11 | >500 |
| 12 | >500 |
| 13 | >500 |
| 14 | >500 |
| 15 | 450 |
| 17 | >500 |
| 18 | 450 |

Eine Zersetzung der Beispielverbindungen in diesen Lösungen wird nicht beobachtet.

Die Löslichkeit der zu Grunde liegenden Wirksubstanz [Verbindung (A)] in 5%-iger wässriger Dextroselösung wird in diesem Test mit < 0.1 mg/Liter bestimmt.

### b) Stabilität in Puffer bei verschiedenen pH-Werten:

0.3 mg der Prüfsubstanz werden in einem 2 ml-HPLC-Vial eingewogen und mit 0.5 ml Acetonitril bzw. Acetonitril/DMSO (9: 1) versetzt. Zum Lösen der Substanz wird das Probengefäß für ca. 10 Sekunden ins Ultraschallbad gegeben. Anschließend werden 0.5 ml der jeweiligen (Puffer-) Lösung zugefügt und die Probe erneut im Ultraschallbad behandelt.

Eingesetzte (Puffer-)Lösungen:

| | |
|---|---|
| pH 2: | 0.03 Mol Zitronensäure, 0.061 Mol Natriumchlorid und 0.0082 Mol Salzsäure *ad* 1 Liter Wasser; |
| pH 4: | 1 Liter Millipore-Wasser wird mit 1 N Salzsäure auf pH 4.0 eingestellt; |
| pH 5: | 0.096 Mol Zitronensäure und 0.2 Mol Natriumhydroxid *ad* 1 Liter Wasser; |
| pH 6: | 0.06 Mol Zitronensäure und 0.16 Mol Natriumhydroxid *ad* 1 Liter Wasser; |
| pH 7.4: | 90.0 g Natriumchlorid, 13.61 g Kaliumdihydrogenphosphat und 83.35 g 1 N Natronlauge *ad* 1 Liter Wasser; diese Lösung wird dann noch 1:10 mit Millipore-Wasser weiter verdünnt; |
| pH 8: | 0.013 Mol Borax und 0.021 Mol Salzsäure *ad* 1 Liter Wasser. |

Über einen Zeitraum von 24 Stunden bei 37°C werden stündlich jeweils 5 µl der Probenlösung per HPLC auf ihren Gehalt an unveränderter Testsubstanz bzw. an entstandener Muttersubstanz (A) analysiert. Quantifiziert wird über die Flächenprozente der entsprechenden Peaks.

### HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 125 mm x 4 mm, 5 µm; Säulentemperatur: 30°C; Eluent A: Wasser + 5 ml Perchlorsäure/Liter, Eluent B: Acetonitril; Gradient: 0-2.0 min 90% A, 10% B; 2.0-18.0 min 64% A, 36% B; 18.0-20.0 min 64% A, 36% B; 20.0-21.0 min 10% A, 90% B; 21.0-23.0 min 90% A, 10% B; 23.0-26.0 min 90% A, 10% B; Flussrate: 2.0 ml/min; UV-Detektion: 294 nm.

In Tabelle 2 sind zu repräsentativen Ausführungsbeispielen die Verhältnisse der Peakflächen (F) zu den jeweiligen Zeitpunkten im Verhältnis zu den Peakflächen beim Startzeitpunkt dargestellt:

**Tabelle 2**

| **Beispiel Nr.** | **pH-Wert** | **% Testsubstanz nach 4 h [F(t=4h) x 100 / F(t=0h)]** | **% Testsubstanz nach 24 h [F(t=24h) x 100 / F(t=0h)]** |
|---|---|---|---|
| 1 | 4 | 100 | 100 |
| 1 | 6 | 100 | 100 |
| 1 | 7.4 | 99 | 96 |
| 1 | 8 | 99 | 90 |
| 2 | 4 | 100 | 100 |
| 2 | 7.4 | 100 | 100 |
| 3 | 4 | 100 | 100 |
| 3 | 7.4 | 99 | 55 |
| 4 | 4 | 100 | 100 |
| 4 | 5 | 100 | 98 |
| 4 | 6 | 97 | 84 |
| 4 | 7.4 | 48 | 2 |
| 5 | 4 | 100 | 100 |
| 5 | 5 | 100 | 98 |
| 5 | 6 | 95 | 70 |
| 6 | 2 | 100 | 100 |
| 6 | 4 | 100 | 100 |
| 6 | 5 | 96 | 80 |
| 6 | 6 | 78 | 25 |
| 6 | 7.4 | 8 | 0 |
| 7 | 4 | 100 | 98 |
| 7 | 7.4 | 51 | 2 |
| 9 | 4 | 100 | 100 |
| 9 | 7.4 | 100 | 100 |
| 10 | 4 | 100 | 99 |
| 10 | 7.4 | 100 | 99 |
| 11 | 4 | 100 | 100 |
| 11 | 7.4 | 90 | 55 |
| 12 | 4 | 99 | 98 |
| 12 | 7.4 | 49 | 2 |
| 13 | 4 | 100 | 99 |
| 13 | 7.4 | 24 | 0 |
| 14 | 4 | 100 | 100 |
| 14 | 7.4 | 68 | 8 |

In diesem Test wird gleichzeitig mit einer Abnahme des Gehalts an Testsubstanz eine Zunahme der Wirkstoff-Verbindung (A) festgestellt.

### c) In vitro-Stabilität in Ratten- und Humanplasma:

1 mg der Prüfsubstanz wird in einem 2 ml-HPLC-Vial eingewogen und mit 1.5 ml DMSO und 1 ml Wasser versetzt. Zum Lösen der Substanz wird das Probengefäß für ca. 10 Sekunden ins Ultraschallbad gestellt. Zu 0.5 ml dieser Lösung werden 0.5 ml 37°C warmes Ratten- bzw. Humanplasma gegeben. Die Probe wird geschüttelt und für eine erste Analyse ca. 10 µl entnommen (Zeitpunkt t₀). Im Zeitraum bis zu 2 Stunden nach Inkubationsbeginn werden 4-6 weitere Aliquote zur Quantifizierung entnommen. Die Probe wird über die Prüfzeit bei 37°C gehalten. Charakterisierung und Quantifizierung erfolgen per HPLC.

### HPLC-Methode:

Agilent 1100 mit DAD (G1314A), binärer Pumpe (G1312A), Autosampler (G1329A), Säulenofen (G1316A), Thermostat (G1330A); Säule: Kromasil 100 C18, 250 mm x 4 mm, 5 µm; Säulentemperatur: 30°C; Eluent A: Wasser + 5 ml Perchlorsäure/Liter, Eluent B: Acetonitril; Gradient: 0-8.0 min 53% A, 47% B; 8.0-18.0 min 53% A, 47% B; 18.0-20.0 min 90% A, 10% B; 20.0-21.0 min 90% A, 10% B; 21.0-22.5 min 98% A, 2% B; 22.5-25.0 min 98% A, 2% B; Flussrate: 2 ml/min; UV-Detektion: 294 nm.

In Tabelle 3 sind für repräsentative Ausführungsbeispiele die jeweiligen Zeiten angegeben, bei denen nach Inkubation mit Rattenplasma 50% der maximal möglichen Menge an Wirkstoff-Verbindung (A) entstanden sind (t_{50%A}). Zur Auswertung wird jeweils das Verhältnis der Peakflächen zu den einzelnen Zeitpunkten gegenüber dem Startzeitpunkt herangezogen.

**Tabelle 3**

| **Beispiel Nr.** | **t_{50%A} [min] in Rattenplasma** |
|---|---|
| 1 | 60 |
| 2 | 30 |
| 3 | 55 |
| 4 | 1.7 |
| 5 | 8.0 |
| 6 | 1.1 |
| 7 | 0.5 |
| 9 | >120 |
| 10 | >120 |
| 12 | 2 |
| 13 | 0.5 |
| 14 | 0.5 |

### d) i.v.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Hamilton^{®}-Glasspritze in die Schwanzvene appliziert (Bolusgabe, Applikationsdauer <10 s). Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ, T_{1/2} (Halbwertszeit) und CL (Clearance) erfolgt aus den gemessenen Plasmakonzentrationen.

Nach i.v.-Applikation der Verbindung aus Beispiel 4, aus Beispiel 5, aus Beispiel 6 bzw. aus Beispiel 7 konnten diese Substanzen bereits zum ersten Messpunkt nicht mehr im Plasma detektiert werden. Lediglich der Wirkstoff (A) war auch bis zum Zeitpunkt von 24 Stunden detektierbar.

### e) p.o.-Pharmakokinetik in Wistar-Ratten:

Am Tag vor der Substanzgabe wird den Versuchstieren (männliche Wistar-Ratten, Körpergewicht 200-250 g) unter Isofluran^{®}-Narkose ein Katheter für die Blutgewinnung in die *Vena jugularis* implantiert.

Am Versuchstag wird eine definierte Dosis der Prüfsubstanz als Lösung mit einer Schlundsonde in den Magen appliziert. Innerhalb von 24 h nach Substanzgabe werden sequentiell Blutproben (8-12 Zeitpunkte) über den Katheter entnommen. Zur Plasmagewinnung werden die Proben in heparinisierten Röhrchen zentrifugiert. Pro Zeitpunkt wird ein definiertes Plasmavolumen zur Proteinfällung mit Acetonitril versetzt. Nach Zentrifugation werden Prüfsubstanz und gegebenenfalls bekannte Spaltprodukte der Prüfsubstanz im Überstand mit einer geeigneten LC/MS-MS-Methode quantitativ bestimmt.

Die Berechnung pharmakokinetischer Kenngrößen der Prüfsubstanz bzw. der daraus freigesetzten Wirkstoff-Verbindung (A) wie AUC, Cₘₐₓ und T_{1/2} (Halbwertszeit) erfolgt aus den gemessenen Plasmakonzentrationen.

Nach p.o.-Applikation der Verbindung aus Beispiel 4, aus Beispiel 5 bzw. aus Beispiel 6 konnten diese Substanzen bereits zum ersten Messpunkt nicht mehr im Plasma detektiert werden. Lediglich der Wirkstoff (A) war auch bis zum Zeitpunkt von 24 Stunden detektierbar.

### f) Bestimmung des Einflusses auf die Herzfrequenz bei narkotisierten Ratten:

Eingesetzt werden männliche Wistar-Ratten mit einem Körpergewicht über 250 g. In der Nacht vor dem Versuch erhalten die Tiere kein Futter, haben aber weiterhin freien Zugang zu Trinkwasser. Präparation und Untersuchungen werden in Trapanal^{®}-Narkose (100 mg/kg i.p.) durchgeführt. Injektion und Infusion erfolgen über einen Katheter in der *V. jugularis,* die Blutdruck-Registrierung über einen Katheter in der *A. femoralis* (Transducer: Fa. Braun, Melsungen). Nach der Präparation werden die Tiere zum Ausgleich von Flüssigkeitsverlusten an eine Dauerinfusion von physiologischer Kochsalz-Lösung angeschlossen. Prüfsubstanz oder Placebo-Lösung werden nach einer Äquilibrierungszeit von ca. 1 h intravenös als Bolus verabreicht. Herzfrequenz und arterieller Blutdruck werden während der Äquilibrierung und über einen Zeitraum von mindestens 30 min nach der Bolusinjektion mit Hilfe eines digitalen Auswerteprogramms aufgezeichnet.

In Tabelle 4 ist die maximale Herzfrequenz-Abnahme in den ersten 30 min nach einem i.v.-Bolus von 100 µg/kg der Wirksubstanz (A) bzw. von äquivalenten Dosierungen repräsentativer Ausführungsbeispiele aufgeführt:

**Tabelle 4**

| **Beispiel Nr.** | **Herzfrequenz-Abnahme [%]** |
|---|---|
| A | 24 |
| 1 | 10 |
| 4 | 19 |
| 5 | 12 |
| 6 | 17 |
| 7 | 15 |

### C. Ausführungsbeispiele für pharmazeutische Zusammensetzungen

Die erfindungsgemäßen Verbindungen können beispielsweise folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%, die jeweils auf einen pH-Wert von 3-5 eingestellt sind) gelöst. Die Lösung wird gegebenenfalls steril filtriert und/oder in sterile und pyrogenfreie Injektionsbehältnisse abgefüllt.

## Patentansprüche

1. Verbindung der Formel (I) 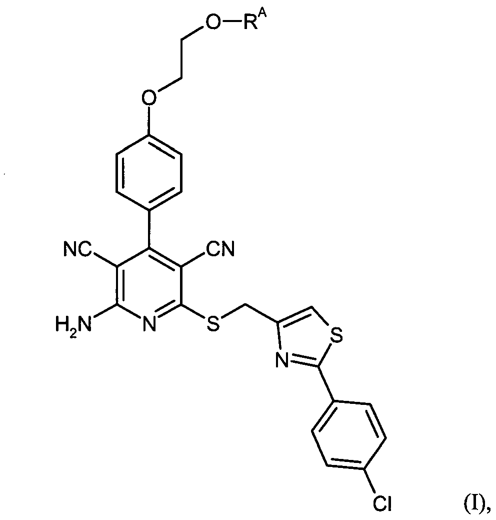 in welcher
R^{A} für eine Gruppe der Formel 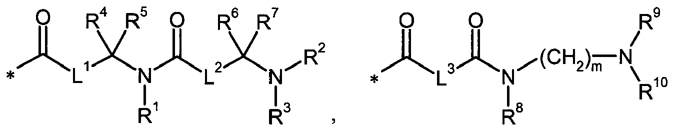 oder 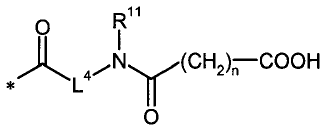 steht, worin
* die Verknüpfungsstelle mit dem O-Atom bedeutet,
L¹ und L² unabhängig voneinander eine Bindung oder -CH₂- bedeuten,
R¹, R² und R³ unabhängig voneinander Wasserstoff oder Methyl bedeuten,
R⁴ und R⁶ gleich oder verschieden sind und unabhängig voneinander Wasserstoff oder Methyl (Alanin), Propan-2-yl (Valin), Propan-1-yl (Norvalin), 2-Methylpropan-1-yl (Leucin), 1-Methylpropan-1-yl (Isoleucin), Butan-1-yl (Norleucin), *tert*.-Butyl (2-*tert*.-Butylglycin), Phenyl (2-Phenylglycin), Benzyl (Phenylalanin), p-Hydroxybenzyl (Tyrosin), Indol-3-ylmethyl (Tryptophan), Imidazol-4-ylmethyl (Histidin), Hydroxymethyl (Serin), 2-Hydroxyethyl (Homoserin), 1-Hydroxyethyl (Threonin), Mercaptomethyl (Cystein), Methylthiomethyl (S-Methylcystein), 2-Mercaptoethyl (Homo-cystein), 2-Methylthioethyl (Methionin), Carbamoylmethyl (Asparagin), 2-Carbamoylethyl (Glutamin), Carboxymethyl (Asparaginsäure), 2-Carboxy-ethyl (Glutaminsäure), 4-Aminobutan-1-yl (Lysin), 4-Amino-3-hydroxy-butan-1-yl (Hydroxylysin), 3-Aminopropan-1-yl (Ornithin), 2-Aminoethyl (2,4-Diaminobuttersäure), Aminomethyl (2,3-Diaminopropionsäure), 3-Gua-nidinopropan-1-yl (Arginin), 3-Ureidopropan-1-yl (Citrullin) bedeuten,
R⁵ und R⁷ unabhängig voneinander Wasserstoff oder Methyl bedeuten,
L³ geradkettiges oder verzweigtes (C₂-C₄)-Alkandiyl, welches darüber hinaus mit Amino substituiert ist, bedeutet,
R⁸, R⁹ und R¹⁰ unabhängig voneinander Wasserstoff oder Methyl bedeuten,
m die Zahl 2, 3, 4, 5 oder 6 bedeutet,
L⁴ geradkettiges oder verzweigtes (C₂-C₄)-Alkandiyl, welches darüber hinaus mit Carboxyl substituiert ist, bedeutet,
R¹¹ Wasserstoff oder Methyl bedeutet
und
n die Zahl 1, 2, 3 oder 4 bedeutet,
sowie ihre Salze, Solvate und Solvate der Salze.

2. Verbindung der Formel (I) nach Anspruch 1, in welcher
R^{A} für eine Gruppe der Formel 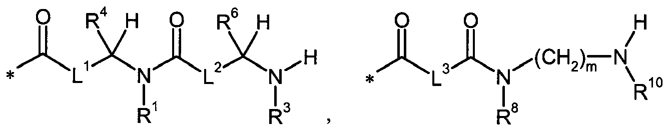 oder 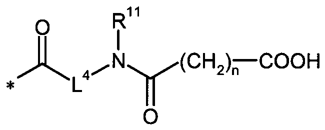 steht, worin
* die Verknüpfungsstelle mit dem O-Atom bedeutet,
L¹ eine Bindung bedeutet,
L² eine Bindung oder -CH₂- bedeutet,
R¹ und R³ unabhängig voneinander Wasserstoff oder Methyl bedeuten,
R⁴ Wasserstoff, Methyl, Propan-2-yl, Propan-1-yl, 2-Methylpropan-1-yl, 1-Methylpropan-1-yl, Butan-1-yl, Benzyl, p-Hydroxybenzyl, Imidazol-4-yl-methyl, Hydroxymethyl, 1-Hydroxyethyl, Carbamoylmethyl oder 2-Carbamoylethyl bedeutet,
R⁶ Wasserstoff, Imidazol-4-ylmethyl, 4-Aminobutan-1-yl, 3-Aminopropan-1-yl, 2-Aminoethyl, Aminomethyl oder 3-Guanidinopropan-1-yl bedeutet,
L³ geradkettiges (C₂-C₄)-Alkandiyl, welches darüber hinaus mit Amino substituiert ist, bedeutet,
R⁸ und R¹⁰ unabhängig voneinander Wasserstoff oder Methyl bedeuten,
m die Zahl 2, 3 oder 4 bedeutet,
L⁴ geradkettiges (C₂-C₄)-Alkandiyl, welches darüber hinaus mit Carboxyl substituiert ist, bedeutet,
R¹¹ Wasserstoff oder Methyl bedeutet
und
n die Zahl 2, 3 oder 4 bedeutet,
sowie ihre Salze, Solvate und Solvate der Salze.

3. Verbindung der Formel (I) nach Anspruch 1 oder 2, in welcher
R^{A} für eine Gruppe der Formel 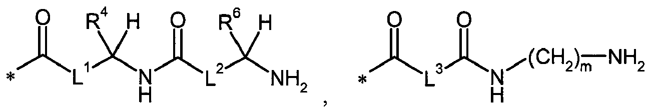 oder 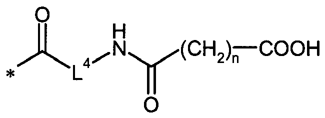 steht, worin
* die Verknüpfungsstelle mit dem O-Atom bedeutet,
L¹ und L² jeweils eine Bindung bedeuten,
R⁴ Wasserstoff, Methyl, Propan-2-yl, Propan-1-yl, 2-Methylpropan-1-yl, 1-Methylpropan-1-yl, Butan-1-yl, Benzyl, p-Hydroxybenzyl, Imidazol-4-yl-methyl, Hydroxymethyl, 1-Hydroxyethyl, Carbamoylmethyl oder 2-Carbamoylethyl bedeutet,
R⁶ Imidazol-4-ylmethyl, 4-Aminobutan-1-yl, 3-Aminopropan-1-yl, 2-Amino-ethyl, Aminomethyl oder 3-Guanidinopropan-1-yl bedeutet,
L³ eine Gruppe der Formel -CH(NH₂)-CH₂-, -CH₂-CH(NH₂)-, -CH₂-CH(NH₂)-CH₂-, -CH(NH₂)-CH₂-CH₂- oder -CH₂-CH₂-CH(NH₂)-bedeutet,
m die Zahl 2, 3 oder 4 bedeutet,
L⁴ eine Gruppe der Formel **-CH₂-CH(COOH)- oder ** -CH₂-CH₂-CH(COOH)- bedeutet, worin
** die Verknüpfungsstelle mit der angrenzenden Carbonylgruppe darstellt,
und
n die Zahl 2 oder 3 bedeutet,
sowie ihre Salze, Solvate und Solvate der Salze.

4. Verbindung der Formel (I) nach Anspruch 1, 2 oder 3, in welcher
R^{A} für eine Gruppe der Formel 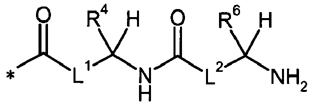 steht, worin
* die Verknüpfungsstelle mit dem O-Atom bedeutet,
L¹ und L² jeweils eine Bindung bedeuten,
R⁴ Wasserstoff, Methyl, Propan-2-yl, 2-Methylpropan-1-yl, Benzyl, Hydroxymethyl oder 1-Hydroxyethyl bedeutet
und
R⁶ Imidazol-4-ylmethyl, 4-Aminobutan-1-yl, 3-Aminopropan-1-yl, 2-Amino-ethyl, Aminomethyl oder 3-Guanidinopropan-1-yl bedeutet,
sowie ihre Salze, Solvate und Solvate der Salze.

5. Verbindung der Formel (I) nach Anspruch 1, 2 oder 3, in welcher
R^{A} für eine Gruppe der Formel 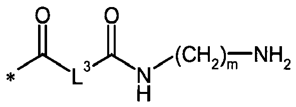 steht, worin
* die Verknüpfungsstelle mit dem O-Atom bedeutet,
L³ eine Gruppe der Formel -CH(NH₂)-CH₂-, -CH₂-CH(NH₂)-, -CH(NH₂)-CH₂-CH₂- oder -CH₂-CH₂-CH(NH₂)- bedeutet
und
m die Zahl 2 oder 3 bedeutet,
sowie ihre Salze, Solvate und Solvate der Salze.

6. Verbindung nach Anspruch 1, 2 oder 3 mit der Formel 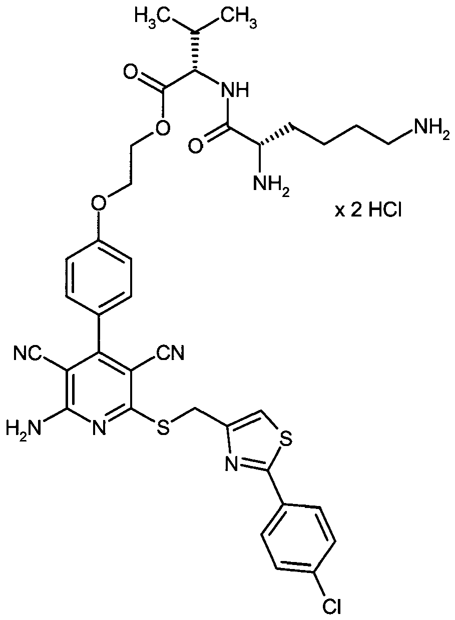 2-{4-[2-Amino-6-({[2-(4-chlorphenyl)-1,3-thiazol-4-yl]methyl}thio)-3,5-dicyanopyridin-4-yl]phenoxy}ethyl-L-lysyl-L-valinat-Dihydrochlorid

7. Verbindung nach Anspruch 1, 2 oder 3 mit der Formel 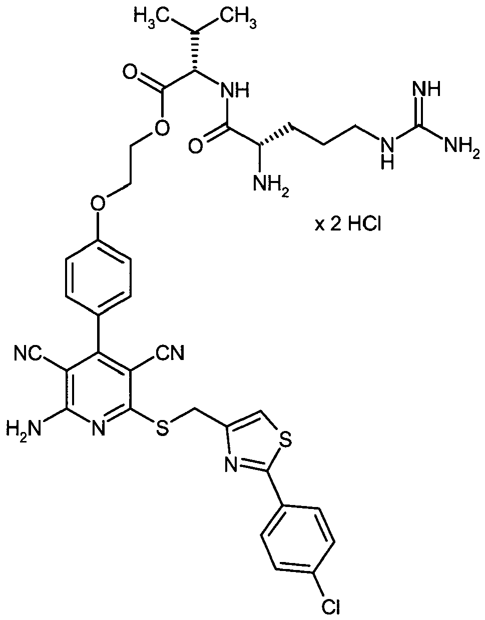 2- {4-[2-Amino-6-({[2-(4-chlorphenyl)-1,3-thiazol-4-yl]methyl}thio)-3,5-dicyanopyridin-4-yl]phenoxy}ethyl-L-arginyl-L-valinat-Dihydrochlorid

8. Verbindung nach Anspruch 1, 2 oder 3 mit der Formel 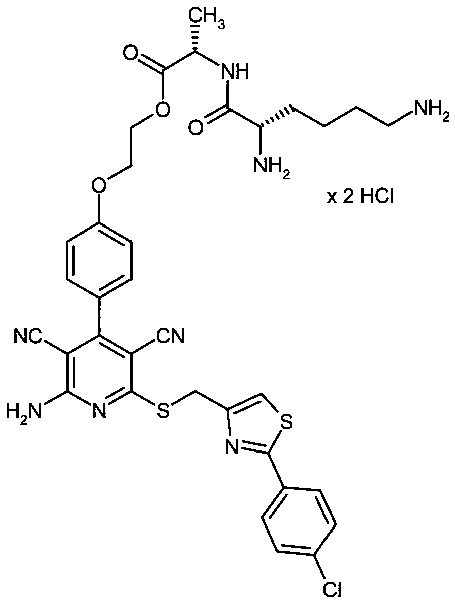 2-{4-[2-Amino-6-({[2-(4-chlorphenyl)-1,3-thiazol-4-yl]methyl}thio)-3,5-dicyanopyridin-4-yl]phenoxy}ethyl-L-lysyl-L-alaninat-Dihydrochlorid

9. Verbindung nach Anspruch 1, 2 oder 3 mit der Formel 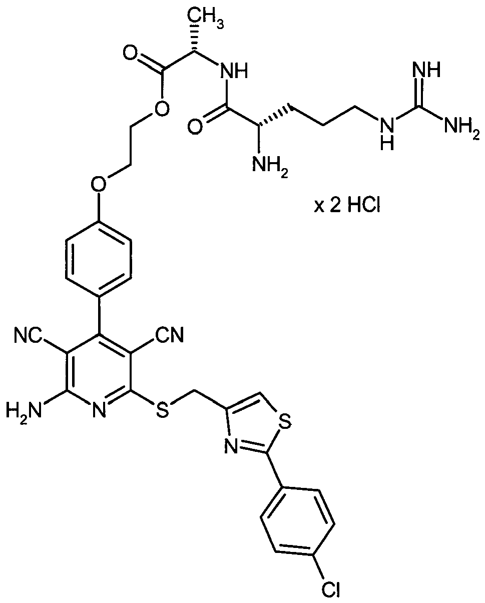 2- {4-[2-Amino-6-({[2-(4-chlorphenyl)-1,3-thiazol-4-yl]methyl}sulfanyl)-3,5-dicyanopyridin-4-yl]phenoxy}ethyl-L-arginyl-L-alaninat-Dihydrochlorid

10. Verbindung nach Anspruch 1, 2 oder 3 mit der Formel 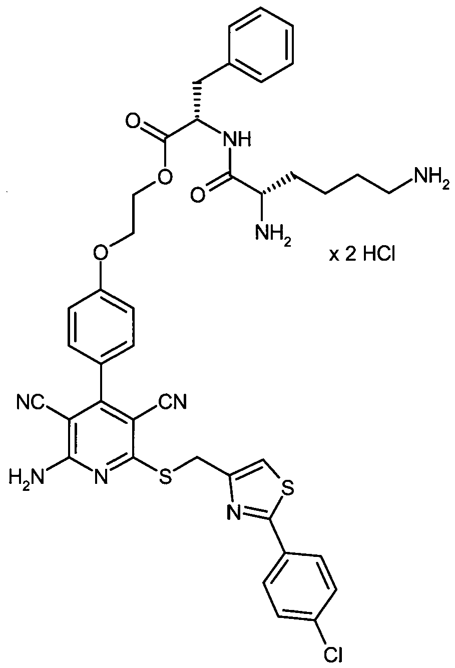 2- {4-[2-Amino-6-({[2-(4-chlorphenyl)-1,3-thiazol-4-yl]methyl}sulfanyl)-3,5-dicyanopyridin-4-yl]phenoxy}ethyl-L-lysyl-L-phenylalaninat-Dihydrochlorid

11. Verbindung nach Anspruch 1, 2 oder 3 mit der Formel 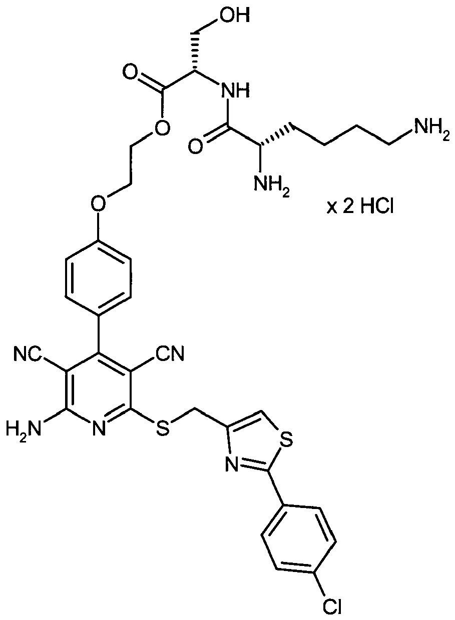 2-{4-[2-Amino-6-({[2-(4-chlorphenyl)-1,3-thiazol-4-yl]methyl}thio)-3,5-dicyanopyridin-4-yl]phenoxy} ethyl-L-lysyl-L-serinat-Dihydrochlorid

12. Verbindung nach Anspruch 1, 2 oder 3 mit der Formel 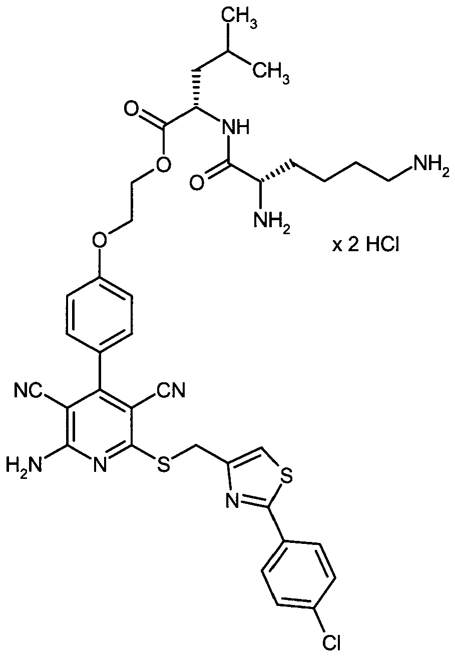 2- {4-[2-Amino-6-({[2-(4-chlorphenyl)-1,3-thiazol-4-yl]methyl}sulfanyl)-3,5-dicyanopyridin-4-yl]phenoxy}ethyl-L-lysyl-L-leucinat-Dihydrochlorid

13. Verfahren zur Herstellung von Verbindungen der Formel (I), wie in den Ansprüchen 1 bis 12 definiert, **dadurch gekennzeichnet, dass** man die Verbindung (A) 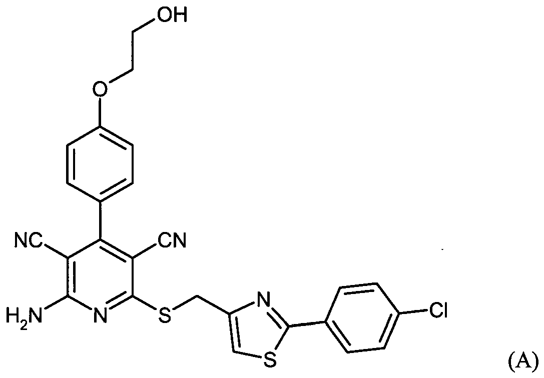 entweder
[A] in einem inerten Lösungsmittel in Gegenwart eines Kondensationsmittels zunächst mit einer Carbonsäure der Formel (II), (III) oder (IV) 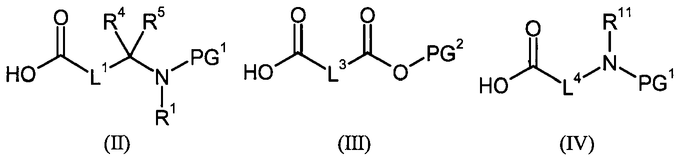
in welchen L¹, L³, L⁴, R¹, R⁴, R⁵ und R¹¹ jeweils die in den Ansprüchen 1 bis 5 angegebenen Bedeutungen haben und
PG¹ für eine temporäre Amino-Schutzgruppe wie beispielsweise *tert*.-Butoxycarbonyl
und
PG² für eine temporäre Carboxyl-Schutzgruppe wie beispielsweise *tert*.-Butyl stehen,
zu Verbindungen der Formel (V), (VI) bzw. (VII) 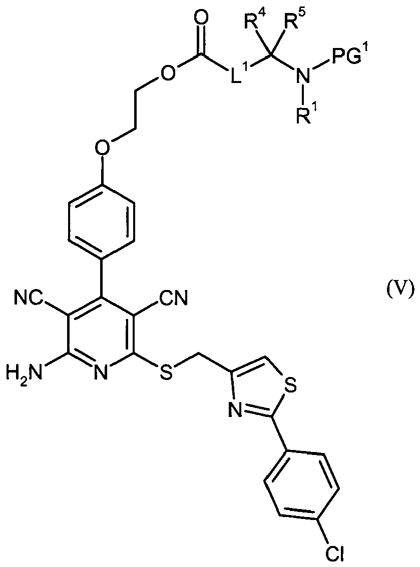 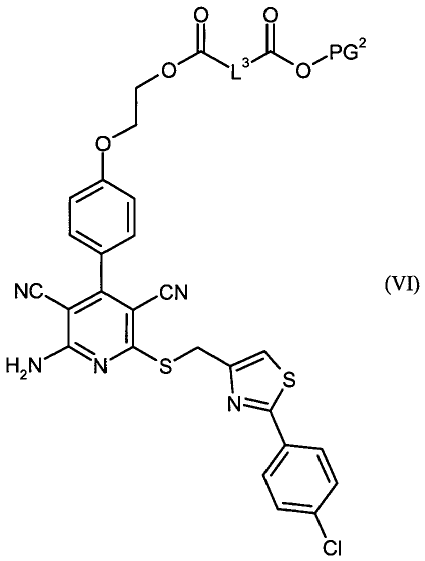 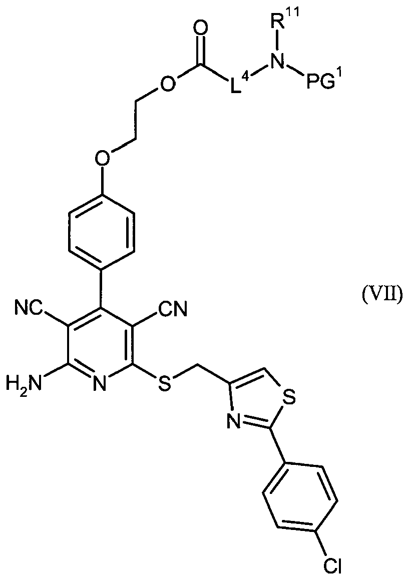 in welchen L¹, L³, L⁴, R¹, R⁴, R⁵, R¹¹, PG¹ und PG² jeweils die oben angegebenen Bedeutungen haben,
verestert, nach Abspaltung der Schutzgruppe PG¹ bzw. PG² dann in einem inerten Lösungsmittel in Gegenwart eines Kondensationsmittels im Fall der Verbindung (V) mit einer Verbindung der Formel (VIII), im Fall der Verbindung (VI) mit einer Verbindung der Formel (IX) und im Fall der Verbindung (VII) mit einer Verbindung der Formel (X) 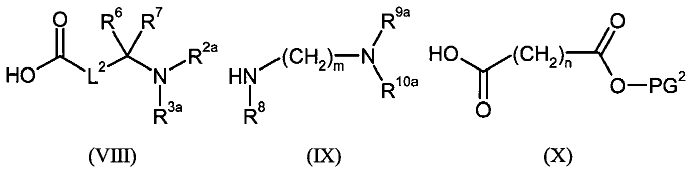
in welchen L², R⁶, R⁷, R⁸, PG², m und n jeweils die in den Ansprüchen 1 bis 5 angegebenen Bedeutungen haben
und
R^{2a} und R^{3a} sowie R^{9a} und R^{10a} jeweils gleich oder verschieden sind und die in den Ansprüchen 1 bis 5 angegebenen Bedeutungen von R², R³, R⁹ bzw. R¹⁰ haben oder für eine temporäre Amino-Schutzgruppe wie beispielsweise *tert.-*Butoxycarbonyl stehen,
kuppelt und anschließend gegebenenfalls vorhandene Schutzgruppen wieder entfernt oder
[B] in einem inerten Lösungsmittel in Gegenwart eines Kondensationsmittels mit einer Verbindung der Formel (XI), (XII) oder (XIII) 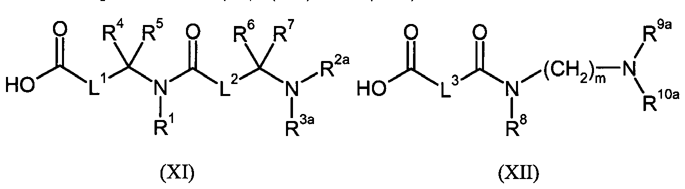 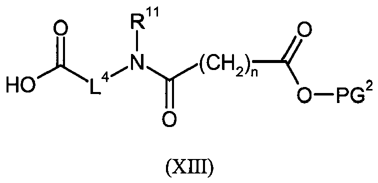
in welchen L¹, L², L³, L⁴, R¹¹, R⁴, R⁵, R⁶, R⁷, R⁸, R¹¹, m und n jeweils die in den Ansprüchen 1 bis 5 angegebenen Bedeutungen haben,
R^{2a} und R^{3a} sowie R^{9a} und R^{10a} jeweils gleich oder verschieden sind und die in den Ansprüchen 1 bis 5 angegebenen Bedeutungen von R², R³, R⁹ bzw. R¹⁰ haben oder für eine temporäre Amino-Schutzgruppe wie beispielsweise *tert.-*Butoxycarbonyl stehen
und
PG² für eine temporäre Carboxyl-Schutzgruppe wie beispielsweise *tert*.-Butyl steht,
kuppelt und anschließend gegebenenfalls vorhandene Schutzgruppen wieder entfernt und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

14. Verbindung, wie in einem der Ansprüche 1 bis 12 definiert, zur Behandlung und/oder Prophylaxe von Krankheiten.

15. Verwendung einer Verbindung, wie in einem der Ansprüche 1 bis 12 defmiert, zur Herstellung eines Arzneimittels zur Behandlung und/oder Prophylaxe von kardiovaskulären Erkrankungen.

16. Arzneimittel enthaltend eine Verbindung, wie in einem der Ansprüche 1 bis 12 definiert, gegebenenfalls in Kombination mit einem oder mehreren inerten, nicht-toxischen, pharmazeutisch geeigneten Hilfsstoffen.

17. Arzneimittel enthaltend eine Verbindung, wie in einem der Ansprüche 1 bis 12 definiert, in Kombination mit einem oder mehreren weiteren Wirkstoffen.

18. Arzneimittel nach Anspruch 16 oder 17 zur Behandlung und/oder Prophylaxe von kardiovaskulären Erkrankungen.

## Claims

1. Compound of the formula (I) 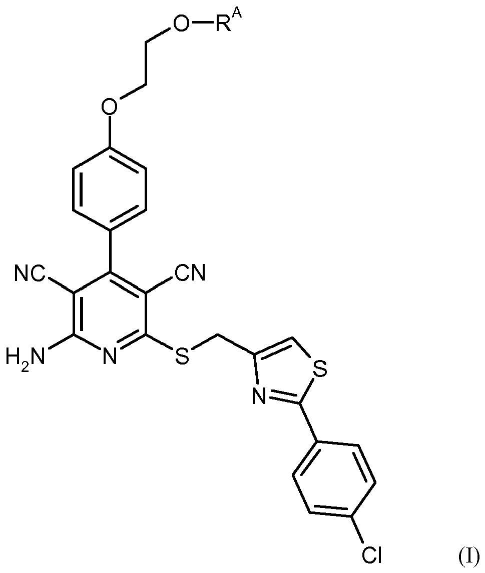 in which
R^{A} is a group of the formula 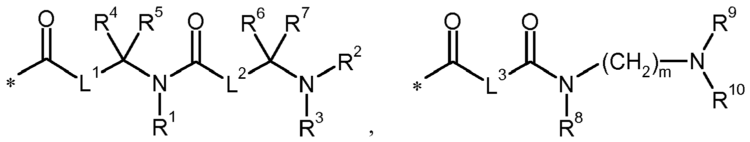 or 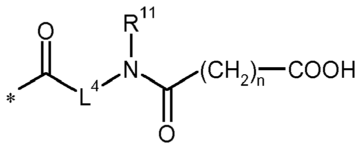 in which
* means the point of linkage to the O atom,
L¹ and L² are independently of one another a bond or -CH₂-,
R¹, R² and R³ are independently of one another hydrogen or methyl,
R⁴ and R⁶ are identical or different and are independently of one another hydrogen or methyl (alanine), propan-2-yl (valine), propan-1-yl (norvaline), 2-methylpropan-1-yl (leucine), 1-methylpropan-1-yl (isoleucine), butan-1-yl (norleucine), tert-butyl (2-tert-butylglycine), phenyl (2-phenylglycine), benzyl (phenylalanine), p-hydroxybenzyl (tyrosine), indol-3-ylmethyl (tryptophan), imidazol-4-ylmethyl (histidine), hydroxymethyl (serine), 2-hydroxyethyl (homoserine), 1-hydroxyethyl (threonine), mercaptomethyl (cysteine), methylthiomethyl (S-methylcysteine), 2-mercaptoethyl (homocysteine), 2-methylthioethyl (methionine), carbamoylmethyl (asparagine), 2-carbamoylethyl (glutamine), carboxymethyl (aspartic acid), 2-carboxyethyl (glutamic acid), 4-aminobutan-1-yl (lysine), 4-amino-3-hydroxybutan-1-yl (hydroxylysine), 3-aminopropan-1-yl (ornithine), 2-aminoethyl (2,4-diaminobutyric acid), aminomethyl (2,3-diaminopropionic acid), 3-guanidinopropan-1-yl (arginine), 3-ureidopropan-1-yl (citrulline),
R⁵ and R⁷ are independently of one another hydrogen or methyl,
L³ is straight-chain or branched (C₂-C₄)-alkanediyl which is additionally substituted by amino,
R⁸, R⁹ and R¹⁰ are independently of one another hydrogen or methyl,
m is the number 2, 3, 4, 5 or 6,
L⁴ is straight-chain or branched (C₂-C₄)-alkanediyl, which is additionally substituted by carboxyl,
R¹ is hydrogen or methyl,
and
n is the number 1, 2, 3 or 4,
and the salts, solvates and solvates of the salts thereof.

2. Compound of the formula (I) according to Claim 1, in which
R^{A} is a group of the formula 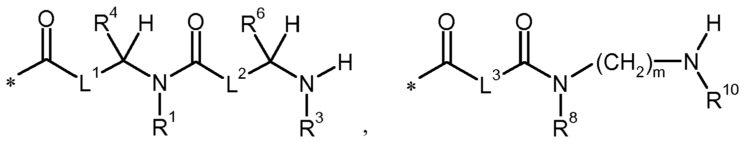 or 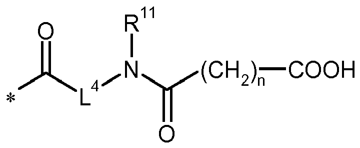 in which
* means the point of linkage to the O atom,
L¹ is a bond,
L² is a bond or -CH₂-,
R¹ and R³ are independently of one another hydrogen or methyl,
R⁴ is hydrogen, methyl, propan-2-yl, propan-1-yl, 2-methylpropan-1-yl, 1-methylpropan-1-yl, butan-1-yl, benzyl, p-hydroxybenzyl, imidazol-4-ylmethyl, hydroxymethyl, 1-hydroxyethyl, carbamoylmethyl or 2-carbamoylethyl,
R⁶ is hydrogen, imidazol-4-ylmethyl, 4-aminobutan-1-yl, 3-aminopropan-1-yl, 2-aminoethyl, aminomethyl or 3-guanidinopropan-1-yl,
L³ is straight-chain (C₂-C₄)-alkanediyl which is additionally substituted by amino,
R⁸ and R¹⁰ are independently of one another hydrogen or methyl,
m is the number 2, 3 or 4,
L⁴ is straight-chain (C₂-C₄)-alkanediyl which is additionally substituted by carboxyl,
R¹¹ is hydrogen or methyl,
and
n is the number 2, 3 or 4,
and the salts, solvates and solvates of the salts thereof.

3. Compound of the formula (I) according to Claim 1 or 2, in which
R^{A} is a group of the formula 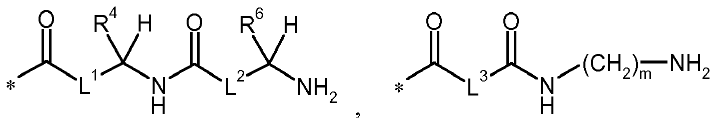 or 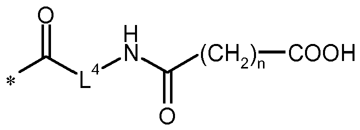 in which
* means the point of linkage to the O atom,
L¹ and L² are each a bond,
R⁴ is hydrogen, methyl, propan-2-yl, propan-1-yl, 2-methylpropan-1-yl, 1-methylpropan-1-yl, butan-1-yl, benzyl, p-hydroxybenzyl, imidazol-4-ylmethyl, hydroxymethyl, 1-hydroxyethyl, carbamoylmethyl or 2-carbamoylethyl,
R⁶ is imidazol-4-ylmethyl, 4-aminobutan-1-yl, 3-aminopropan-1-yl, 2-aminoethyl, aminomethyl or 3-guanidinopropan-1-yl,
L³ is a group of the formula -CH(NH₂)-CH₂-, -CH₂-CH(NH₂)-, -CH₂-CH(NH₂)-CH₂-, -CH(NH₂)-CH₂-CH₂- or -CH₂-CH₂-CH(NH₂)-,
m is the number 2, 3 or 4,
L⁴ is a group of the formula **-CH₂-CH(COOH)- or **-CH₂-CH₂-CH(COOH)-, in which
** represents the point of linkage to the adjoining carbonyl group,
and
n is the number 2 or 3,
and the salts, solvates and solvates of the salts thereof.

4. Compound of the formula (I) according to Claim 1, 2 or 3, in which
R^{A} is a group of the formula 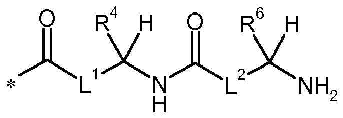 in which
* means the point of linkage to the O atom,
L¹ and L² are each a bond,
R⁴ is hydrogen, methyl, propan-2-yl, 2-methylpropan-1-yl, benzyl, hydroxymethyl or 1-hydroxyethyl,
and
R⁶ is imidazol-4-ylmethyl, 4-aminobutan-1-yl, 3-aminopropan-1-yl, 2-aminoethyl, aminomethyl or 3-guanidinopropan-1-yl,
and the salts, solvates and solvates of the salts thereof.

5. Compound of the formula (I) according to Claim 1, 2 or 3, in which
R^{A} is a group of the formula 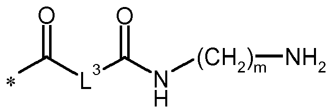 in which
* means the point of linkage to the O atom,
L³ is a group of the formula -CH(NH₂)-CH₂-, -CH₂-CH(NH₂)-, - CH(NH₂)-CH₂-CH₂- or -CH₂-CH₂-CH(NH₂)-,
and
m is the number 2 or 3,
and the salts, solvates and solvates of the salts thereof.

6. Compound according to Claim 1, 2 or 3 having the formula 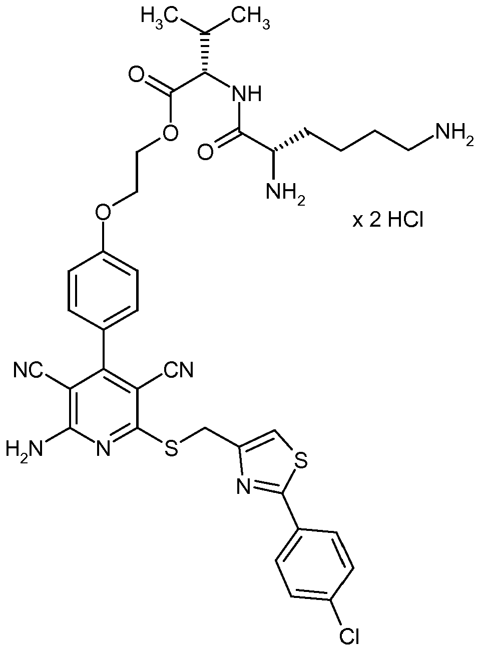 2- {4-[2-amino-6-({[2-(4-chlorophenyl)-1,3-thiazol-4-yl]methyl}thio)-3,5-dicyanopyridin-4-yl]phenoxy} ethyl L-lysyl-L-valinate dihydrochloride.

7. Compound according to Claim 1, 2 or 3 having the formula 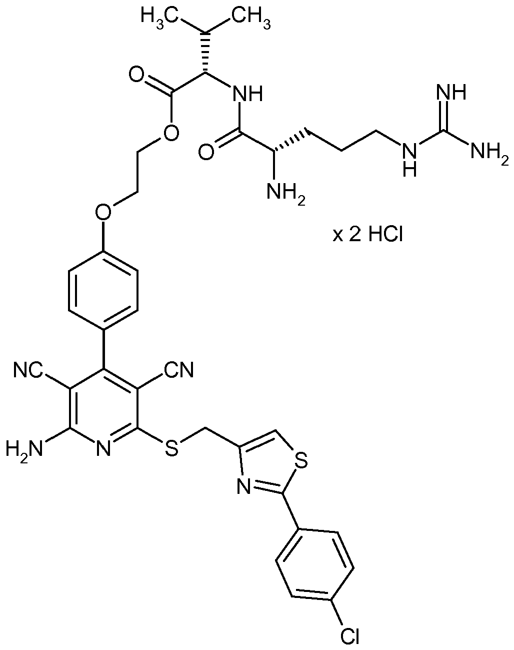 2- {4-[2-amino-6-({[2-(4-chlorophenyl)-1,3-thiazol-4-yl]methyl}thio)-3,5-dicyanopyridin-4-yl]phenoxy} ethyl L-arginyl-L-valinate dihydrochloride.

8. Compound according to Claim 1, 2 or 3 having the formula 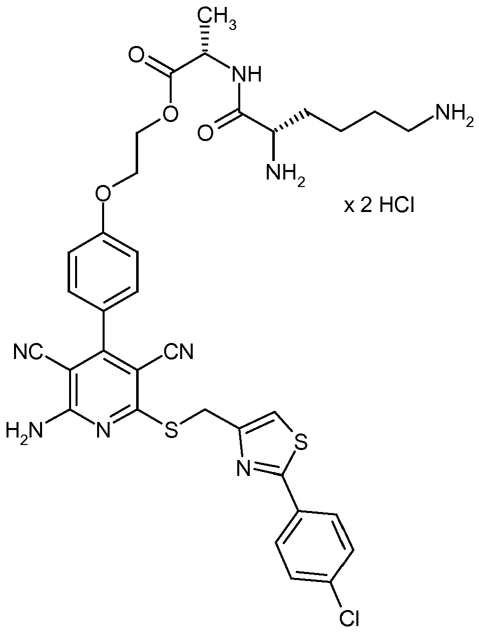 2- {4-[2-amino-6-({[2-(4-chlorophenyl)-1,3-thiazol-4-yl]methyl}thio)-3,5-dicyanopyridin-4-yl]phenoxy} ethyl L-lysyl-L-alaninate dihydrochloride.

9. Compound according to Claim 1, 2 or 3 having the formula 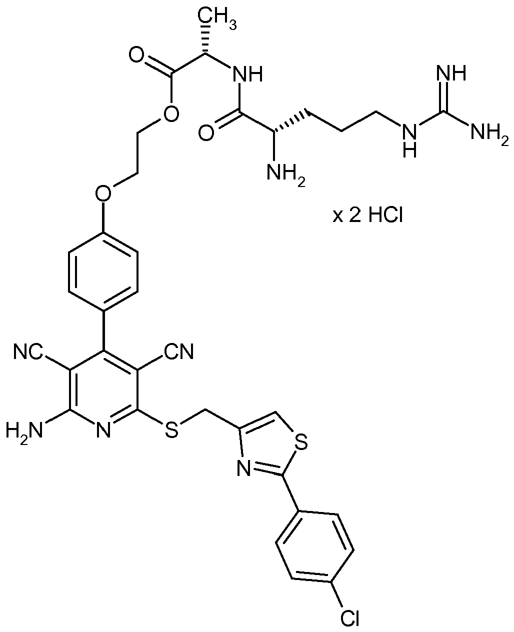 2- {4-[2-amino-6-({[2-(4-chlorophenyl)-1,3-thiazol-4-yl]methyl} sulfanyl)-3,5-dicyanopyridin-4-yl]phenoxy} ethyl L-arginyl-L-alaninate dihydrochloride.

10. Compound according to Claim 1, 2 or 3 having the formula 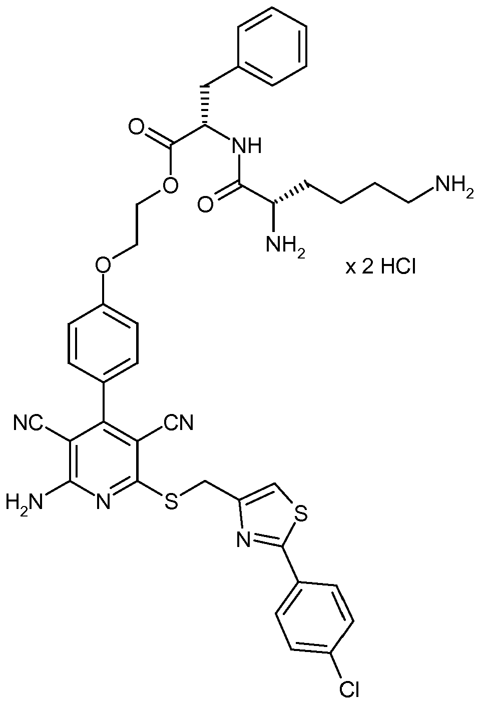 2-{4-[2-amino-6-({[2-(4-chlorophenyl)-1,3-thiazol-4-yl]methyl}sulfanyl)-3,5-dicyanopyridin-4-yl]phenoxy}ethyl L-lysyl-L-phenylalaninate dihydrochloride.

11. Compound according to Claim 1, 2 or 3 having the formula 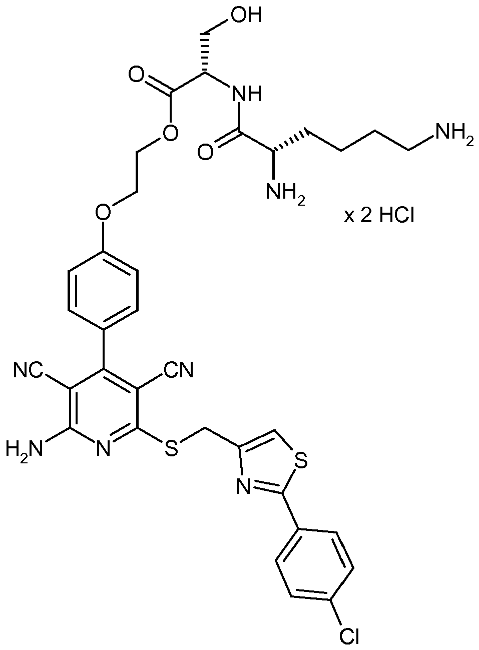 2- {4-[2-amino-6-({[2-(4-chlorophenyl)-1,3-thiazol-4-yl]methyl}thio)-3,5-dicyanopyridin-4-yl]phenoxy} ethyl L-lysyl-L-serinate dihydrochloride.

12. Compound according to Claim 1, 2 or 3 having the formula 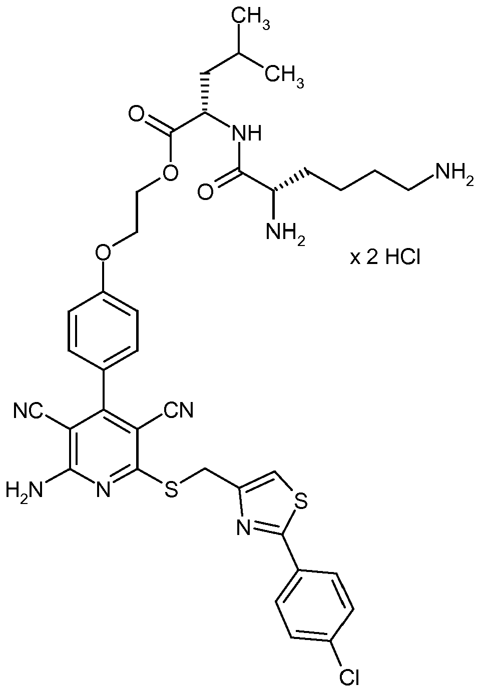 2- {4-[2-amino-6-({[2-(4-chlorophenyl)-1,3-thiazol-4-yl]methyl} sulfanyl)-3,5-dicyanopyridin-4-yl]phenoxy} ethyl L-lysyl-L-leucinate dihydrochloride.

13. Process for preparing compounds of the formula (I) as defined in Claims 1 to 12, **characterized in that** the compound (A) 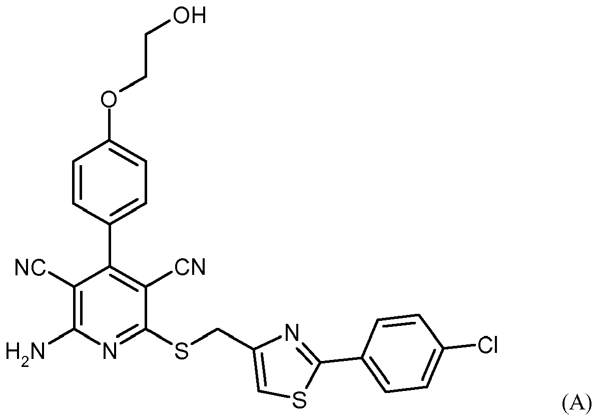 either
[A] is esterified in an inert solvent in the presence of a condensing agent initially with a carboxylic acid of the formula (II), (III) or (IV) 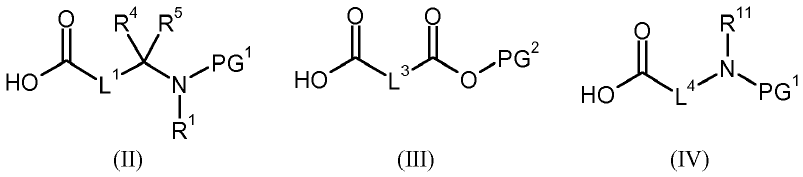
in which L¹, L³, L⁴, R¹, R⁴, R⁵ and R¹¹ each have the meanings indicated in Claims 1 to 5, and
PG¹ is a temporary amino protective group such as, for example, *tert-*butoxycarbonyl
and
PG² is a temporary carboxyl protective group such as, for example, *tert*-butyl,
to give compounds of the formula (V), (VI) or (VII), 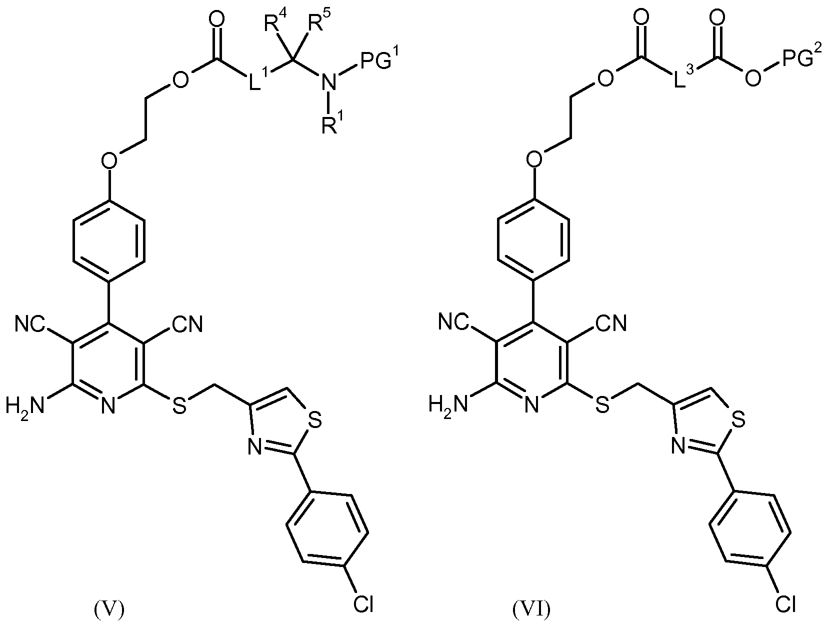 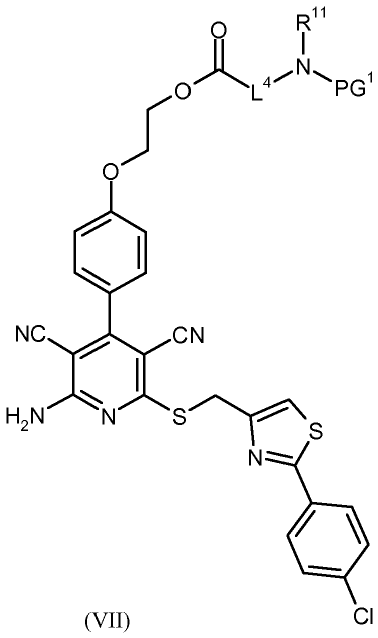 in which L¹, L³, L⁴, R¹, R⁴, R⁵, R¹¹, PG¹ and PG² each have the meanings indicated above,
then, after elimination of the protective group PG¹ or PG², is coupled in an inert solvent in the presence of a condensing agent in the case of compound (V) with a compound of the formula (VIII), in the case of compound (VI) with a compound of the formula (IX) and in the case of compound (VII) with a compound of the formula (X) 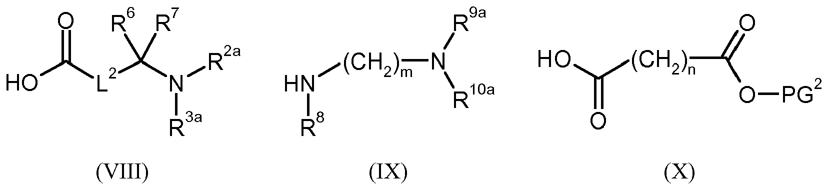
in which L², R⁶, R⁷, R⁸, PG², m and n each have the meanings indicated in Claims 1 to 5,
and
R^{2a} and R^{3a}, and R^{9a} and R^{10a}, are in each case identical or different and have the meanings of respectively R², R³, R⁹ and R¹⁰ indicated in Claims 1 to 5, or are a temporary amino protective group such as, for example, *tert*-butoxycarbonyl,
and subsequently protective groups which are present where appropriate are removed again,
or
[B] is coupled in an inert solvent in the presence of a condensing agent with a compound of the formula (XI), (XII) or (XIII) 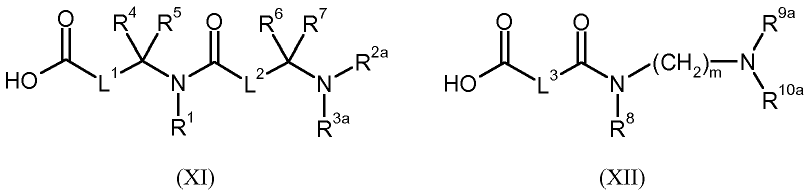 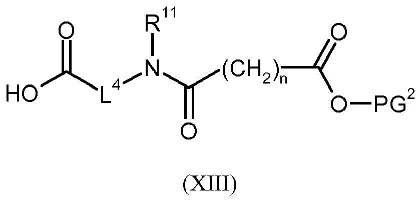
in which L¹ L², L³, L⁴, R¹, R⁴, R⁵, R⁶, R⁷, R⁸, R¹¹, m and n each have the meanings indicated in Claims 1 to 5,
R^{2a} and R^{3a}, and R^{9a} and R^{10a}, are in each case identical or different and have the meanings of respectively R², R³, R⁹ and R¹⁰ indicated in Claims 1 to 5, or are a temporary amino protective group such as, for example, *tert*-butoxycarbonyl,
and
PG² is a temporary carboxyl protective group such as, for example, *tert*-butyl,
and subsequently protective groups which are present where appropriate are removed again,
and the resulting compounds of the formula (I) are converted where appropriate with the appropriate (i) solvents and/or (ii) acids or bases into the solvates, salts and/or solvates of the salts thereof.

14. Compound as defined in any of Claims 1 to 12 for the treatment and/or prophylaxis of diseases.

15. Use of a compound as defined in any of Claims 1 to 12 for the manufacture of a medicament for the treatment and/or prophylaxis of cardiovascular disorders.

16. Medicament comprising a compound as defined in any of Claims 1 to 12, where appropriate in combination with one or more inert, non-toxic pharmaceutically suitable excipients.

17. Medicament comprising a compound as defined in any of Claims 1 to 12 in combination with one or more further active ingredients.

18. Medicament according to Claim 16 or 17 for the treatment and/or prophylaxis of cardiovascular disorders.

## Revendications

1. Composé de formule (I) 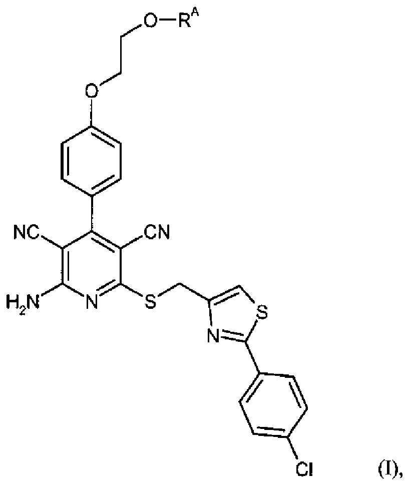 dans laquelle
R^{A} représente un groupe de formule 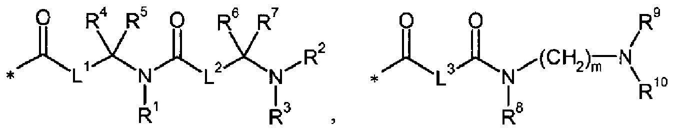 ou 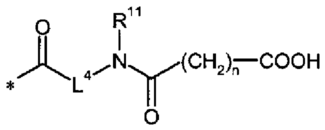 dans laquelle
* signifie le site de liaison avec l'atome de O,
L¹ et L² signifient, indépendamment l'un de l'autre, une liaison ou -CH₂-,
R¹, R² et R³ signifient, indépendamment les uns des autres, hydrogène ou méthyle,
R⁴ et R⁶ sont identiques ou différents et signifient, indépendamment l'un de l'autre, hydrogène ou méthyle (alanine), propan-2-yle (valine), propan-1-yle (norvaline), 2-méthylpropan-1-yle (leucine), 1-méthylpropan-1-yle (isoleucine), butan-1-yle (norleucine), tert-butyle (2-tert-butylglycine), phényle (2-phénylglycine), benzyle (phénylalanine), p-hydroxybenzyle (tyrosine), indol-3-ylméthyle (tryptophane), imidazol-4-ylméthyle (histidine), hydroxyméthyle (sérine), 2-hydroxyéthyle (homosérine), 1-hydroxyéthyle (thréonine), mercaptométhyle (cystéine), méthylthiométhyle (S-méthylcystéine), 2-mercaptoéthyle (homocystéine), 2-méthylthioéthyle (méthionine), carbamoylméthyle (asparagine), 2-carbamoyléthyle (glutamine), carboxyméthyle (acide aspartique), 2-carboxyéthyle (acide glutamique), 4-aminobutan-1-yle (lysine), 4-amino-3-hydroxybutan-1-yle (hydroxylysine), 3-aminopropan-1-yle (ornithine), 2-aminoéthyle (acide 2,4-diaminobutyrique), aminométhyle (acide 2,3-diaminopropionique), 3-guanidinopropan-1-yle (arginine), 3-uréidopropan-1-yle (citrulline),
R⁵ et R⁷ signifient, indépendamment l'un de l'autre, hydrogène ou méthyle,
L³ signifie (C₂-C₄) -alcanediyle linéaire ou ramifié, qui est en outre substitué par amino,
R⁸, R⁹ et R¹⁰ signifient, indépendamment les uns des autres, hydrogène ou méthyle,
m signifie le nombre 2, 3, 4, 5 ou 6,
L⁴ signifie (C₂-C₄) -alcanediyle linéaire ou ramifié, qui est en outre substitué par carboxyle,
R¹¹ signifie hydrogène ou méthyle, et
n signifie le nombre 1, 2, 3 ou 4,
ainsi que ses sels, solvates et les solvates des sels.

2. Composé de formule (I) selon la revendication 1, dans laquelle
R^{A} représente un groupe de formule 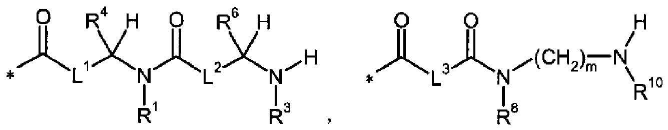 ou 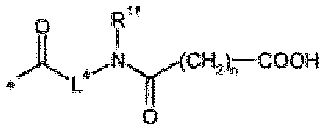 dans laquelle
* signifie le site de liaison avec l'atome de O,
L¹ signifie une liaison,
L² signifie une liaison ou -CH₂-,
R¹ et R³ signifient, indépendamment l'un de l'autre, hydrogène ou méthyle,
R⁴ signifie hydrogène, méthyle, propan-2-yle, propan-1-yle, 2-méthylpropan-1-yle, 1-méthylpropan-1-yle, butan-1-yle, benzyle, p-hydroxybenzyle, imidazol-4-ylméthyle, hydroxyméthyle, 1-hydroxyéthyle, carbamoylméthyle ou 2-carbamoyléthyle,
R⁶ signifie hydrogène, imidazol-4-ylméthyle, 4-aminobutan-1-yle, 3-aminopropan-1-yle, 2-aminoéthyle, aminométhyle ou 3-guanidinopropan-1-yle,
L³ signifie (C₂-C₄)-alcanediyle linéaire, qui est en outre substitué par amino,
R⁸ et R¹⁰ signifient, indépendamment l'un de l'autre, hydrogène ou méthyle,
m signifie le nombre 2, 3 ou 4,
L⁴ signifie (C₂-C₄)-alcanediyle linéaire, qui est en outre substitué par carboxyle,
R¹¹ signifie hydrogène ou méthyle, et
n signifie le nombre 2, 3 ou 4,
ainsi que ses sels, solvates et les solvates des sels.

3. Composé de formule (I) selon la revendication 1 ou 2, dans laquelle
R^{A} représente un groupe de formule 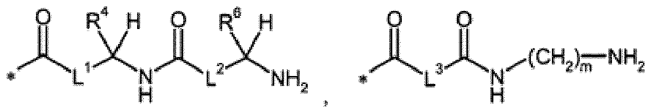 ou 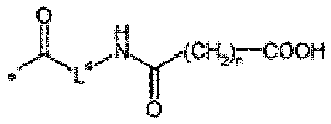 dans laquelle
* signifie le site de liaison avec l'atome de O,
L¹ et L² signifient à chaque fois une liaison,
R⁴ signifie hydrogène, méthyle, propan-2-yle, propan-1-yle, 2-méthylpropan-1-yle, 1-méthylpropan-1-yle, butan-1-yle, benzyle, p-hydroxybenzyle, imidazol-4-ylméthyle, hydroxyméthyle, 1-hydroxyéthyle, carbamoylméthyle ou 2-carbamoyléthyle,
R⁶ signifie imidazol-4-ylméthyle, 4-aminobutan-1-yle, 3-aminopropan-1-yle, 2-aminoéthyle, aminométhyle ou 3-guanidinopropan-1-yle,
L³ signifie un groupe de formule -CH(NH₂)-CH₂-, -CH₂-CH(NH₂)-, -CH₂-CH(NH₂)-CH₂-, -CH(NH₂)-CH₂-CH₂- ou -CH₂-CH₂-CH(NH₂)-,
m signifie le nombre 2, 3 ou 4,
L⁴ signifie un groupe de formule **-CH₂-CH(COOH)-ou **-CH₂-CH₂-CH(COOH)- dans lesquelles
** signifie le site de liaison avec le groupe carbonyle adjacent,
et
n signifie le nombre 2 ou 3,
ainsi que ses sels, solvates et les solvates des sels.

4. Composé de formule (I) selon la revendication 1, 2 ou 3, dans laquelle
R^{A} représente un groupe de formule 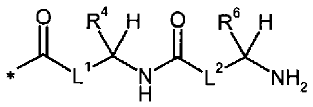 dans laquelle
* signifie le site de liaison avec l'atome de O,
L¹ et L² signifient à chaque fois une liaison,
R⁴ signifie hydrogène, méthyle, propan-2-yle, 2-méthylpropan-1-yle, benzyle, hydroxyméthyle ou 1-hydroxyéthyle et
R⁶ signifie imidazol-4-ylméthyle, 4-aminobutan-1-yle, 3-aminopropan-1-yle, 2-aminoéthyle, aminométhyle ou 3-guanidinopropan-1-yle,
ainsi que ses sels, solvates et les solvates des sels.

5. Composé de formule (I) selon la revendication 1, 2 ou 3, dans laquelle
R^{A} représente un groupe de formule 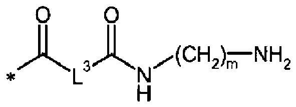 dans laquelle
* signifie le site de liaison avec l'atome de O,
L³ signifie un groupe de formule -CH(NH₂)-CH₂-, -CH₂-CH(NH₂)-, -CH(NH₂)-CH₂-CH₂- ou -CH₂-CH₂-CH(NH₂)- et
m signifie le nombre 2 ou 3,
ainsi que ses sels, solvates et les solvates des sels.

6. Composé selon la revendication 1, 2 ou 3 de formule 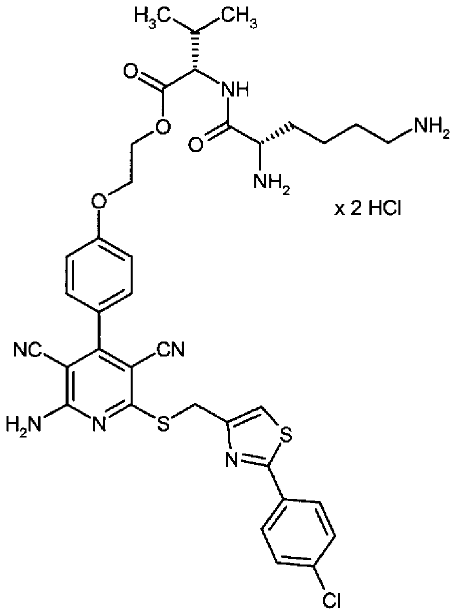 dichlorhydrate de L-lysyl-L-valinate de 2-{4-[2-amino-6-({[2-(4-chlorophényl)-1,3-thiazol-4-yl]méthyl}thio)-3,5-dicyanopyridin-4-yl]phénoxy}éthyle.

7. Composé selon la revendication 1, 2 ou 3 de formule 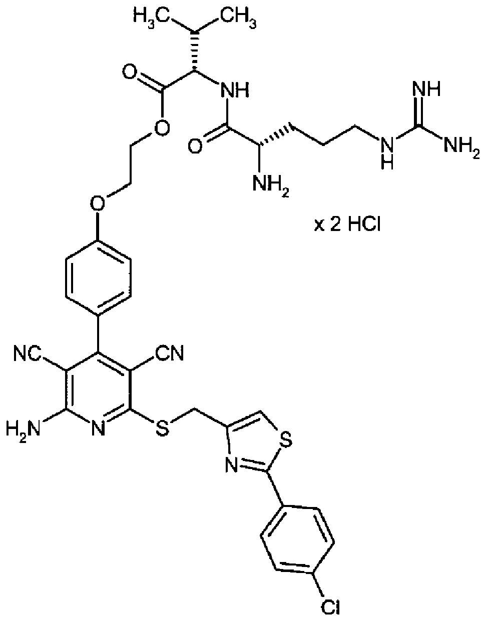 dichlorhydrate de L-arginyl-L-valinate de 2-{4-[2-amino-6-({[2-(4-chlorophényl)-1,3-thiazol-4-yl]méthyl}thio)-3,5-dicyanopyridin-4-yl]phénoxy}éthyle.

8. Composé selon la revendication 1, 2 ou 3 de formule 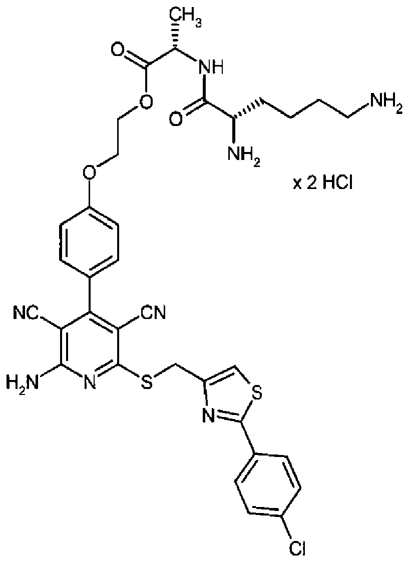 dichlorhydrate de L-lysyl-L-alaninate de 2-{4-[2-amino-6-({[2-(4-chlorophényl)-1,3-thiazol-4-yl]méthyl}thio)-3,5-dicyanopyridin-4-yl]phénoxy}éthyle.

9. Composé selon la revendication 1, 2 ou 3 de formule 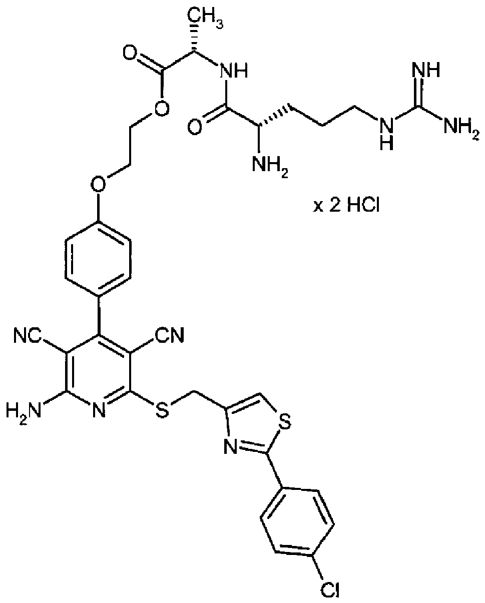 dichlorhydrate de L-arginyl-L-alaninate de 2-{4-[2-amino-6-({[2-(4-chlorophényl)-1,3-thiazol-4-yl]méthyl}sulfanyl)-3,5-dicyanopyridin-4-yl]phénoxy}éthyle.

10. Composé selon la revendication 1, 2 ou 3 de formule 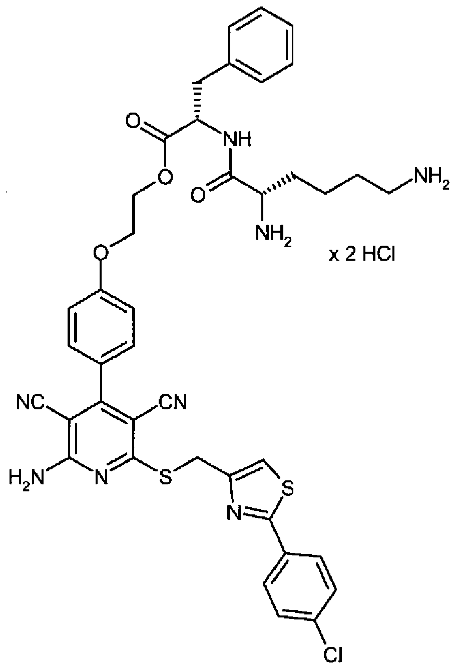 dichlorhydrate de L-lysyl-L-phénylalaninate de 2-{4-[2-amino-6-({[2-(4-chlorophényl)-1,3-thiazol-4-yl]méthyl}sulfanyl)-3,5-dicyanopyridin-4-yl]phénoxy}éthyle.

11. Composé selon la revendication 1, 2 ou 3 de formule 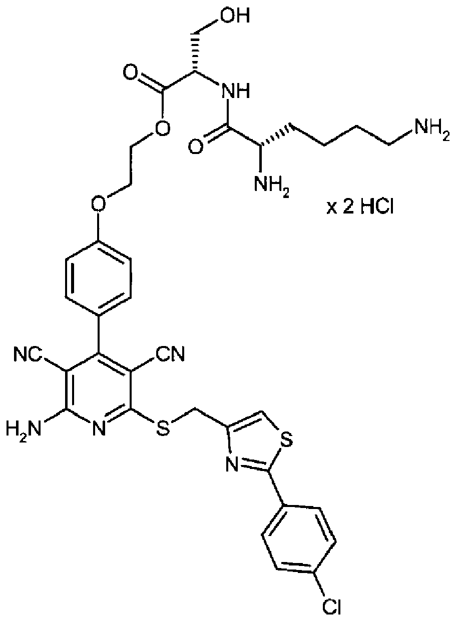 dichlorhydrate de L-lysyl-L-sérinate de 2-{4-[2-amino-6-({[2-(4-chlorophényl)-1,3-thiazol-4-yl]méthyl}thio)-3,5-dicyanopyridin-4-yl]phénoxy}éthyle.

12. Composé selon la revendication 1, 2 ou 3 de formule 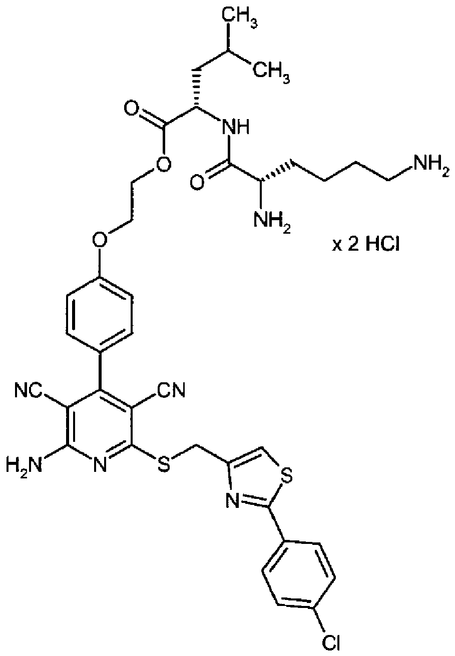 dichlorhydrate de L-lysyl-L-leucinate de 2-{4-[2-amino-6-({[2-(4-chlorophényl)-1,3-thiazol-4-yl]méthyl}sulfanyl)-3,5-dicyanopyridin-4-yl]phénoxy}éthyle.

13. Procédé pour la préparation de composés de formule (I) tels que définis dans les revendications 1 à 12, **caractérisé en ce que** le composé (A) 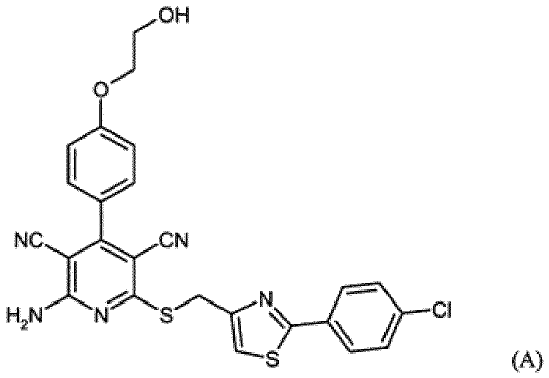 soit
[A] est d'abord estérifié dans un solvant inerte en présence d'un agent de condensation, avec un acide carboxylique de formule (II), (III) ou (IV) 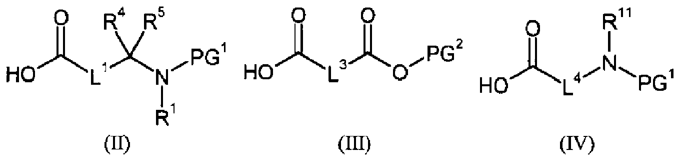 dans lesquelles L¹, L³, L⁴, R¹, R⁴, R⁵ et R¹¹ présentent à chaque fois les significations indiquées dans les revendications 1 à 5 et
PG¹ représente un groupe de protection temporaire de la fonction amino, tel que par exemple tert-butoxycarbonyle et
PG² représente un groupe de protection temporaire de la fonction carboxyle, tel que par exemple tert-butyle,
en composés de formule (V), (VI) ou (VII) 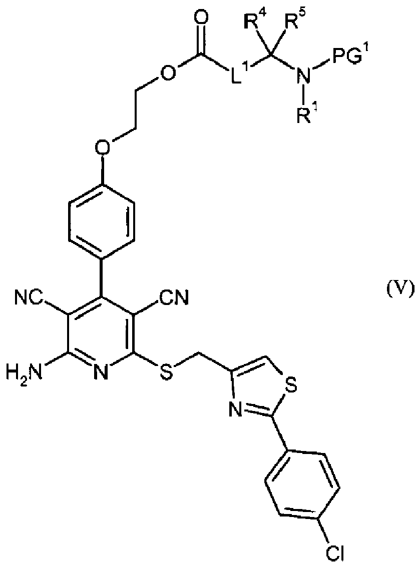 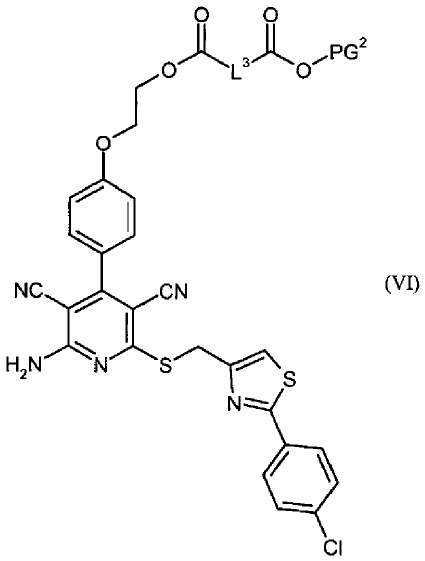 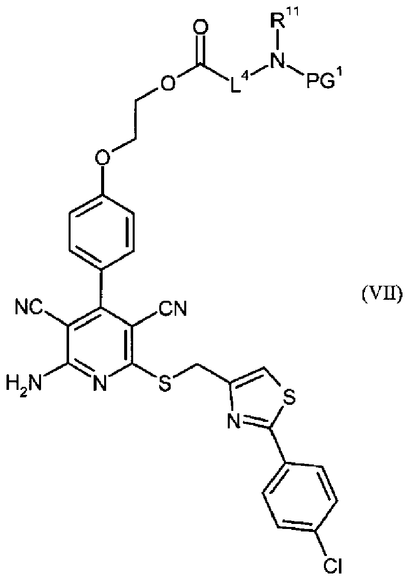 dans lesquelles L¹, L³, L⁴, R¹, R⁴, R⁵, R¹¹, PG¹ et PG² présentent à chaque fois les significations indiquées ci-dessus,
après dissociation du groupe de protection PG¹ ou PG², on couple alors dans un solvant inerte, en présence d'un agent de condensation, dans le cas du composé (V) avec un composé de formule (VIII), dans le cas du composé (VI) avec un composé de formule (IX) et dans le cas du composé (VII) avec un composé de formule (X) 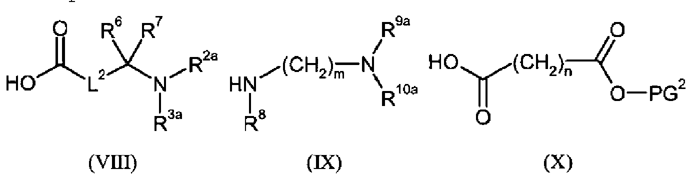 dans lesquelles L², R⁶, R⁷, R⁸, PG², m et n présentent à chaque fois les significations indiquées dans les revendications 1 à 5 et
R^{2a} et R^{3a} ainsi que R^{9a} et R^{10a} sont à chaque fois identiques ou différents et présentent les significations de R², R³, R⁹ ou R¹⁰ indiquées dans les revendications 1 à 5 ou représentent un groupe de protection temporaire de la fonction amino, tel que par exemple tert-butoxycarbonyle,
puis on élimine à nouveau les groupes de protection le cas échéant présents soit
[B] est couplé dans un solvant inerte en présence d'un agent de condensation, avec un composé de formule (XI), (XII) ou (XIII) 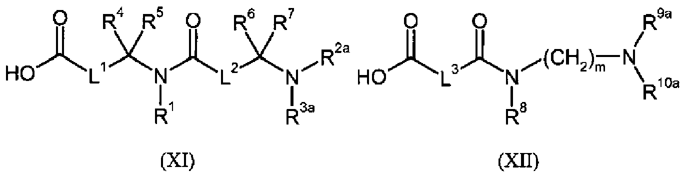 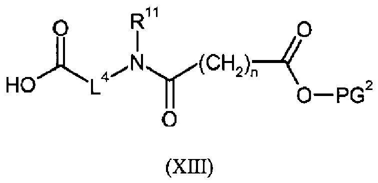 dans lesquelles L¹, L², L³, L⁴, R¹, R⁴, R⁵, R⁶, R⁷, R⁸, R¹¹, m et n présentent à chaque fois les significations indiquées dans les revendications 1 à 5,
R^{2a} et R^{3a} ainsi que R^{9a} et R^{10a} sont identiques ou différents et présentent les significations de R², R³, R⁹ ou R¹⁰ indiquées dans les revendications 1 à 5 ou représentent un groupe de protection temporaire de la fonction amino, tel que par exemple tert-butoxycarbonyle et
PG² représente un groupe de protection temporaire de la fonction carboxyle, tel que par exemple tert-butyle,
puis on élimine à nouveau les groupes de protection le cas échéant présents
et on transforme les composés obtenus de formule (I) le cas échéant avec (i) les solvants et/ou (ii) les acides ou les bases correspondants en leurs solvates, sels et/ou solvates des sels.

14. Composé tel que défini dans l'une quelconque des revendications 1 à 12, destiné au traitement et/ou à la prophylaxie de maladies.

15. Utilisation d'un composé tel que défini dans l'une des revendications 1 à 12 pour la préparation d'un médicament destiné au traitement et/ou à la prophylaxie de maladies cardiovasculaires.

16. Médicament contenant un composé, tel que défini dans l'une quelconque des revendications 1 à 12, le cas échéant en combinaison avec un ou plusieurs adjuvants inertes, non toxiques, pharmaceutiquement appropriés.

17. Médicament contenant un composé tel que défini dans l'une quelconque des revendications 1 à 12 en combinaison avec une ou plusieurs autres substances actives.

18. Médicament selon la revendication 16 ou 17 destiné au traitement et/ou à la prophylaxie de maladies cardiovasculaires.
